# EUROPEAN PATENT SPECIFICATION

(11) **EP 3 589 955 B1**
(45) Date of publication and mention of the grant of the patent: **07.06.2023**
(21) Application number: 18709742.3
(22) Date of filing: 01.03.2018
(51) Int. Cl.: G01N 33/569

(54) **URINARY TRACT INFECTION DIAGNOSTIC**
HARNWEGINFEKTIONSDIAGNOSTIK
DIAGNOSTIC D'UNE INFECTION DES VOIES URINAIRES

(30) Priority: 01.03.2017 GB 201703313
(43) Date of publication of application: 08.01.2020
(73) Proprietor: Mologic Limited, Thurleigh, Bedfordshire MK44 2YP (GB)
(72) Inventor: PAREKH, Gita, Thurleigh Bedfordshire MK44 2YP (GB); DAVIS, Paul, Thurleigh Bedfordshire MK44 2YP (GB); THOMPSON, Julie, Thurleigh Bedfordshire MK44 2YP (GB); DUVOIX, Annelyse, Thurleigh Bedfordshire MK44 2YP (GB)
(74) Representative: Boult Wade Tennant LLP
(86) International application number: PCT/GB2018/050535
(87) International publication number: WO 2018/158583

(56) References cited:
- US-A1- 2010 166 739
- US-A1- 2013 165 344
- NANDA NEHA ET AL: "Novel biomarkers for the diagnosis of urinary tract infection-a systematic review", BIOMARKER INSIGHTS, vol. 4, 1 January 2009 (2009-01-01), pages 111-121, XP002598543, ISSN: 1177-2719
- YIM HYUNG EUN ET AL: "Predictive value of urinary and serum biomarkers in young children with febrile urinary tract infections", PEDIATRIC NEPHROLOGY, vol. 29, no. 11, 13 June 2014 (2014-06-13) , pages 2181-2189, XP035398964, ISSN: 0931-041X, DOI: 10.1007/S00467-014-2845-0 [retrieved on 2014-06-13]
- SHINSUKE ARAO ET AL: "Measurement of urinary lactoferrin as a marker of urinary tract infection", JOURNAL OF CLINICAL MICROBIOLOGY, AMERICAN SOCIETY FOR MICROBIOLOGY, US, vol. 37, no. 3, 1 March 1999 (1999-03-01), pages 553-557, XP002677901, ISSN: 0095-1137
- N J VAN DER ZIJL ET AL: "Urinary matrix metalloproteinase-8 and -9 activities in type 2 diabetic subjects: A marker of incipient diabetic nephropathy?", CLINICAL BIOCHEMISTRY, vol. 43, no. 7-8, 1 May 2010 (2010-05-01), pages 635-639, XP055465608, US, CA ISSN: 0009-9120, DOI: 10.1016/j.clinbiochem.2010.02.006
- YANBAO YU ET AL: "Diagnosing inflammation and infection in the urinary system via proteomics", JOURNAL OF TRANSLATIONAL MEDICINE, vol. 13, no. 1, 8 April 2015 (2015-04-08), page 111, XP021221501, ISSN: 1479-5876, DOI: 10.1186/S12967-015-0475-3

## Description

### FIELD OF THE INVENTION

The present invention relates to the detection of urinary tract infections (UTI) and companion methods for monitoring a UTI in a subject based upon measuring the levels of various biomarkers, including at multiple time points in the subject.

### BACKGROUND TO THE INVENTION

A urinary tract infection (UTI) is an infection that affects part of the urinary tract. When it affects the lower urinary tract it is also known as a bladder infection (cystitis) and when it affects the upper urinary tract it is also known as kidney infection (pyelonephritis). Symptoms from a lower urinary tract include pain with urination, frequent urination, and feeling the need to urinate despite having an empty bladder. Symptoms of a kidney infection include fever and flank pain usually in addition to the symptoms of a lower UTI. In some cases the urine may appear bloody. In the very old and the very young, symptoms may be vague or non-specific. A common cause of infection is the presence of pathogenic *Escherichia coli* in the urinary tract, though other bacteria, viruses or fungi may be the cause. Risk factors include female anatomy, sexual intercourse, diabetes, obesity, and family history. Kidney infection, if it occurs, usually follows a bladder infection but may also result from a blood-borne infection.

Urinary tract infections (UTIs) are considered to be one the most common human infections and are estimated to affect 150 million people worldwide on an annual basis (Stamm & Norrby, Journal of Infectious Diseases (2001) 183:S1-S4). The gold standard tests for UTI are microscopic analysis and urine culture (Wilson & Gaido, Clinical Infectious Diseases (2004) 38(8):1150-1158), but there is typically a 2 day delay before culture results can be obtained, making this method unhelpful in most situations. In practice, in the clinical setting, most physicians use a urine dip-stick to ascertain the presence of nitrite, leucocytes and blood in the sample, and then treat empirically ((Schmiemann, Deutsches Ärzteblatt International (2010) 107(21): 361-367). Several documents disclose the use of urinary protein biomarkers for the assessment of UTI (Nanda et al, Biomarker Insights (2009), 4:111-121 ; US2010/166739A1; Yim et al, Pediatric Nephrology (2014), 29(11):2181-2189; Arao et al, Journal of Clinical Microbiology (1999), 37(3):553-557). US2013/165344A1 discloses MMP8 as one of a plurality of urinary biomarkers for evaluating the renal status. Yu et al, Journal of Translational Medicine (2015), 13(1):111 discloses that MMP8 and a plurality of other urinary proteins are upregulated in UTI. Approximately one third of UTI cases diagnosed by clinical criteria alone are misdiagnosed (Little, Health Technology Assessment, (2009) 13(19):iii-iv, ix-xi, 1-73; Foxman, American Journal of Medicine, (2002) S1:5-13). Consequent overtreatment with antibiotics is a significant contributor to the increasing global growth in antibiotic resistant bacteria. In a report into antimicrobial resistance (www.who.int/drugresistance/WHO_Global_Strategy_English.pdf) the World Health Organisation estimates that half of all antibiotic consumption may be unnecessary. Similarly, the UK government has recently published a Five Year Antimicrobial Resistance Strategy (www.gov.uk/government/uploads/system/uploads/attachment_data/file/244058/20130902 _UK_5_year_AMR_strategy.pdf) which clearly identifies the urgent need for the development of rapid point-of-care diagnostics as one of the key areas of action.

### DESCRIPTION OF THE INVENTION

There is a need for a more sensitive and specific assay for detecting a UTI that could be deployed in near patient settings or at the point of care. The ability to obtain rapid, accurate point-of-care detection of UTIs will have an enormous positive impact; timely, appropriate antibiotic treatment could be initiated and imprecise empirical treatment and incorrect prescription of antibiotics significantly reduced. Accordingly, the present inventors have identified individual biomarkers and combinations thereof that are effective in detecting, and monitoring, a UTI.

Thus, the invention provides a method for detecting a urinary tract infection in a subject comprising:
i) determining the protein level of at least MMP8 in a urine sample obtained from the subject; and
ii) comparing the determined protein level of at least MMP8 with a threshold level wherein an increased protein level of at least MMP8 in the urine sample relative to the threshold level is indicative of the presence of a urinary tract infection.

The invention further provides a method for monitoring a urinary tract infection in a subject comprising:
i) determining the protein level of at least MMP8 in urine samples obtained from the subject at multiple time points; and
ii) comparing the determined protein level of at least MMP8 with a threshold level wherein the continued presence of the non-decreased or increased protein level of at least MMP8 relative to the threshold level or relative to the level measured in a sample taken from an earlier time point is indicative that the urinary tract infection persists and/or that treatment has not been effective and/or the decreased protein level of at least MMP8 relative to the threshold level or relative to the level measured in a sample taken from an earlier time point is indicative of recovery from, or successful treatment of, a urinary tract infection.

The invention further provides a method for monitoring treatment of a urinary tract infection in a subject comprising:
i) determining the protein level of at least MMP8 in a urine sample obtained from the subject prior to treatment of the urinary tract infection in order to set a threshold level;
ii) determining the protein level of at least MMP8 in a further urine sample obtained from the subject following treatment of the urinary tract infection wherein a non-decreased or increased protein level of at least MMP8 relative to the threshold level is indicative that the treatment has not been effective and/or a decreased protein level of at least MMP8 relative to the threshold level is indicative of successful treatment of the urinary tract infection.

The invention further provides a system or test kit for detecting a urinary tract infection in a subject, comprising:
a. One or more testing devices for determining protein levels of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a urine sample obtained from the subject
b. A processor; and
c. A storage medium comprising a computer application that, when executed by the processor, is configured to:
   i. Access and/or calculate the determined protein level of at least MMP8 in the sample on the one or more testing devices
   ii. Calculate a test score from the protein level of at least MMP8 in the sample that detects a urinary tract infection; and
   iii. Output from the processor the detected result for the subject.

The invention further provides a system or test kit for monitoring a urinary tract infection in
a subject, comprising:
a. One or more testing devices for determining the protein level of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a urine sample obtained from the subject at multiple time points
b. A processor; and
c. A storage medium comprising a computer application that, when executed by the processor, is configured to:
   i. Access and/or calculate the determined protein level of at least MMP8 in the sample on the one or more testing devices
   ii. Calculate a test score from the protein level of at least MMP8 in the sample, optionally including a comparison of the level with those taken at one or more earlier time points, that detects a urinary tract infection; and
Output from the processor the detected result for the subject.

In one embodiment, the invention provides a method for detecting a urinary tract infection in a subject comprising, consisting essentially of or consisting of:
i) determining the protein level of at least MMP8 and optionally one or more biomarkers selected from human neutrophil elastase (HNE), Cystatin C, matrix metalloproteinase-9 (MMP9), human serum albumin (HSA), interleukin-8 (IL-8), interleukin-6 (IL-6), interleukin-1 beta (IL-1b), fibrinogen, RBP4, active MMP9 and MMP2, neutrophil gelatinase-associated lipocalin (NGAL), Desmosine, myeloperoxidase (MPO) and C-reactive protein (CRP) in a urine sample obtained from the subject; and
ii) comparing the determined protein level of at least MMP8 with a threshold level wherein an increased protein level of at least MMP8 in the urine sample relative to the threshold level is indicative of the presence of a urinary tract infection.

As discussed above, a urinary tract infection (UTI) is an infection that affects part of the urinary tract. When it affects the lower urinary tract it may also be described as a bladder infection (cystitis) and when it affects the upper urinary tract it may also be described as a kidney infection (pyelonephritis).

The biomarkers useful in the invention are typically protein biomarkers, although desmosine is an amino acid rather than a protein. Some are enzymes. Methods for determining their levels are disclosed in further detail hereinbelow.

The structure and function of the extracellular matrix (ECM) is vital in a number of physiological processes, including embryonic development, tissue repair and remodelling. ECM structure is ultimately regulated by the balance of extracellular proteases and protease inhibitors, which includes the collagen-cleaving **Matrix MetalloProteinase-8 (MMP8)** as well as **Matrix MetalloProteinase-9 (MMP9).** They are tightly regulated on a transcriptional level as well as on the activating level (Hadler-Olsen et al., FEBS Journal (2011) 278(1):28-45). They are produced as pro-forms and cleavage to their active forms is carried out by other MMPs and proteinases. MMPs are also regulated by the tissue inhibitors of metalloproteinases (TIMPs), which bind to the catalytic site of MMPs, thereby inhibiting uncontrolled digestion of ECM components (Bode et al., Annals of the New York Academy of Sciences (1999) 878:73-91).

**Human Neutrophil Elastase (HNE)** is a 29.5kDa serine proteinase that has broad substrate specificity. It is secreted by neutrophils and macrophages during inflammation and degrades proteins including collagen and elastin, damaging bacteria and surrounding tissues. HNE is also capable of cleaving pro-MMP9 into MMP9 while additionally cleaving TIMP1, an MMP9 inhibitor, increasing the protease/anti-protease imbalance and the risk of tissue damage (Jackson et al., Molecular Medicine (2010) 16(5-6): 159-166). Human Neutrophil elastase and α1-antitrypsin are a pair of protease and protease inhibitor counterparts. The loss of the protease/anti-protease balance can lead to extensive tissue damage. A deficiency in α1-antitrypsin is responsible for early onset of chronic liver disease, emphysema, and aneurysm (Sun and Yang, The Lancet Oncology (2004) 5(3): 182-190).

**Cystatin C** is a 13kDa protein containing four characteristic disulfide-paired cysteine residues. It acts as a cysteine proteinase inhibitor belonging to the type 2 cystatin superfamily and is ubiquitously expressed at moderate levels. Cystatin C is mainly used as a biomarker of kidney function and could replace creatinine which cannot detect mild renal impairment, and varies with muscle mass and protein intake (Roos et al., Clinical Biochemistry (2007) 40 (5-6): 383-391). Cystatin C is removed from the bloodstream by glomerular filtration in the kidneys. Impairment in kidney function induces a rise of cystatin C levels in the blood while reducing levels in the urine, promoting a change in the protease balance. Cystatin C is also a marker of inflammation, which can lead to kidney impairment, and is linked to an increased risk of cardiovascular events (Taglieri et al., Clinical Chemistry (2009) 55 (11): 1932-43).

**Human serum albumin** is the version of serum albumin found in human blood. It is the most abundant protein in human blood plasma; it constitutes about half of serum protein. It is produced in the liver. It is soluble and monomeric. Albumin transports hormones, fatty acids, and other compounds, buffers pH, and maintains oncotic pressure, among other functions. Albumin is synthesized in the liver as preproalbumin, which has an N-terminal peptide that is removed before the nascent protein is released from the rough endoplasmic reticulum. The product, proalbumin, is in turn cleaved in the Golgi vesicles to produce the secreted albumin. The reference range for albumin concentrations in serum is approximately 35 - 50 g/L (3.5 - 5.0 g/dL). It has a serum half-life of approximately 20 days. It has a molecular mass of 66.5 kDa.

**Interleukin 8** (IL-8 or chemokine (C-X-C motif) ligand 8, CXCL8) is a chemokine produced by macrophages and other cell types such as epithelial cells, airway smooth muscle cells and endothelial cells. Endothelial cells store IL-8 in their storage vesicles, the Weibel-Palade bodies. In humans, the interleukin-8 protein is encoded by the IL8 gene. IL-8 is initially produced as a precursor peptide of 99 amino acids long which then undergoes cleavage to create several active IL-8 isoforms. In culture, a 72 amino acid peptide is the major form secreted by macrophages. There are many receptors on the surface membrane capable of binding IL-8; the most frequently studied types are the G protein-coupled serpentine receptors CXCR1 and CXCR2. Expression and affinity for IL-8 differs between the two receptors (CXCR1 > CXCR2). Through a chain of biochemical reactions, IL-8 is secreted and is an important mediator of the immune reaction in the innate immune system response.

**Interleukin 6** (IL-6) is a cytokine that functions in inflammation and the maturation of B cells.

**Interleukin 1 beta** (IL-1b) is a member of the interleukin 1 cytokine family. This cytokine is produced by activated macrophages as a proprotein, which is proteolytically processed to its active form by caspase 1 (CASP1/ICE). This cytokine is an important mediator of the inflammatory response, and is involved in a variety of cellular activities, including cell proliferation, differentiation, and apoptosis.

**Retinol binding protein 4** (RBP4) belongs to the lipocalin family and is the specific carrier for retinol (vitamin A alcohol) in the blood. It delivers retinol from the liver stores to the peripheral tissues.

**Fibrinogen** (factor I) is a glycoprotein involved in blood coagulation.

**Neutrophil gelatinase-associated lipocalin** (NGAL), also known as Lipocalin-2 (LCN2) and oncogene 24p3, is a protein that in humans is encoded by the LCN2 gene. NGAL is involved in innate immunity by sequestrating iron that in turn limits bacterial growth. It is expressed in neutrophils and in low levels in the kidney, prostate, and epithelia of the respiratory and alimentary tracts.

**Desmosine** is formed from three allysyl side chains plus one unaltered lysyl side chain from the same or neighbouring elastin polypeptides.

**Myeloperoxidase** (MPO) is a peroxidase enzyme that in humans is encoded by the MPO gene on chromosome 17. MPO is most abundantly expressed in neutrophil granulocytes (a subtype of white blood cells), and produces hypohalous acids to carry out their antimicrobial activity. It is a lysosomal protein stored in azurophilic granules of the neutrophil and released into the extracellular space during degranulation. MPO has a heme pigment, which causes its green color in secretions rich in neutrophils, such as pus and some forms of mucus.

**C-reactive protein** (CRP) is a pentameric protein that in humans is encoded by the *CRP* gene located on chromosome 1 (1q23.2). It is a member of the small pentraxins family and is 224 amino acids in length. It has a monomer molecular mass of 25,106 Da.

Once a UTI is detected in a subject, the present inventors have shown that the biomarkers described herein can be used to monitor the subject to determine whether the UTI persists over time. For instance, once a UTI is detected according to the methods of the invention, preferably at the point-of-care, the subject may be administered a treatment for the UTI. The biomarkers described herein can be used to monitor the subject to determine whether the UTI persists over the course of treatment and, therefore, whether or not the treatment is successful. Thus, the methods of the invention firstly provide a more rapid detection of a UTI compared with known methods. This prevents the inappropriate use of treatments such as antibiotics based on a false UTI diagnosis by known methods (e.g. by virtue of a subject being treated for a UTI with antibiotics due to a false UTI diagnosis based on symptoms alone). Moreover, where a UTI is detected by the methods of the invention, failure of a particular treatment to treat the UTI can more rapidly be detected by the methods of the present invention relative to known methods in the art, preferably at the point-of-care, enabling alternative treatments to be prescribed for the subject. This is clearly beneficial for the subject as well as, for example, reducing ineffective antibiotic use and positive selection pressure for pathogens responsible for the UTI which are resistant to one or more antibiotics. Since the invention relies upon the determination of protein levels of the markers, which may be quantitative or semi-quantitative, monitoring can provide by comparison with previously measured levels, a relative indication of the progression, or treatment, of the UTI.

Accordingly, in one embodiment, the invention provides a method of monitoring a urinary tract infection in a subject comprising, consisting essentially of or consisting of:
i) determining the protein level of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in urine samples obtained from the subject at multiple time points; and
ii) comparing the determined protein level of at least MMP8 with a threshold level
wherein the continued presence of the non-decreased or increased protein level of at least MMP8 relative to the threshold level or relative to the level measured in a sample taken from an earlier time point is indicative that the urinary tract infection persists and/or that treatment has not been effective and/or the decreased protein level of at least MMP8 relative to the threshold level or relative to the level measured in a sample taken from an earlier time point is indicative of recovery from, or successful treatment of, a urinary tract infection.

The methods described herein with regard to the detection of a UTI and monitoring of a UTI may be combined to provide a method for detecting and monitoring a urinary tract infection in a subject comprising, consisting essentially of or consisting of:
i)
   a) determining the protein level of at least MMP8 and optionally one or more biomarkers selected from, human neutrophil elastase (HNE), Cystatin C, matrix metalloproteinase-9 (MMP9), human serum albumin (HSA), interleukin-8 (IL-8), interleukin-6 (IL-6), interleukin-1 beta (IL-1b), fibrinogen, RBP4, active MMP9 and MMP2, Neutrophil gelatinase-associated lipocalin (NGAL), Desmosine, myeloperoxidase (MPO) and C-reactive protein (CRP) in a urine sample obtained from the subject; and
   b) comparing the determined protein level of at least MMP8 with a threshold level
   wherein the increased protein level of at least MPP8 in the urine sample relative to the threshold level is indicative of the presence of a urinary tract infection; and, where a urinary tract infection is detected
ii) determining the protein level of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a further urine sample obtained from the subject at a later time point; wherein a non-decreased or increased protein level of at least MMP8 relative to the threshold level or to the level measured in step (i) is indicative that the urinary tract infection persists and/or a decreased protein level of at least MMP8 relative to the threshold level or to the level measured in step (i) is indicative of recovery from, or successful treatment of, the urinary tract infection.

Similarly, the invention also provides a method for monitoring treatment of a urinary tract infection in a subject comprising, consisting essentially of or consisting of:
i) determining the protein level of at least MMP8 and optionally one or more biomarkers selected from HNE, Cystatin C, MMP9, HSA, IL-8, interleukin-6 (IL-6), interleukin-1 beta (!L-1b), fibrinogen, RBP4, active MMP9 and MMP2, NGAL, Desmosine, MPO and CRP in a urine sample obtained from the subject prior to treatment of the urinary tract infection in order to set a threshold level;
ii) determining the protein level of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a further urine sample obtained from the subject following treatment of the urinary tract infection
wherein a non-decreased or increased protein level of at least MMP8 relative to the threshold level is indicative that the treatment has not been effective and/or a decreased protein level of at least MMP8 relative to the threshold level is indicative of successful treatment of the urinary tract infection.

All markers described herein have been shown to produce useful detection of UTls when used individually.

According to all aspects of the invention, in some embodiments (which may be preferred) the protein levels of at least two or three of the biomarkers are determined, wherein at least one of the markers is MMP8. In particular embodiments, non-decreased or increased protein levels of at least two or three of the biomarkers, wherein at least one marker is MMP8, in the urine sample relative to the threshold level or relative to the levels measured in a sample taken from an earlier time point are indicative of the presence of a UTI. Conversely, decreased protein levels of at least two or three of the biomarkers, wherein at least one marker is MMP8, in the urine sample relative to the threshold level or relative to the levels measured in a sample taken from an earlier time point are indicative of recovery from a UTI. The invention does not preclude other biomarkers (i.e. beyond those listed) from also being employed.

In further embodiments, the protein levels of at least four, five, six, seven, eight or all of the biomarkers are determined as long as at least one marker is MMP8. In some embodiments, non-decreased or increased protein levels of at least four, five, six, seven, eight or all of the biomarkers in the urine sample, wherein at least one marker is MMP8, relative to the threshold level or relative to the levels measured in a sample taken from an earlier time point are indicative of the presence of a UTI. Conversely, decreased protein levels of at least four, five, six, seven, eight or all of the biomarkers in the urine sample, wherein at least one marker is MMP8, relative to the threshold level or relative to the levels measured in a sample taken from an earlier time point are indicative of recovery from a UTI.

Similarly, where the subject has received treatment for a UTI as described herein, non-decreased or increased protein levels of at least two, three, four, five, six, seven, eight or all of the biomarkers in the urine sample, wherein at least one marker is MMP8, relative to the threshold level or relative to the levels measured in a sample taken from an earlier time point may be indicative that the treatment has not been effective. Thus, the clinician may prescribe an alternative treatment for the subject. Conversely, decreased protein levels of at least two, three, four, five, six, seven, eight or all of the biomarkers in the urine sample, wherein at least one marker is MMP8, relative to the threshold level or relative to the levels measured in a sample taken from an earlier time point may be indicative that the treatment has been effective and should be continued (or ceased if the UTI has been cured).

In certain embodiments according to all aspects of the invention, the biomarkers comprise at least MMP8 and optionally HNE, Cystatin C, MMP9, IL-8, interleukin-6 (IL-6), interleukin-1 beta (IL-1b) and MPO or at least MMP8 and optionally HNE, Cystatin C, MMP9, IL-8, interleukin-6 (IL-6), interleukin-1 beta (IL-1b), MPO, fibrinogen and CRP. This may optionally be in combination with at least one of HSA, desmosine and NGAL.

In certain embodiments according to all aspects of the invention, the biomarkers comprise MMP8, or comprise at least MMP8 and at least one of HNE, fibrinogen or CRP, optionally:
(i) both MMP8 and HNE;
(ii) MMP8, HNE and fibrinogen in combination; or
(iii) MMP8, HNE and CRP in combination.

That is to say, in the context of the methods of the invention, the methods comprise determining the levels of at least MMP8 and preferably comprise determining the levels of at least MMP8, and at least one of HNE, fibrinogen or CRP, optionally:
(i) both MMP8 and HNE;
(ii) MMP8, HNE and fibrinogen in combination; or
(iii) MMP8, HNE and CRP in combination.

Thus, in addition to MMP8, the biomarkers whose protein levels are determined may comprise at least one of NGAL, MMP9, Desmosine or MPO, optionally NGAL, MMP9 and Desmosine in combination or all of NGAL, MMP9, Desmosine and MPO. The biomarkers may additionally comprise Cystatin C.

A specific biomarker combination shown herein to be particularly useful in detecting and monitoring a UTI is MMP8, HNE and Cystatin C. Thus, in a preferred embodiment according to all aspects of the invention, the biomarkers comprise MMP8, HNE and Cystatin C (in combination). Thus, the invention provides a method for detecting a urinary tract infection in a subject comprising, consisting essentially of or consisting of:
i) determining the protein levels of matrix metalloproteinase-8 (MMP8), human neutrophil elastase (HNE) and Cystatin C in a urine sample obtained from the subject; and
ii) comparing each determined protein level with a threshold level
wherein an increased protein level of at least MMP8 in the urine sample relative to the threshold level is indicative of the presence of a urinary tract infection.

The invention also provides a method of monitoring a urinary tract infection in a subject comprising, consisting essentially of or consisting of:
i) determining protein levels MMP8, HNE and Cystatin C in urine samples obtained from the subject at multiple time points; and
ii) comparing each determined protein level with a threshold level
wherein the continued presence of a non-decreased or increased protein level of at least MMP8 relative to the threshold level or relative to the level measured in a sample taken from an earlier time point is indicative that the urinary tract infection persists and/or that treatment has not been effective and/or a decreased protein level of at least one MMP8 relative to the threshold level or relative to the level measured in a sample taken from an earlier time point is indicative of recovery from, or successful treatment of, a urinary tract infection.

The methods described herein with regard to the detection of a UTI and monitoring of a UTI may be combined to provide a method for detecting and monitoring a urinary tract infection in a subject comprising, consisting essentially of or consisting of:
i)
   a) determining protein levels of matrix metalloproteinase-8 (MMP8), human neutrophil elastase (HNE) and Cystatin C in a urine sample obtained from the subject; and
   b) comparing each determined protein level with a threshold level wherein an increased protein level of at least MMP8 in the urine sample relative to the threshold level is indicative of the presence of a urinary tract infection; and,
   where a urinary tract infection is detected
ii) determining protein levels of MMP8, HNE and Cystatin C in a further urine sample obtained from the subject at a later time point;
wherein a non-decreased or increased protein level of at least MMP8 relative to the threshold level or to the level measured in step (i) is indicative that the urinary tract infection persists and/or a decreased protein level of at least MMP8 relative to the threshold level or to the levels measured in step (i) is indicative of recovery from, or successful treatment of, the urinary tract infection.

Similarly, the invention also provides a method for monitoring treatment of a urinary tract infection in a subject comprising, consisting essentially of or consisting of:
i) determining protein levels of MMP8, HNE and Cystatin C in a urine sample obtained from the subject prior to treatment of the urinary tract infection in order to set a threshold level;
ii) determining the protein level of at least MMP8 and optionally HNE, Cystatin C, MMP9, HSA, IL-8, interleukin-6 (IL-6), interleukin-1 beta (IL-1b), fibrinogen, RBP4, active MMP9 and MMP2, NGAL, Desmosine and MPO in a further urine sample obtained from the subject following treatment of the urinary tract infection
wherein a non-decreased or increased protein level of at least MMP8 relative to the threshold level is indicative that the treatment has not been effective and/or a decreased protein level of at least MMP8 relative to the threshold level is indicative of successful treatment of the urinary tract infection.

Another specific biomarker combination shown herein to be particularly useful in detecting and monitoring a UTI is MMP8, HNE and fibrinogen. Thus, in a preferred embodiment according to all aspects of the invention, the biomarkers comprise MMP8, HNE and fibrinogen (in combination). Thus, the invention provides a method for detecting a urinary tract infection in a subject comprising, consisting essentially of or consisting of:
i) determining protein levels of matrix metalloproteinase-8 (MMP8), human neutrophil elastase (HNE) and fibrinogen in a urine sample obtained from the subject; and
ii) comparing each determined protein level with a threshold level
wherein an increased protein level of at least MMP8 in the urine sample relative to the threshold level is indicative of the presence of a urinary tract infection.

The invention also provides a method of monitoring a urinary tract infection in a subject comprising, consisting essentially of or consisting of:
i) determining protein levels of MMP8, HNE and fibrinogen in urine samples obtained from the subject at multiple time points; and
ii) comparing each determined protein level with a threshold level
wherein the continued presence of a non-decreased or increased protein level of at least MMP8 relative to the threshold level or relative to the level measured in a sample taken from an earlier time point is indicative that the urinary tract infection persists and/or that treatment has not been effective and/or a decreased protein level of at least MMP8 relative to the threshold level or relative to the level measured in a sample taken from an earlier time point is indicative of recovery from, or successful treatment of, a urinary tract infection.

The methods described herein with regard to the detection of a UTI and monitoring of a UTI may be combined to provide a method for detecting and monitoring a urinary tract infection in a subject comprising, consisting essentially of or consisting of:
i)
   a) determining protein levels of matrix metalloproteinase-8 (MMP8), human neutrophil elastase (HNE) and fibrinogen in a urine sample obtained from the subject; and
   b) comparing each determined protein level with a threshold level
   wherein an increased level of at least MMP8 in the urine sample relative to the threshold level is indicative of the presence of a urinary tract infection; and, where a urinary tract infection is detected
ii) determining protein levels of MMP8, HNE and fibrinogen in a further urine sample obtained from the subject at a later time point;
wherein a non-decreased or increased protein level of at least MMP8 relative to the threshold level or to the level measured in step (i) is indicative that the urinary tract infection persists and/or a decreased protein level of at least MMP8 relative to the threshold level or to the level measured in step (i) is indicative of recovery from, or successful treatment of, the urinary tract infection.

Similarly, the invention also provides a method for monitoring treatment of a urinary tract infection in a subject comprising, consisting essentially of or consisting of:
i) determining protein levels of MMP8, HNE and fibrinogen in a urine sample obtained from the subject prior to treatment of the urinary tract infection in order to set a threshold level;
ii) determining protein levels of at least MMP8 and optionally HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a further urine sample obtained from the subject following treatment of the urinary tract infection
wherein a non-decreased or increased protein levels of at least MMP8 relative to the threshold level is indicative that the treatment has not been effective and/or a decreased protein levels of at least MMP8 relative to the threshold level is indicative of successful treatment of the urinary tract infection.

Yet another specific biomarker combination shown herein to be particularly useful in detecting and monitoring a UTI is MMP8, HNE and CRP. Thus, in a preferred embodiment according to all aspects of the invention, the biomarkers comprise MMP8, HNE and fibrinogen (in combination). Thus, the invention provides a method for detecting a urinary tract infection in a subject comprising, consisting essentially of or consisting of:
i) determining protein levels of matrix metalloproteinase-8 (MMP8), human neutrophil elastase (HNE) and C-reactive protein (CRP) in a urine sample obtained from the subject; and
ii) comparing each determined protein level with a threshold level wherein an increased protein level of at least MMP8 in the urine sample relative to the threshold level is indicative of the presence of a urinary tract infection.

The invention also provides a method of monitoring a urinary tract infection in a subject comprising, consisting essentially of or consisting of:
i) determining protein levels MMP8, HNE and CRP in urine samples obtained from the subject at multiple time points; and
ii) comparing each determined protein level with a threshold level
wherein the continued presence of a non-decreased or increased protein level of at least MMP8 relative to the threshold level or relative to the level measured in a sample taken from an earlier time point is indicative that the urinary tract infection persists and/or that treatment has not been effective and/or a decreased protein level of at least MMP8 relative to the threshold level or relative to the level measured in a sample taken from an earlier time point is indicative of recovery from, or successful treatment of, a urinary tract infection.

The methods described herein with regard to the detection of a UTI and monitoring of a UTI may be combined to provide a method for detecting and monitoring a urinary tract infection in a subject comprising, consisting essentially of or consisting of:
i)
   a) determining protein levels of matrix metalloproteinase-8 (MMP8), human neutrophil elastase (HNE) and C-reactive protein (CRP) in a urine sample obtained from the subject; and
   b) comparing each determined protein level with a threshold level
   wherein an increased protein level of at least MMP8 in the urine sample relative to the threshold level is indicative of the presence of a urinary tract infection; and, where a urinary tract infection is detected
ii) determining protein levels of MMP8, HNE and CRP in a further urine sample obtained from the subject at a later time point;
wherein a non-decreased or increased protein level of at least MMP8 relative to the threshold level or to the level measured in step (i) is indicative that the urinary tract infection persists and/or a decreased protein level of at least MMP8 relative to the threshold level or to the level measured in step (i) is indicative of recovery from, or successful treatment of, the urinary tract infection.

Similarly, the invention also provides a method for monitoring treatment of a urinary tract infection in a subject comprising, consisting essentially of or consisting of:
i) determining protein levels of MMP8, HNE and CRP in a urine sample obtained from the subject prior to treatment of the urinary tract infection in order to set a threshold level;
ii) determining protein levels of at least MMP8 and optionally HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a further urine sample obtained from the subject following treatment of the urinary tract infection
wherein a non-decreased or increased protein level of at least MMP8 relative to the threshold level is indicative that the treatment has not been effective and/or a decreased protein level of at least MMP8 relative to the threshold level is indicative of successful treatment of the urinary tract infection.

For general monitoring, according to all relevant aspects of the invention, at least 2, 3, 4, 5, 6, 7, 8, 9, 10, 11, 12, 13 or 14 or more urine samples may be taken from the subject at different times and the levels of the one or more biomarkers is determined, wherein at least one biomarker is MMP8. The urine samples may be taken every 6 to 24 hours, such as daily, or every 2, 3, 4, 5, 6 days or weekly for example. This depends on the nature of the subject and the perceived risk and/or progression of a UTI. For example, urine samples may be taken more frequently, such as daily for patients at significant risk of developing a UTI e.g. patients with diabetes or a family history of UTls. The frequency of sampling may vary depending on the results of the previous test. Thus, more frequent testing may be undertaken where increased levels of the one or more biomarkers are detected in later samples, for example in response to treatment. Conversely, detection of stable and/or decreasing and/or decreased levels in multiple urine samples may lead to a reduction in frequency of testing, or cessation.

The invention provides for patient selection for therapy and, thus, avoids unnecessary treatment with antibiotics. Incorrect use of antibiotics fuels antibiotic resistance and can cause severe adverse drug reactions.

Accordingly, the invention also relates to a method of selecting a subject for treatment with an antibiotic comprising performing a method described herein and selecting the subject for treatment where a UTI is detected and/or persists.

In a related aspect, the present invention provides a method of predicting responsiveness of a subject to treatment with an antibiotic comprising performing a method described herein and predicting responsiveness of the subject to treatment where a UTI is detected or persists.

A method of treating a UTI may comprise administering an antibiotic to the subject suffering from a UTI, wherein the subject has been selected for treatment by performing a method described herein.

A method of treating a UTI may comprise administering an antibiotic to the subject suffering from a UTI, wherein the subject displays, in a urine sample, an altered (i.e. increased) level of at least one biomarker selected from MMP8, HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP.

Antibiotics useful in treating a UTI are known in the art and any suitable antibiotic may be employed. The antibiotic may be a broad spectrum antibiotic. This is particularly useful if a UTI is detected but where the origin of the infection has not yet been characterised. Once a UTI has been detected, the infection may be characterised so as to allow more targeted therapy (e.g. as bacterial, which may be gram-positive or gram-negative, viral or fungal). The antibiotic may be selected from an aminoglycoside, a cephalosporin, a glycopeptide, a penicillin, a quinolone, aztreonam, clindamycin, imipenem-cilastin, linezolid, metronidazole, rifampin, an antifungal and an antiviral (which may be a broad spectrum antiviral). Thus, combinations of broad spectrum antibiotics and more focussed therapies may be employed as part of the methods described herein.

The methods of the invention may be implemented in a system integrating the various components or test kit of parts. Such a system or test kit is preferably suitable for use, and ideally very easy to use (e.g. with a readily interpreted output), by clinicians in a healthcare setting such as a GP surgery or hospital. Such systems or test kits may also be useful for home applications.

Accordingly, in another aspect, the invention provides a system or test kit for detecting a urinary tract infection in a subject, comprising, consisting essentially of or consisting of:
a. One or more testing devices for determining protein levels of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a urine sample obtained from the subject
b. A processor; and
c. A storage medium comprising a computer application that, when executed by the processor, is configured to:
   i. Access and/or calculate the determined protein level of at least MMP8 in the sample on the one or more testing devices
   ii. Calculate a test score from the level of MMP8 in the sample that detects a urinary tract infection; and
   iii. Output from the processor the detected result for the subject.

Also provided is system or test kit for monitoring a urinary tract infection in a subject, comprising, consisting essentially of or consisting of:
a. One or more testing devices for determining the protein level of at least MMP8 and optionally one or more biomarkers selected from MMP8, HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a urine sample obtained from the subject at multiple time points
b. A processor; and
c. A storage medium comprising a computer application that, when executed by the processor, is configured to:
   i. Access and/or calculate the determined protein level of at least MMP8 in the sample on the one or more testing devices
   ii. Calculate a test score from the protein level of at least MMP8 in the sample, optionally including a comparison of the levels with those taken at one or more earlier time points, that detects a urinary tract infection; and
   iii. Output from the processor the detected result for the subject.

The processor and storage medium may be comprised within a reader device. The reader device may comprise a housing dimensioned so as to receive the one or more testing devices.

The determined protein levels may be stored in the reader so as to be accessible when a future sample is tested using a further testing device. Similarly, the levels taken at one or earlier time points may be stored for future access.

The one or more testing devices may comprise some form of unique identifier that can be read by the reader device to thereby assign the results to a particular subject. Alternatively, the user may be able to input an identifier into the reader prior to use thereby identifying the subject. These features are particularly important for monitoring functions, especially where the reader may be used by multiple different subjects.

The invention also provides a testing device, or testing kit comprising, consisting essentially of or consisting of:
a. A sample receiving zone to which a urine sample from a subject is added
b. A conjugate zone comprising at least one labelled binding reagent which specifically binds to MMP8 and optionally one or two labelled binding reagents, each of which specifically binds to one of the biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP
c. A solid support defining a liquid flow path for the sample and comprising a corresponding test line for at least MMP8, the test line comprising:
   i. an immobilised further binding reagent that also specifically binds to MMP8 thereby immobilising MMP8 at the test line to produce a signal via the labelled binding reagent also specifically bound to MMP8; or
   ii. an immobilised version of MMP8 or an analogue thereof able to compete with MMP8 in the sample for specific binding to the labelled binding reagent.

The test lines for each biomarker are spatially separated to permit levels of each biomarker to be measured and discriminated from levels of the other biomarker(s).

In particular embodiments, the boundary of the sample receiving zone may be marked for the user's convenience; for instance, using one or more symbols such as arrows. The user should dip the sample receiving zone portion of the strip into the sample up to the one or more symbols. This ensures that the sample receiving zone is sufficiently brought into contact with the sample to be tested but that the downstream components (e.g. test lines) are not.

The testing device or testing kit may further comprise:
d. At least one labelled control binding reagent that binds to a binding partner immobilised at a control line downstream of the test line(s) for at least MMP8 and thus confirms that the test has completed successfully.

The control line is spatially separated from the test lines for each biomarker.

By way of illustration, in specific embodiments the binding partner immobilised at the control line comprises BSA-Biotin and the labelled control binding reagent that binds to the immobilized binding partner comprises an anti-Biotin antibody complexed with gold particles.

The testing device or testing kit may further comprise:
e. An absorbent material downstream of the test (and control, where present) line(s) to absorb excess sample.

In specific embodiments, the solid support comprises a chromatographic medium or a capillary flow device. The invention may be provided in a test strip format in some embodiments.

In particular embodiments, a region is provided downstream of the test lines (and control line and/or absorbent material if present) which can be held by hand by the user. Thus, the user can easily manipulate the testing device or testing kit without compromising the sample and subsequent testing thereof. The region may be called a "hold region" and can be made of any suitable material, such as plastic. The region may be visibly marked "hold region" or simply "hold" or similar for the user's convenience.

In some embodiments, the testing device or testing kit further comprises a vessel suitable for, or preferably specifically designed for, collecting a urine sample. The vessel may be coloured to protect any light sensitive analytes (biomarkers). Thus, the urine sample may first be collected and subsequently an aliquot of the sample brought into contact with the sample receiving zone. Once collected and prior to testing the sample, the collected sample may be stored and/or frozen.

The testing device or testing kit may further comprise a visual aid such as a printed document (e.g. a printed card) displaying different line intensity patterns from which the user can interpret the results of the completed assay(s). By way of example to illustrate the concept, where the biomarker is detected via a sandwich assay format as described herein, the lines may be graded (Grade lines 1-10) wherein Grade line 1 is the lightest coloured line followed by Grade line 2 which is more intense in colour and so on to Grade line 10 which is the darkest (i.e. most intensely coloured) line; Grade lines 1 and 2 being calibrated at or below a pre-determined threshold level and indicating that the specific biomarker is present but within normal (i.e. non-infectious) parameters and therefore a UTI is not detected whilst Grade lines 3-10, calibrated above the pre-determined threshold level, indicate that the specific biomarker is present in abnormally high levels and therefore a UTI is detected. The increasing intensities of Grade lines 3-10 enables the user, particularly when analysing multiple samples taken over time using the monitoring methods described herein, to understand whether the biomarker levels are continuing to abnormally increase and therefore whether the severity of the UTI is becoming greater and/or current treatment is ineffective. A null grade line (Grade line 0) may also be provided for which no coloured line is displayed on the visual aid indicating that the biomarker is absent (or present at negligible levels) from the urine sample. Thus, the user can read the assay results by eye and obtain a final result as to whether a UTI is detected or not. In an alternative example, where the biomarker is detected via a competition assay format as described herein, the lines may be graded (Grade lines 1-10) wherein Grade line 1 is the lightest coloured line followed by Grade line 2 which is more intense in colour and so on to Grade line 10 which is the darkest (i.e. most intensely coloured) line. By virtue of the nature of the assay, a weaker signal at the test line is obtained as the concentration of the biomarker to be detected increases in the urine sample, as the skilled person will appreciate. Thus, in this example, Grade lines 0-4 are calibrated above a pre-determined threshold level and indicate that the specific biomarker is present in abnormally high levels and therefore a UTI is detected whilst Grade lines 5-10, calibrated at or below the pre-determined threshold level, indicate that the specific biomarker is present but within normal (i.e. non-infectious) parameters and therefore a UTI is not detected. In this example, the null grade line (Grade line 0) provided, for which no coloured line is displayed on the visual aid, indicates that the biomarker is present at such a high concentration that all of (or nearly all of) the labelled binding reagent was bound by the biomarker in the sample and therefore was unable to bind at the test line in sufficient concentration to be observable. Again, the user can read the assay results by eye and obtain a final result as to whether a UTI is detected or not.

In other more preferred embodiments, the testing device or testing kit may further comprise a reader to quantify levels of the biomarkers at the respective test lines. The reader may further comprise, consist essentially of or consist of:
a. A processor; and
b. A storage medium comprising a computer application that, when executed by the processor, is configured to:
   i. Access and/or calculate the determined protein level of at least MMP8 in the sample on the one or more testing devices
   ii. Calculate a test score from the protein level of at least MMP8 in the sample that detects a urinary tract infection; and
   iii. Output from the processor the detected result for the subject.

In other embodiments the reader further comprises, consists essentially of or consists of:
a. A processor; and
b. A storage medium comprising a computer application that, when executed by the processor, is configured to:
   i. Access and/or calculate the determined protein level of at least MMP8 in the sample on the one or more testing devices
   ii. Calculate a test score from the protein level of at least MMP8 in the sample by comparing the level with the one taken at one or more earlier time points to thereby detect a urinary tract infection; and
   iii. Output from the processor the detected result for the subject.

The reader device may comprise a housing dimensioned so as to receive the one or more testing device, testing kit or testing composition of matter.

The determined levels may be stored in the reader so as to be accessible when a future sample is tested using a further testing device. Similarly, the levels taken at one or earlier time points may be stored for future access.

The testing device, testing kit or testing composition of matter may comprise some form of unique identifier that can be read by the reader device to thereby assign the results to a particular subject. Alternatively, the user may be able to input an identifier into the reader prior to use thereby identifying the subject. These features are particularly important for monitoring functions, especially where the reader may be used by multiple different subjects.

The test score that is calculated by the reader, according to all relevant aspects, is simply a processing of the comparison of measured levels of biomarker(s) compared to threshold. It permits the overall detected results to be output. The output may be a simple visual indication. It may be a colour coded signal e.g. red indicates a UTI and green indicates no detected UTI. It may be a "UTI" or "OK" type output. It may be a numerical output of levels and the user is required to interpret that output against a scale or chart of values. Combinations of outputs are also possible.

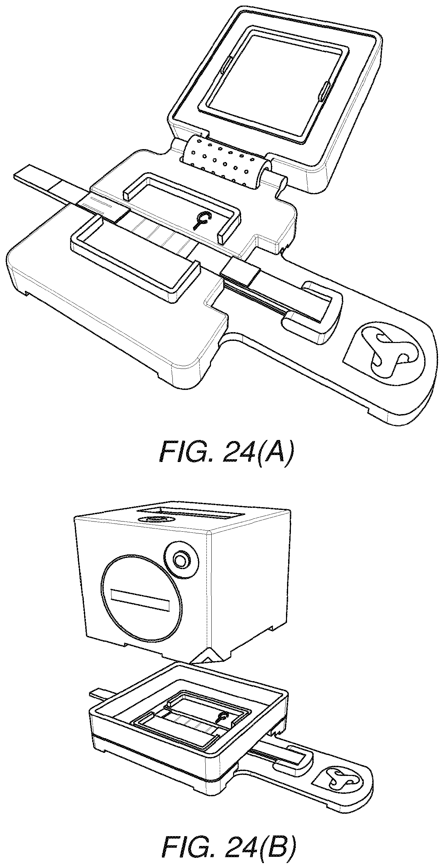

In particular embodiments, the testing device or testing kit comprises a test strip and a reader as described herein along with an adaptor. The adaptor serves to reversibly mount/house both the test strip and reader in order to correctly orientate the reader and test strip relative to one another to enable a reading to be taken. For instance, the adaptor may have a book-type configuration as shown in illustrative Figure 24. Thus, the adaptor may comprise a first and second portion connected by a hinge. The first portion is dimensioned to receive and house the test strip. It may be dimensioned in such a way that the test strip may only be housed in a specific orientation that enables the reader (when present) to take a measurement. The second portion may be rotated about the hinge to sit over the test strip housed in the first portion and sandwich the test strip between the first and second portion such that the test strip is secured in place. The second portion further comprises a transparent window or hole through which at least the solid support region containing the test lines of the test strip can be viewed when the test strip is sandwiched between the first and second portion of the adaptor. In addition, the second portion comprises means to reversibly secure the reader above the secured test strip to enable a reading to be taken. It should be noted that the embodiment of Figure 24 is for illustrative purposes only. Other adaptor configurations may include, for instance, an adaptor in which the first and second portion form a single, fused unit comprising a cavity into which the test strip may be slid into place and secured. Further adaptor configurations will be clear to the skilled person in view of the teachings herein.

In some embodiments according to all relevant aspects, the biomarkers comprise at least MMP8 and optionally HNE, Cystatin C, MMP9, IL-8, interleukin-6 (IL-6), interleukin-1 beta (IL-1b) and MPO or comprise at least MMP8 and optionally HNE, Cystatin C, MMP9, IL-8, interleukin-6 (IL-6), interleukin-1 beta (IL-1b), MPO, fibrinogen and CRP. This may optionally be in combination with at least one of HSA, desmosine and NGAL.

In certain embodiments according to all aspects of the invention, the biomarkers comprise at least MMP8, and may comprise at least MMP8 and at least one of HNE, fibrinogen or CRP, optionally:
(i) both MMP8 and HNE;
(ii) MMP8, HNE and fibrinogen in combination; or
(iii) MMP8, HNE and CRP in combination.

That is to say, in the context of the methods of the invention, the methods preferably comprise determining the protein level of at least MMP8, and may comprise determining the protein levels of at least MMP8 and at least one of HNE, fibrinogen or CRP, optionally:
(i) both MMP8 and HNE;
(ii) MMP8, HNE and fibrinogen in combination; or
(iii) MMP8, HNE and CRP in combination.

In particular embodiments according to all relevant aspects, the biomarkers comprise at least MMP8, and may comprise at least MMP8 and at least one of HNE, fibrinogen or CRP, optionally:
(i) both MMP8 and HNE
(ii) MMP8, HNE and fibrinogen in combination; or
(iii) MMP8, HNE and CRP in combination.

When this is the case, in some embodiments, the test lines for MMP8 (and optionally HNE, fibrinogen and/or CRP) respectively comprise an immobilised further binding reagent that also specifically binds to MMP8 (or HNE, or fibrinogen or CRP) respectively thereby immobilising MMP8 (HNE, fibrinogen and/or CRP) at the respective test line to produce a signal via the labelled binding reagent also specifically bound to MMP8 (HNE, fibrinogen or CRP). This is sometimes called a "sandwich-type" assay format in the art. In further embodiments, the biomarkers additionally comprise Cystatin C. When this is the case, in some embodiments, the test line for Cystatin C comprises an immobilised version of Cystatin C or an analogue thereof able to compete with Cystatin C in the sample for specific binding to the labelled binding reagent. This is sometimes called a "competition-type" assay format in the art. Sandwich-type assays are preferred for all biomarkers as they provide a positive output. Similarly, in alternative embodiments, a competition-type assay format may be employed to detect at least MMP8, optionally both MMP8 and HNE, or at least MMP8 and at least one of HNE, fibrinogen or CRP, optionally MMP8, HNE and fibrinogen (in combination) or MMP8, HNE and CRP (in combination), and/or a sandwich-type assay format may be employed to detect Cystatin C. The skilled person is well able based on the teachings herein and knowledge in the art to decide upon and implement a particular assay format based on, for instance, the level of specificity desired to detect the one or more biomarkers and/or the cost of the assay. In a preferred embodiment, the biomarkers are MMP8, HNE and Cystatin C (in combination). It is shown herein that the assay formats used in the invention are stable over long periods, including at room temperature (and above). In another preferred embodiment, the biomarkers are MMP8, HNE and fibrinogen (in combination). In yet another preferred embodiment, the biomarkers are MMP8, HNE and CRP (in combination).

The subject according to all aspects of the invention is a mammalian subject, typically a human. The subject may be selected as suspected of suffering from a UTI. This may be based on particular symptoms. Symptoms from a lower urinary tract include pain with urination, frequent urination, and feeling the need to urinate despite having an empty bladder. Symptoms of a kidney infection include fever and flank pain usually in addition to the symptoms of a lower UTI. In some cases the urine may appear bloody. In the very old and the very young, symptoms may be vague or non-specific. For monitoring, the subject may already be known to be suffering from a UTI or may be identified as such according to the methods of the invention. Other subject specific information such as pre-existing conditions may also be taken into account when interpreting the results. Combinations of markers may improve specificity of the detection of a UTI in some circumstances. In some embodiments, the subject is otherwise healthy apart from being suspected of suffering from (or suffering from in the case of monitoring) a UTI.

It should be noted that the invention is performed *in vitro* based upon urine samples. Urine samples can typically be obtained by a subject on their own without intervention. The methods of the invention therefore do not include active steps of obtaining a sample for testing from the subject. The use of a urine sample is particularly advantageous from a compliance perspective in view of sample acquisition typically being non-invasive and provides adequate volume for the various testing devices described herein, in particular the lateral flow formats in which multiple biomarkers are determined from a single sample.

The invention relies upon determining protein levels of UTI biomarkers. There are various known techniques by which biomarker levels may be measured. Thus, by biomarker levels is meant the level of expression and/or activity and/or amount and/or concentration of the biomarker. Expression levels of the biomarkers are measured in urine. Expression levels may correlate with activity and can thus be used as a surrogate of activity. Expression levels are measured at the level of protein according to any suitable method. Protein modifications, such as glycosylation may also be relevant and can be measured by any suitable method. Many such methods are well known in the art and include use of mass spectrometry (e.g. MALDI-TOF mass spectrometry).

The protein expression level and/or amount and/or concentration of a biomarker may rely upon a binding reagent such as an antibody or aptamer that binds specifically to the biomarker of interest. The antibody may be of monoclonal or polyclonal origin. Fragments and derivative antibodies may also be utilised, to include without limitation Fab fragments, ScFv, single domain antibodies, nanoantibodies, heavy chain antibodies, aptamers etc. which retain specific binding function and these are included in the definition of "antibody". Such antibodies are useful in the methods of the invention. They may be used to measure the protein level of a particular biomarker (e.g. protein, or in some instances one or more specific isoforms of a protein. The skilled person is well able to identify epitopes that permit specific isoforms to be discriminated from one another).

Methods for generating specific antibodies are known to those skilled in the art. Antibodies may be of human or non-human origin (e.g. rodent, such as rat or mouse) and be humanized etc. according to known techniques (Jones et al., Nature (1986) May 29-Jun. 4;321(6069):522-5; Roguska et al., Protein Engineering, 1996, 9(10):895-904; and Studnicka et al., Humanizing Mouse Antibody Frameworks While Preserving 3-D Structure. Protein Engineering, 1994, Vol.7, pg 805).

In certain embodiments the expression level and/or amount and/or concentration of a biomarker is determined using an antibody or aptamer conjugated to a label. By label is meant a component that permits detection, directly or indirectly. For example, the label may be an enzyme, optionally a peroxidase, or a fluorophore. Gold labels may be utilised, e.g. in the form of colloidal gold.

A label is an example of a detection agent. By detection agent is meant an agent that may be used to assist in the detection of the antibody-biomarker (e.g. protein) complex. Where the antibody is conjugated to an enzyme the detection agent may comprise a chemical composition such that the enzyme catalyses a chemical reaction to produce a detectable product. The products of reactions catalysed by appropriate enzymes can be, without limitation, fluorescent, luminescent, or radioactive or they may absorb or reflect visible or ultraviolet light. Examples of detectors suitable for detecting such detectable labels include, without limitation, x-ray film, radioactivity counters, scintillation counters, spectrophotometers, colorimeters, fluorometers, luminometers, photodetectors and densitometers. In certain embodiments the detection agent may comprise a secondary antibody. The expression level is then determined using an unlabelled primary antibody that binds to the target protein and a secondary antibody conjugated to a label, wherein the secondary antibody binds to the primary antibody.

Additional techniques for determining expression level at the level of protein and/or the amount and/or concentration of a biomarker include, for example, Western blot, immunoprecipitation, immunocytochemistry, mass spectrometry, ELISA and others (see ImmunoAssay: A Practical Guide, edited by Brian Law, published by Taylor & Francis, Ltd., 2005 edition). To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies. Levels of protein may be detected using a lateral flow assay in some embodiments.

Similarly, activity, such as enzymatic activity, may be measured in the urine sample. Enzymatic activity may be measured for example by detecting processing of a substrate, which may be labelled, in the sample. For example, the assay may be a fluorogenic substrate assay. Enzyme activity may be detected using a suitable lateral flow assay. Examples of suitable assay formats include the assays set forth in International Patent Applications WO2009/024805, WO2009/063208, WO2007/128980, WO2007/096642, WO2007/096637, WO2013/156794, WO2015/059487 and WO2013/156795.

"MMP2 and MMP9 activity" is a measure of matrix metalloproteinase activity in the sample. The activity of MMPs can be measured by any suitable technique, examples of which are disclosed above. Preferably, MMP (MMP9 and MMP2) activity is measured using zymography.

According to the invention, determined levels of the biomarkers are compared with a corresponding threshold level. This allows an increase (or decrease) relative to threshold to be identified. Threshold levels of the biomarker, according to all aspects of the invention, may be defined from population studies or be specific to the individual (i.e. personalised). For detecting a UTI, typically, the threshold levels are pre-determined levels based on a population study. Personalised levels may be more relevant to monitoring applications, although monitoring is preferably also achieved by comparison with pre-determined threshold levels. Threshold levels may be set with reference to a training data set comprising samples defined in relation to UTI status. Thus, threshold levels do not need to be measured each time an assay according to the invention is performed. They can be pre-programmed into a reader device or provided for comparative purposes when performing the methods of the invention. Because the threshold levels may vary according to the measuring technique adopted they are not stated as fixed values but can be implemented according to the present invention by one skilled in the art once a specific measuring technique has been selected. Where specific to the individual, the levels may reflect those in a urine sample taken from the subject at an earlier time point. The invention may therefore rely upon a personalised baseline level of the relevant biomarker or biomarkers against which the threshold is calculated. Calculation may be on an on-going basis to coincide with testing. Thus, the threshold may be a rolling threshold derived from the rolling baseline. In this context, it is apparent that levels of the biomarker or biomarkers do not have to be measured in absolute terms and may be measured in absolute or relative terms. The markers simply have to be measured in a manner which permits a comparison to be made with biomarker levels in samples taken at different time points. Alternatively, the threshold level for each marker may be set based on a population analysis. The threshold level may be set to maximise sensitivity and/or specificity of detection as would be readily appreciated by one skilled in the art. Example thresholds are set forth herein for information purposes.

In some embodiments, the threshold level of the biomarker is set by determining the levels of the biomarker in samples taken from the subject at earlier time points. In its simplest form, the invention may rely upon a simple comparison between the test sample and the level of the biomarker in the previously taken sample (i.e. a single earlier time point). However, the earlier time points may comprise at least two, and possibly 3, 4, 5, 6, 7, 8, 9, 10 etc, earlier measurements immediately preceding the determination of the level of the biomarker in the current sample.

Where biomarker levels are measured at multiple time points those levels may be averaged to provide the threshold for the test sample, above which a UTI is detected. In some embodiments, the threshold may be set with reference to a sliding window within which levels of the biomarkers have been measured to provide a baseline. The threshold level is thus "learned" by the system. It is not a fixed threshold and is adapted to the subject, thereby taking into account insignificant fluctuations in biomarker levels from the baseline that are not predictive or indicative of a UTI. Accordingly, the threshold may be set around the baseline to specify an allowable range of the biomarker levels beyond which a statistically significant increase (or decrease) in level is indicated. In the presence of drift of the baseline level of the biomarker, it is possible that the parameter limits may be narrowed such that a further change in level of the biomarkers is deemed significant. For example, if the baseline biomarker level is drifting upwards over time, the difference between a measured increase and baseline may need to be smaller (compared to the situation in which the baseline is relatively stable) to be considered to have exceeded the threshold (i.e. to be significant). For example, a difference from baseline of at least 5, 10, 15, 20% or more may be considered significant generally. This difference may be reduced if there have been multiple previous measurements displaying a trend upwards or downwards but in each case by an amount less than the threshold difference. The difference (in order to be considered significant) may thus be reduced to at least 1, 2, 3, 4, 5% or more as appropriate in the event of a drift upwards or downwards in the baseline.

The threshold may be set in relation to multiple biomarkers as discussed in greater detail herein. Thus, the detection of a UTI may be identified based upon a deviation from baseline that is cumulative according to the multiple biomarkers measured. Typically, however, each biomarker will be measured individually with reference to a biomarker-specific baseline and against a biomarker-specific threshold. It is shown herein that use of multiple individual biomarkers may provide an improved ability to detect a UTI. Thus, the invention may rely upon a plurality of baselines/thresholds depending upon the individual biomarkers employed. The methods and systems may weight the contribution of a plurality of biomarkers.

For those embodiments which rely upon measuring the levels of multiple (e.g. three) biomarkers, the final detection of a UTI requires that the measured levels are integrated, ideally to provide a simply binary result that is readily interpreted. A suitable algorithm may be employed in order to interpret the data from the levels of the at least two or three or more biomarkers and apply it in order to detect a UTI. In some embodiments, the biomarkers levels may be inter-dependent and thus the algorithm is based on this predicted relationship. In certain embodiments, the determined levels of the at least two or three (or more) biomarkers are analysed in a pre-determined sequence to monitor the subject. This may give rise to a decision tree, as explained further herein to detect a UTI. The levels of the first of the multiple biomarkers may influence the subsequent thresholds required for the other biomarkers in order to detect a UTI, as would readily be appreciated by one skilled in the art. The output of the methods may also guide future sampling and treatment of the subject. Thus, in some embodiments, for a given sample, the biomarker levels may be analysed in sequence until a biomarker is found with an increased level (or all biomarkers have been examined). If a biomarker is detected at increased level the further biomarkers may or may not also be assessed to determine if their level is also increased. The likelihood of a UTI may be higher in the event that multiple biomarkers are increased in a sample and the algorithm may account for this in the outcome, e.g. by weighting the observations. Thus, the sample may be "graded" based upon how many of the biomarkers are increased in level compared to threshold. For example, Grade 1 may indicate only one of the biomarkers is increased in the urine sample, Grade 2 may indicate two of the markers is increased etc. Grade 3 or above may detect a UTI. This is not to be confused with the Grade line concept of the illustrative visual aid described herein. Alternatively, the presence of an increased level of one biomarker is enough to detect a UTI but an increased level of one, two or more further biomarkers increases the confidence level of the detection.

In some embodiments, the determined levels of the at least one, two, three or more biomarkers are weighted. Weighting is a well-known method of applying a degree of relative significance to the at least one, two, three or more biomarkers. The algorithm may be a threshold based algorithm as discussed herein.

The levels of the measured biomarkers may be combined using logistic regression, decision tree analysis, neural networks and/or machine learning. Logistic regression analysis involves formulating a statistical model which adds different markers together in a weighted fashion. Similar to linear regression which can be resolved using the equation y=mx+c, logistic regression allows for the addition of multiple biomarkers and only allows for a binary outcome so the y is replaced with the logit (defined as the In(odds) of being in the positive outcome group). Mathematically, the logistic regression equation is logit=(βnXn)+c. This uses quantitative data of all biomarkers in a weighted fashion in the calculation.

In decision tree analysis, an individual is assessed for one biomarker at a time until they reach a terminal node which classifies the patient into the positive or negative outcome group. This uses cut-off values for each individual biomarker and does not necessarily use all biomarkers in the algorithm, depending upon at which point they are categorised. This type of analysis is therefore suitable for embodiments of the invention in which the levels of more than one biomarker are determined.

Use of neural network displays some similarity to logistic regression in that each node is a summation of the input (marker) multiplied by a weighting (beta coefficient). However, summation is performed a number of times; there are a number of nodes and the input to these nodes can be nodes themselves rather than the measured levels. The nodes are first entered at random with random weights, the difference between the expected output and the observed output is then calculated. If it is not 0 (which is likely to be the case) the weightings are altered in the preceding layer and then in the layer before that until the input variables are reached. The outputs are recalculated and the differences are calculated again, and the model weighting readjusted. This can continue indefinitely until the difference in expected and observed outputs is minimal.

The methods of the invention may be performed using systems or test kits as described herein. The methods of the invention may be performed using a testing device or testing kit as described herein.

As used herein, the processor may be comprised within any computer, server, embedded system, or computing system. The computer may include various internal or attached components such as a system bus, system memory, storage media, input/output interface, and a network interface for communicating with a network, for example.

The computer may be implemented as a conventional computer system, an embedded controller, a laptop, a server, a customized machine, any other hardware platform, such as a laboratory computer or device, for example, or any combination thereof. The computing machine may be a distributed system configured to function using multiple computing machines interconnected via a data network or bus system, for example.

The processor may be configured to execute code or instructions to perform the operations and functionality described herein, manage request flow and address mappings, and to perform calculations and generate commands. The processor may be configured to monitor and control the operation of the components in the computing machine. The processor may be a general purpose processor, a processor core, a multiprocessor, a reconfigurable processor, a microcontroller, a digital signal processor ("DSP"), an application specific integrated circuit ("ASIC"), a graphics processing unit ("GPU"), a field programmable gate array ("FPGA"), a programmable logic device ("PLD"), a controller, a state machine, gated logic, discrete hardware components, any other processing unit, or any combination or multiplicity thereof. The processor may be a single processing unit, multiple processing units, a single processing core, multiple processing cores, special purpose processing cores, co-processors, or any combination thereof. According to certain example embodiments, the processor, along with other components of the computing machine, may be a virtualized computing machine executing within one or more other computing machines.

The storage medium may be selected from a hard disk, a floppy disk, a compact disc read only memory ("CD-ROM"), a digital versatile disc ("DVD"), a Blu-ray disc, a magnetic tape, a flash memory, other non-volatile memory device, a solid-state drive ("SSD"), any magnetic storage device, any optical storage device, any electrical storage device, any semiconductor storage device, any physical-based storage device, any other data storage device, or any combination or multiplicity thereof. The storage media may store one or more operating systems, application programs and program modules such as module, data, or any other information. The storage media may be part of, or connected to, the computing machine. The storage media may also be part of one or more other computing machines that are in communication with the computing machine, such as servers, database servers, cloud storage, network attached storage, and so forth.

The storage media may therefore represent examples of machine or computer readable media on which instructions or code may be stored for execution by the processor. Machine or computer readable media may generally refer to any medium or media used to provide instructions to the processor. Such machine or computer readable media associated with the module may comprise a computer software product.

The input/output ("I/O") interface may be configured to couple to one or more external devices, to receive data from the one or more external devices, and to send data to the one or more external devices. Such external devices along with the various internal devices may also be known as peripheral devices. The I/O interface may include both electrical and physical connections for operably coupling the various peripheral devices to the computing machine or the processor. The I/O interface may be configured to communicate data, addresses, and control signals between the peripheral devices, the computing machine, or the processor. The I/O interface may be configured to implement any standard interface, such as small computer system interface ("SCSI"), serial-attached SCSI ("SAS"), fiber channel, peripheral component interconnect ("PCI"), PCI express (PCIe), serial bus, parallel bus, advanced technology attached ("ATA"), serial ATA ("SATA"), universal serial bus ("USB"), Thunderbolt, FireWire, various video buses, and the like. The I/O interface may be configured to implement only one interface or bus technology.

Alternatively, the I/O interface may be configured to implement multiple interfaces or bus technologies. The I/O interface may be configured as part of, all of, or to operate in conjunction with, the system bus. The I/O interface may include one or more buffers for buffering transmissions between one or more external devices, internal devices, the computing machine, or the processor.

The I/O interface may couple the computing machine to various input devices including mice, touch-screens, scanners, electronic digitizers, sensors, receivers, touchpads, trackballs, cameras, microphones, keyboards, any other pointing devices, or any combinations thereof. The I/O interface may couple the computing machine to various output devices including video displays, speakers, printers, projectors, tactile feedback devices, automation control, robotic components, actuators, motors, fans, solenoids, valves, pumps, transmitters, signal emitters, lights, and so forth.

The computing machine may operate in a networked environment using logical connections through the network interface to one or more other systems or computing machines across the network. The network may include wide area networks (WAN), local area networks (LAN), intranets, the Internet, wireless access networks, wired networks, mobile networks, telephone networks, optical networks, or combinations thereof. The network may be packet switched, circuit switched, of any topology, and may use any communication protocol. Communication links within the network may involve various digital or an analog communication media such as fiber optic cables, free-space optics, waveguides, electrical conductors, wireless links, antennas, radio-frequency communications, and so forth.

The processor may be connected to the other elements of the computing machine or the various peripherals discussed herein through the system bus. It should be appreciated that the system bus may be within the processor, outside the processor, or both. According to some embodiments, any of the processor, the other elements of the computing machine, or the various peripherals discussed herein may be integrated into a single device such as a system on chip ("SOC"), system on package ("SOP"), or ASIC device.

Embodiments of the system or test kit may comprise a computer program that embodies the functions described and illustrated herein, wherein the computer program is implemented in a computer system that comprises instructions stored in a machine-readable medium and a processor that executes the instructions. However, it should be apparent that there could be many different ways of implementing embodiments in computer programming. Further, a skilled programmer would be able to write such a computer program to implement one or more of the disclosed embodiments described herein. Therefore, disclosure of a particular set of program code instructions is not considered necessary for an adequate understanding of how to make and use the embodiments. Further, those skilled in the art will appreciate that one or more aspects of embodiments described herein may be performed by hardware, software, or a combination thereof, as may be embodied in one or more computing systems. Moreover, any reference to an act being performed by a computer should not be construed as being performed by a single computer as more than one computer may perform the act.

The example embodiments described herein can be used with computer hardware and software that perform the methods and processing functions described previously. The systems, methods, and procedures described herein can be embodied in a programmable computer, computer-executable software, or digital circuitry. The software can be stored on computer-readable media. For example, computer-readable media can include a floppy disk, RAM, ROM, hard disk, removable media, flash memory, memory stick, optical media, magneto-optical media, CD-ROM, etc. Digital circuitry can include integrated circuits, gate arrays, building block logic, field programmable gate arrays (FPGA), etc.

The methods, systems, test kits, testing devices, testing kits and testing compositions of matter may incorporate means for Automatic Identification and Data Capture (AIDC), such as a Radio-frequency identification tag or card (RIF)

For the avoidance of doubt, the discussion of the invention hereinabove applies, in addition to the methods, to the systems, test kits, testing devices, and testing kits of the invention and all embodiments can be applied accordingly. However, for clarity and by way of exemplification of how the discussion applies directly to the systems, test kits, testing devices, and testing kits, further specific embodiments are outlined below.

In some embodiments, the systems, test kits, testing devices, and testing kits take the form of a portable system. An example system upon which the various products of the invention may be based is the Alere^{™} DDS^{®}2 mobile test system. This system comprises an analyser, into which a test cartridge is inserted. The user then also inserts a sample collection device into the analyser. The analyser incorporates a full colour screen to read the results. The analyser thus houses the processor and storage medium which permits the assays to be run. The test cartridge represents the one or more testing devices for determining levels of the at least one, two, three (or more) biomarkers. The systems or test kits of the invention may incorporate a separate sample collection device or this may be integrated into the one or more testing devices.

In specific embodiments, the systems, test kits, testing devices, and testing kits further comprise a display for the output from the processor. This is intended to give a simple visual and/or audible read-out of the assays performed on the sample. The display may be operably connected to the processor running the computer application. The output or read-out may be an instruction to the subject in some embodiments. The output may be colour coded or numerical to reflect the various possible outcomes of monitoring as discussed herein. It is possible for the display to provide levels of the markers measured in the sample and provide suitable training and/or documentation to assist the user in interpretation of the data. However, this is not preferred for obvious reasons of susceptibility to human error. A combination of both types of information may, however, be presented in some embodiments. Thus, the display may present both quantitative and qualitative read-outs in some embodiments. Probability values related to the predictive and identification outcomes may also represent an output in some embodiments. The display is typically an integral part of the reader device.

In specific embodiments, the one or more testing devices, or testing kits comprise disposable single use devices to which the sample is applied. Typically the one or more testing devices, or testing kits may comprise a sample receiving zone to which the sample is added. The devices typically also incorporate a solid support which defines a liquid/capillary flow path for the sample once applied to the sample receiving zone. The sample receiving zone may be an integral part of the solid support. The solid support may comprise a chromatographic medium, such as a membrane material in some embodiments (e.g. nitrocellulose). A sample applied to the sample application zone will typically rehydrate the necessary reagents to detect the marker. The reagents include binding reagents which specifically interact with the biomarkers or a substrate for effector molecules where activity is measured. Further reagents immobilized further along the flow path bind to the complex of biomarker and binding reagent. The binding reagent is labelled to provide a signal at the site of immobilization of the complex of biomarker and binding reagent (through binding to the further reagent). Suitable labels include fluorescent labels, magnetic labels, latex or gold as would be readily understood by one skilled in the art.

When enzymatic activity is being assayed the binding reagent and/or further binding reagent may bind with a substrate only after it has been modified by the enzymatic activity, or may only bind if the substrate has not been modified by the enzymatic activity. Examples of enzymatic activity assays include those set forth in International Patent Applications WO2009/024805, WO2009/063208, WO2007/128980, WO2007/096642, WO2007/096637, WO2013/156794, WO2015/059487 and WO2013/156795.

The binding reagent and further reagent are typically antibodies (as defined herein). Thus, in specific embodiments, the one or more testing devices, or testing kits may comprise a lateral flow test strip. In some embodiments, a single lateral flow test strip is employed to permit detection of all biomarkers that are to be determined in the test sample. In other embodiments, a separate lateral flow test strip is provided for each biomarker that is determined.

The devices or kits may also include a control zone to confirm sample has passed through the device satisfactorily. In the event this is not the case the system or test kit or reader of the testing device, or testing kit may indicate an invalid result to the user, for example via the display. The devices, or kits may act as competitive or sandwich assays, as discussed herein. ELISA (enzyme linked immunosorbent assay) is an example of a suitable assay format that may be incorporated in the testing devices used in the invention. Again, typically all reagents to detect the protein level of at least MMP8 and optionally at least one or more biomarkers are pre-loaded onto the testing device or kit such that they can interact with the sample once added to the device (for example via the sample receiving zone). This minimizes intervention and thus error caused by the subject. Thus, effectively, the device may only require the user to apply the sample and subsequently observe the output of the assay.

The systems, test kits, and testing devices may incorporate a suitable reader to provide a quantitative output (in conjunction with the processor and storage medium). As already mentioned this output can be an absolute or a relative output. Suitable readers may incorporate an illuminator to expose the device to a specific wavelength or wavelengths of light and a suitable detector for the reflected or emitted light. The systems, test kits, and testing devices may also incorporate a suitable processor and computer application to output the results based upon the detected signal. Thus, the processor running the computer application will be in operable connection with the reader. By "operable connection" is meant a functional connection that permits the exchange of a signal or information between the elements.

As discussed above, where protein levels are measured the binding reagent may comprise an antibody (to include derivatives, fragments and aptamers).

The one or more testing devices or kits may comprise an enzyme detection device. These devices may be particularly useful for investigating enzymatic activity.

The system or test kit may incorporate the appropriate number of testing devices to permit each biomarker to be determined. This is particularly the case where the biomarkers are detecting using different platforms. Thus, in some embodiments, the one or more testing devices comprise one or more lateral flow activity assays, ELISAs, fluorogenic substrate assays etc. In some embodiments, the one or more testing devices comprise one or more lateral flow activity assays, ELISAs or competition assays. In some embodiments, the one or more testing devices comprise one or more lateral flow assays and ELISAs.

In certain embodiments, the computer application is configured to output from the processor detection of a UTI if an increase in the protein level of at least MMP8 is calculated. In specific embodiments, the output is an indication that the subject should receive treatment.

### DESCRIPTION OF THE FIGURES

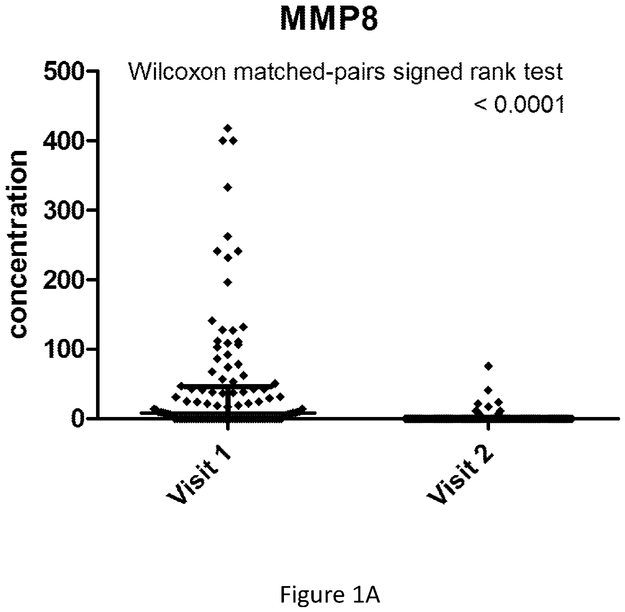

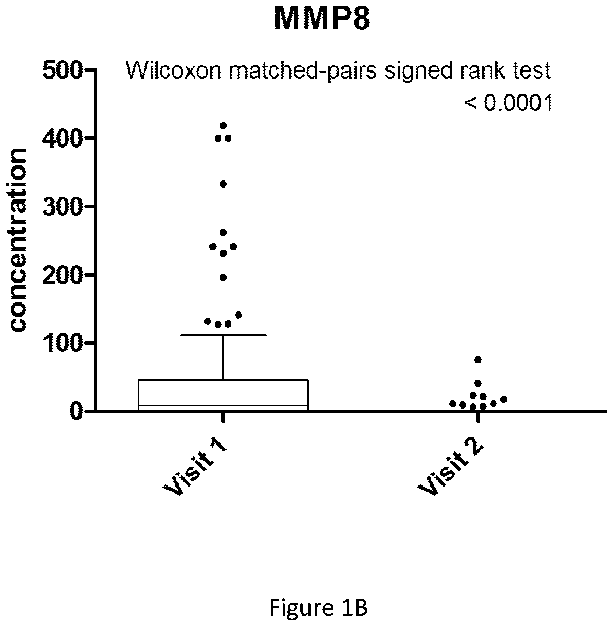

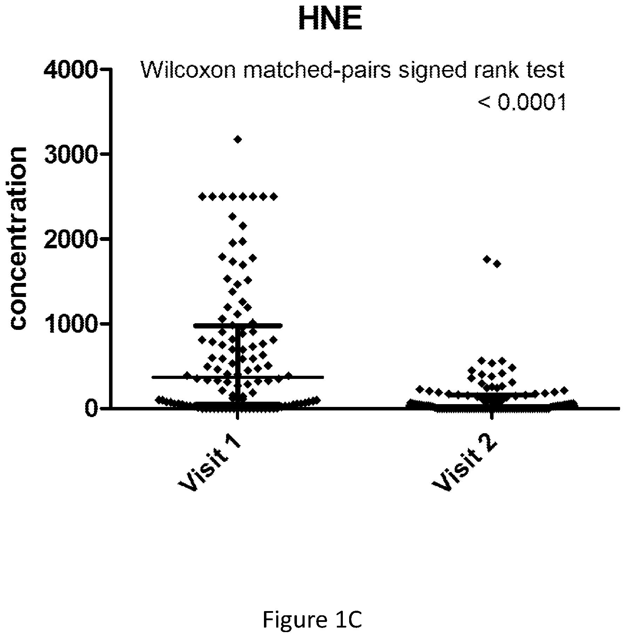

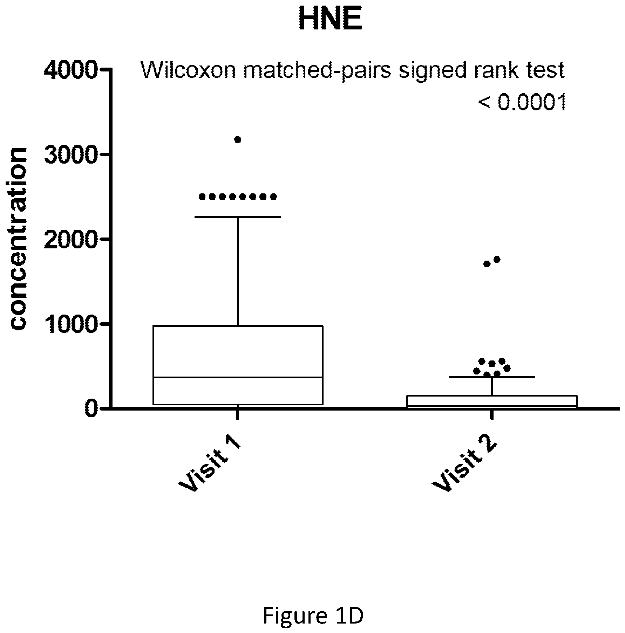

**Figure** 1 - Concentration of MMP8 (Figure 1A and 1B) and HNE (Figure 1C and 1D) in samples taken at a first visit (i.e. at the time of presenting with acute symptoms - "Suspected UTI") and a second visit (i.e. typically after symptoms have been resolved) from 112 patients. Samples taken at Visit 1 and Visit 2 from each patient were assessed for statistical significance in relation to the levels of MMP8 in each sample using a Wilcoxon matched-pairs signed rank test. The same data is shown using two different statistical plots (1A and 1C are scatter plots) and (1B and 1D respectively are box plots).

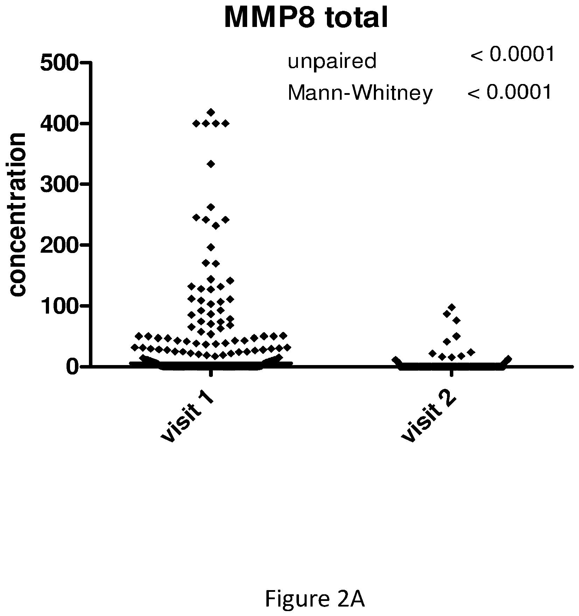

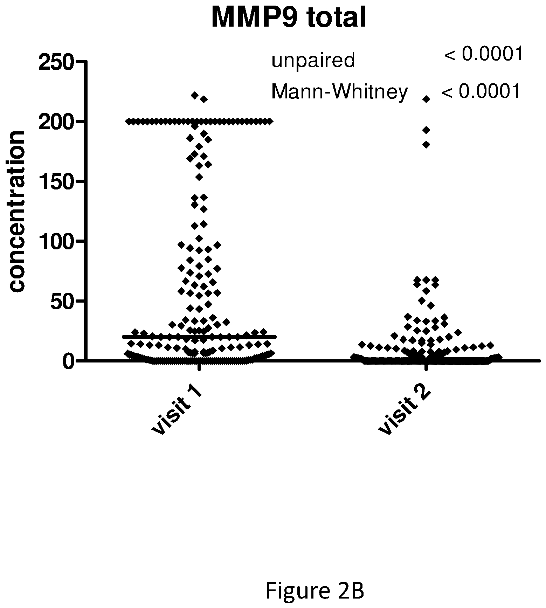

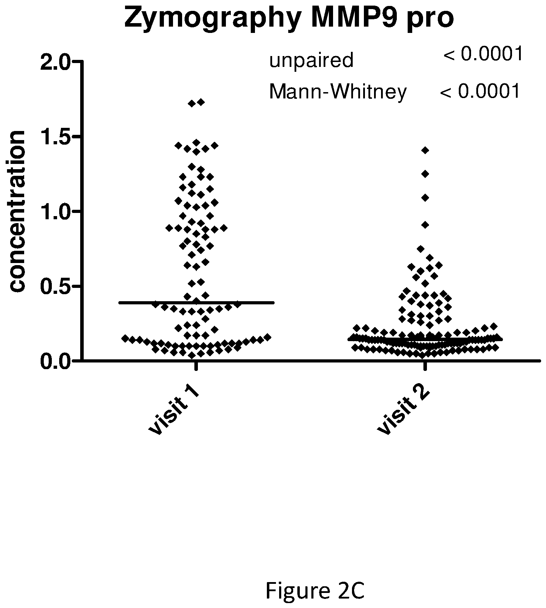

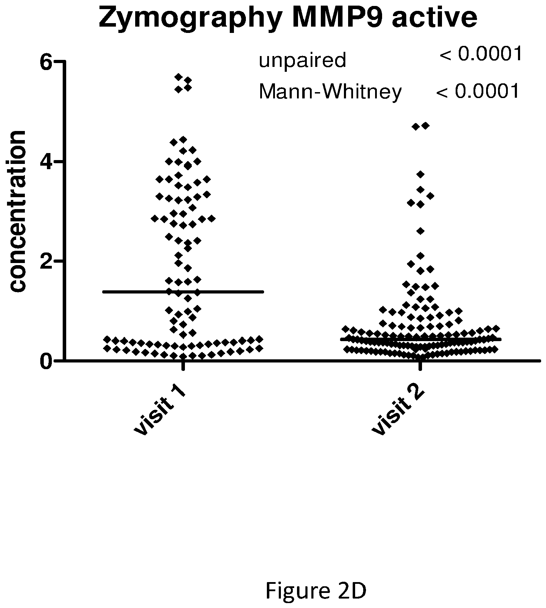

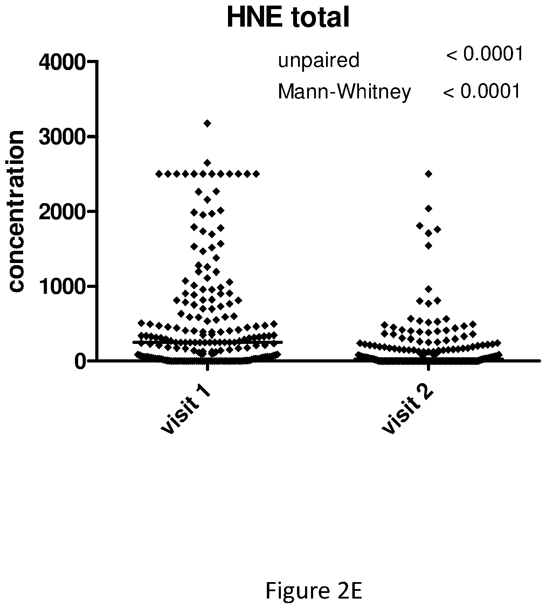

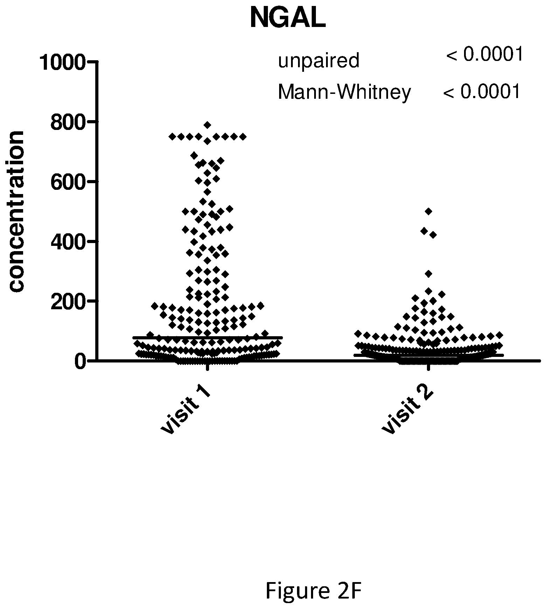

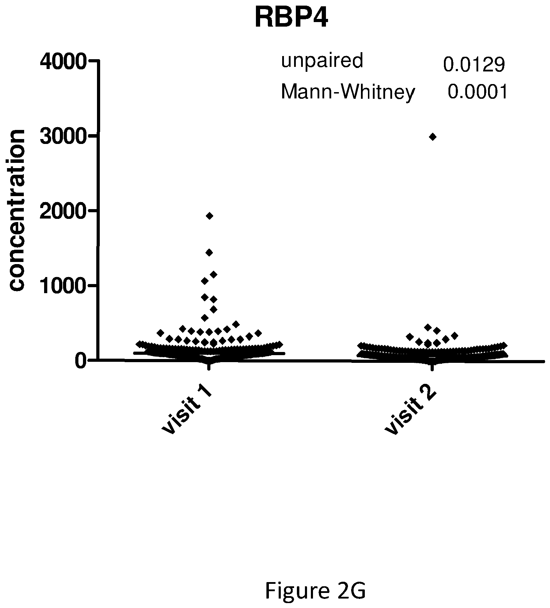

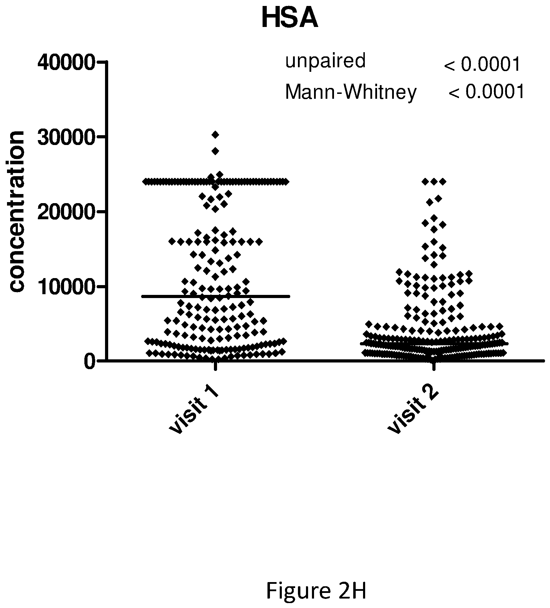

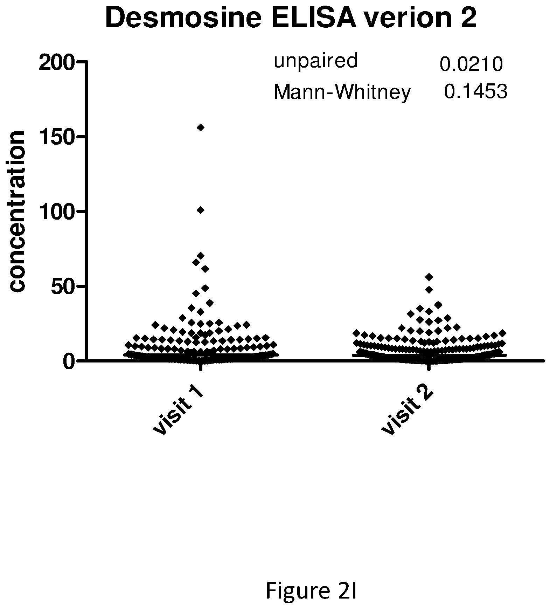

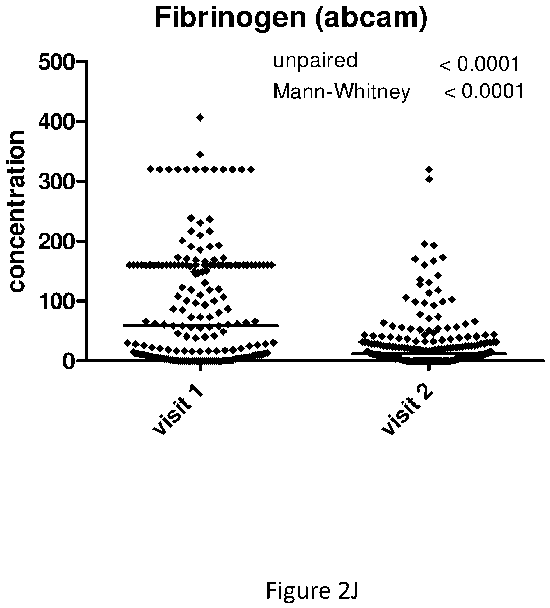

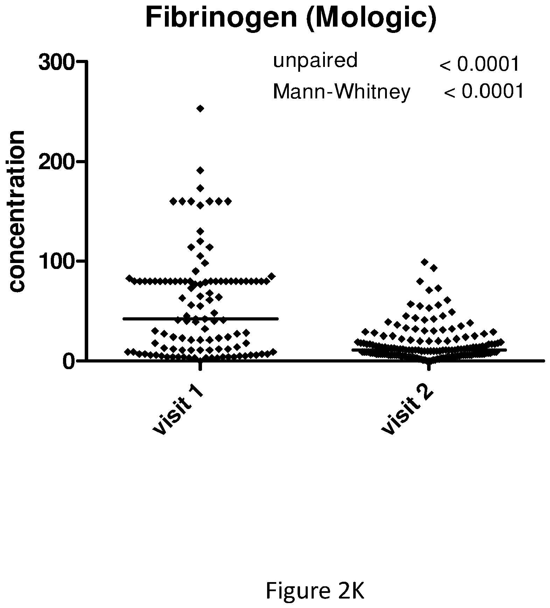

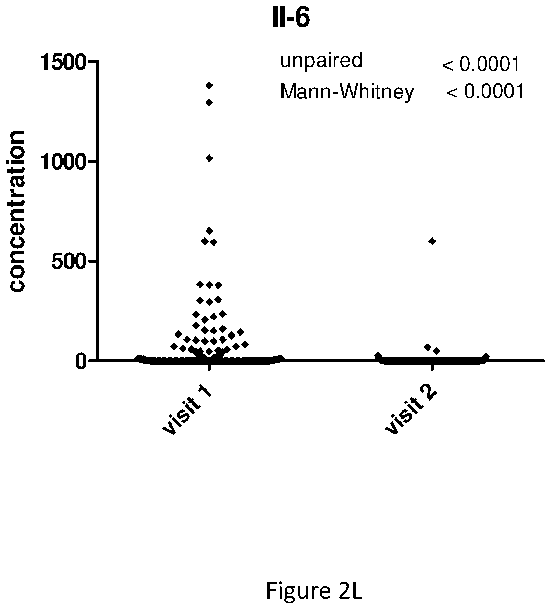

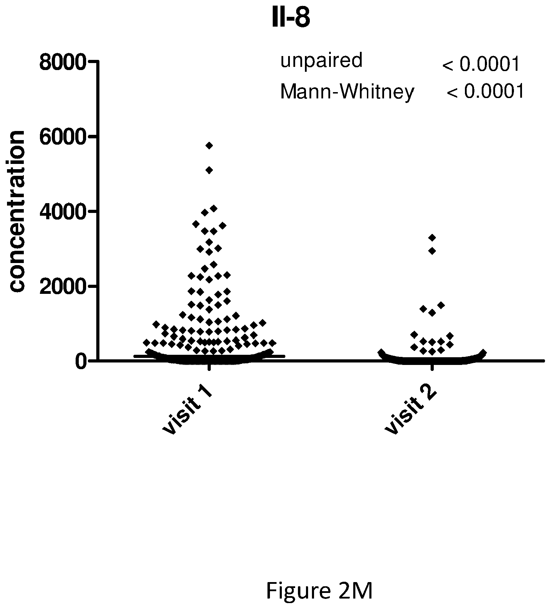

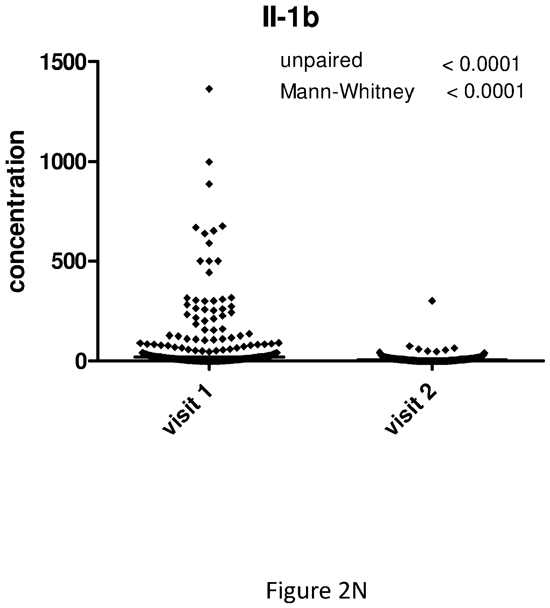

**Figure 2** - Concentration of MMP8 (Figure 2A), MMP9 (Figure 2B), active MMP (Figures 2C and 2D), HNE (Figure 2E), NGAL (Figure 2F), RBP4 (Figure 2G), HSA (Figure 2H), desmosine (Figure 2I), Fibrinogen (Figure 2J and 2K), IL-6 (Figure 2L), IL-8 (Figure 2M), IL-1b (Figure 2N) in samples taken at a first visit (i.e. at the time of presenting with acute symptoms - "Suspected UTI") and a second visit (i.e. typically after symptoms have been resolved) from over 200 patients. Samples taken at Visit 1 and Visit 2 from each patient were assessed for statistical significance in relation to the levels of each marker in each sample using a Wilcoxon matched-pairs signed rank test.
**Figure 3** - Receiver operating curve for MMP8, MMP9, HNE, HSA and IL-8 as biomarkers for a UTI in samples from 112 patients that had both a visit 1 and a visit 2 sample taken. Blue = MMP8; Green = MMP9; Dark yellow = HNE; Purple = HSA; Light yellow = IL-8. Diagonal segments are produced by ties. Area under the curve (AUC) values are shown for all 5 biomarkers.
**Figure 4** **-** Receiver operating curve for MMP8, MMP9, HNE, HSA and IL-8 as biomarkers for a UTI in samples from 93 patients for whom the visit 1 sample gave a positive HNE result with respect to detection of a UTI. Blue = HNE; Green = MMP8; Dark yellow = MMP9; Purple = HSA; Light yellow = IL-8. Diagonal segments are produced by ties. Area under the curve (AUC) values are shown for all 5 biomarkers.
**Figure 5** - Correlation between the lateral flow assay and ELISA assay for HNE detection
**Figure 6** - Correlation between the lateral flow assay and ELISA assay for MMP8 detection
**Figure 7** - Correlation between the lateral flow assay and ELISA assay for Cystatin C detection
**Figure 8** - MMP8: Wilcoxon matched-pairs signed rank test for UTI positive visit 1 and visit 2 patients (Figure 8A-C) along with ROC analysis and AUC values (Figure 8D).
**Figure** 9 - MMP8: Wilcoxon matched-pairs signed rank test for UTI negative visit 1 and visit 2 patients (Figure 9A-C) along with ROC analysis and AUC values (Figure 9D).
**Figure 10** - HNE: Wilcoxon matched-pairs signed rank test for UTI positive visit 1 and visit 2 patients (Figure 10A-C) along with ROC analysis and AUC values (Figure 10D).
**Figure 11** - HNE: Wilcoxon matched-pairs signed rank test for UTI negative visit 1 and visit 2 patients (Figure 11A-C) along with ROC analysis and AUC values (Figure 11D).
**Figure 12** - Cystatin C: Wilcoxon matched-pairs signed rank test for UTI positive visit 1 and visit 2 patients (Figure 12A-C) along with ROC analysis and AUC values (Figure 12D).
**Figure 13** - Cystatin C: Wilcoxon matched-pairs signed rank test for UTI negative visit 1 and visit 2 patients (Figure 13A-C) along with ROC analysis and AUC values (Figure 13D).
**Figure 14** - MMP8 + HNE: Wilcoxon matched-pairs signed rank test for UTI positive visit 1 and visit 2 patients (Figure 14A-C) along with ROC analysis and AUC values (Figure 14D).
**Figure 15** - MMP8 + HNE: Wilcoxon matched-pairs signed rank test for UTI negative visit 1 and visit 2 (Figure 15A-C) patients along with ROC analysis and AUC values (Figure 15D).
**Figure 16** - MMP8, HNE + Cystatin C: Wilcoxon matched-pairs signed rank test for UTI positive visit 1 and visit 2 (Figure 16A-C) patients along with ROC analysis and AUC values (Figure 16D).
**Figure 17** - MMP8, HNE + Cystatin C: Wilcoxon matched-pairs signed rank test for UTI negative visit 1 and visit 2 patients (Figure 17A-C) along with ROC analysis and AUC values (Figure 17D).
**Figure 18** - MMP8: Wilcoxon matched-pairs signed rank test for UTI positive and negative samples (confirmed by microbiological testing) (Figure 18A and 18B) along with ROC analysis and AUC values (Figure 18C).
**Figure 19** - HNE: Wilcoxon matched-pairs signed rank test for UTI positive and negative samples (confirmed by microbiological testing) (Figure 19A and B) along with ROC analysis and AUC values (Figure 19C).
**Figure 20** - Cystatin C: Wilcoxon matched-pairs signed rank test for UTI positive and negative samples (confirmed by microbiological testing) (Figure 20A and B) along with ROC analysis and AUC values (Figure 20C).
**Figure 21** - MMP8 + HNE: Wilcoxon matched-pairs signed rank test for UTI positive and negative samples (confirmed by microbiological testing) (Figure 21A and B) along with ROC analysis and AUC values (Figure 21C).
**Figure 22** - MMP8, HNE + Cystatin C: Wilcoxon matched-pairs signed rank test for UTI positive and negative samples (confirmed by microbiological testing) (Figure 22A and B) along with ROC analysis and AUC values (Figure 22C).

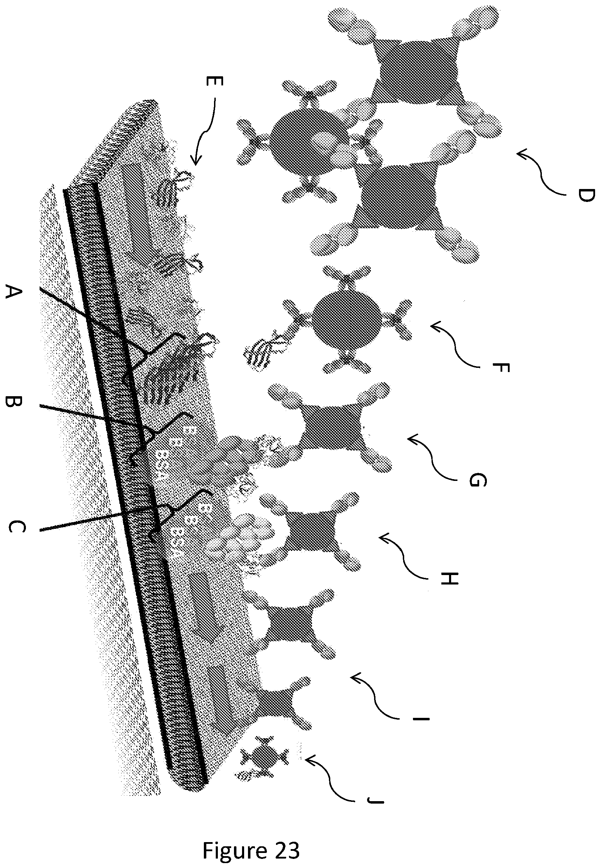

**Figure 23** - Exemplary test strip of the invention and use thereof. Blue arrows indicate direction of flow along the test strip. (A) Cystatin C immuno-capture test line. (B) Anti-HNE antibody immuno-capture test line. (C) Anti-MMP8 antibody immuno-capture test line. (D) Gold particles bearing anti-MMP8 antibody, anti-HEN antibody or anti-Cystatin C antibody. (E) MMP8, HNE or Cystatin C present in a urine sample obtained from a subject. (F) Antibodies bound to gold particles capture Cystatin C and prevent it binding to the Cystatin C immuno-capture test line. (G) Antibodies bound to gold particles capture HNE and bind to the anti-HNE antibody immuno-capture test line. (H) Antibodies bound to gold particles capture MMP8 and bind to the anti-MMP8 antibody immuno-capture test line. (I) Anti-HNE and anti-MMP8 antibodies bound to gold particles but which have not bound to HNE and MMP8 respectively escape the capture test line. (J) Anti-Cystatin C antibodies bound to gold particles and also bound to Cystatin C escape the capture test line. BSA - bovine serum albumin.
**Figure 24** - (A) An exemplary test strip is placed in an adaptor for a reader. (B) A reader is placed above the test strip appropriately housed in the adaptor for the reader.
**Figure 25** - Standard curves for (A) MMP8, (B) HNE and (C) Cystatin C present in a sample determined according to the invention.
**Figure 26** - Quantification of MMP8 in fresh and frozen urine samples according to the methods and apparatus of the invention.
**Figure 27** - Quantification of HNE in fresh and frozen urine samples according to the methods and apparatus of the invention.
**Figure 28** - Quantification of Cystatin C in fresh and frozen urine samples according to the methods and apparatus of the invention.
**Figure 29** - UTRiPLEX assay variability with urine samples taken over 5 days from a first healthy volunteer
**Figure 30** - UTRiPLEX assay variability with urine samples taken over 5 days from a second healthy volunteer
**Figure 31** - UTRiPLEX assay variability with urine samples taken over 5 days from a third healthy volunteer
**Figure 32** - UTRiPLEX assay variability with urine samples taken over 5 days from a fourth healthy volunteer
**Figure 33** - UTRiPLEX assay variability with urine samples taken over 5 days from a fifth healthy volunteer
**Figure 34** **-** UTRiPLEX assay variability with urine samples taken over 5 days from a sixth healthy volunteer
**Figure 35** - UTRiPLEX assay variability with urine samples taken over 5 days from a seventh healthy volunteer
**Figure 36** - UTRiPLEX assay variability with urine samples taken over 5 days from a eighth healthy volunteer
**Figure 37** - UTRiPLEX assay variability with a urine sample from a first healthy volunteer stored either at room temperature or 4°C.
**Figure 38** - UTRiPLEX assay variability with a urine sample from a first healthy volunteer stored either at room temperature or 4°C as shown in Figure 37 shown per each biomarker individually
**Figure 39** - UTRiPLEX assay variability with a urine sample from a second healthy volunteer stored either at room temperature or 4°C.
**Figure 40** - UTRiPLEX assay variability with a urine sample from a second healthy volunteer stored either at room temperature or 4°C as shown in Figure 39 shown per each biomarker individually
**Figure 41** - UTRiPLEX assay variability with a urine sample from a third healthy volunteer stored either at room temperature or 4°C.
**Figure 42** - UTRiPLEX assay variability with a urine sample from a third healthy volunteer stored either at room temperature or 4°C as shown in Figure 41 shown per each biomarker individually
**Figure 43** - Daily monitoring of a subject suffering from a UTI (based on positive culture results) over 43 days. The levels of MMP8, HNE and Cystatin C as measured via UTRiPLEX are shown. Also shown are leukocyte levels and nitrite levels in each sample measured using known Multistix tests. Leukocyte levels were graded: negative (neg), Trace, Small and Large. Nitrite levels were graded: + (present) and - (absent).
**Figure 44** - Results of HNE stability studies at 4°C (Figure 44A), room temperature (Figure 44B) and 37°C (Figure 44C); represented as average calculated reader values.
**Figure 45** - Results of MMP8 stability studies at 4°C (Figure 45A), room temperature (Figure 45B) and 37°C (Figure 45C); represented as average calculated reader values.
**Figure 46** - Results of Cystatin C stability studies at 4°C (Figure 46A), room temperature (Figure 46B) and 37°C (Figure 46C); represented as average calculated reader values.
**Figure 47** - ROC curves for each of CRP, HNE, MMP-8 and fibrinogen when used to detect a urinary tract infection pre-boric acid addition (Figure 47A) and post-boric acid addition (Figure 47B).
**Figure 48** - Decision tree analysis for detecting a urinary tract infection using the combination of CRP + HNE + MMP-8 pre-boric acid addition (Figure 48A) and post-boric acid addition (Figure 48B).

### EXAM PLES

The invention will be further understood with reference to the following experimental examples.

### Example 1 - Selection of Biomarkers

Samples were collected as part of an I4I funded project, whereby a total of 189 urine samples from adult women presenting with suspected uncomplicated UTI were fully analysed with respect to 60 biomarkers.

There were two objectives:
i. To define a panel of biomarkers to distinguish between negative and positive culture
ii. To select a biomarker that provided the best discrimination between the suspected UTI samples and those from patients who have recovered (healthy).

In order to distinguish between patients presenting with different types of infection, binary logistic regression was used to predict which biomarkers are significant for outcome on their own, without interaction with other parameters. The markers that were significant in this analysis were then analysed further using the Stuttgart Neural Network Simulation (RSNNS) in R where the biological markers significant on their own were weighed against each other and interactions taken into consideration. The results were visualised by drawing Receiver operating curves (ROC), and areas under the curve (AUC) were calculated as a measure of how well the selected markers predict outcome in a given case. First-generation fingerprints are available for the discrimination of UTI samples according to their microbiological profile (2 distinct outcomes: negative, positive). Whilst other combinations were possible, the combinations selected for further study were based on available reagents.
Marker 1 = HNE
Marker 2 = Cystatin C
Marker 3 = MMP8

AUCs were measured for different combinations of markers 1-3 and are shown in the table below:

| **Combination of Markers** | **AUC** |
|---|---|
| Markers 1 + 2 | 0.857826 |
| Markers 1 + 3 | 0.915173 |
| Markers 1 + 2 + 3 | 0.922939 |

These statistical analyses suggest that a minimum number of 2 biomarkers is sufficient to constitute diagnostically relevant immune fingerprints able to detect a UTI.

In an independent analysis, we also looked at biomarkers that would be able to discriminate between urine samples taken at visit 1 (i.e. at the time of presenting with acute symptoms - 'suspected UTI') and two weeks later at visit 2 (i.e. typically after symptoms have been resolved).

A cross sectional analysis of the population, not taking into account individual personalised threshold values was conducted. Non parametric tests - Mann-Whitney and unpaired t-tests were used and receiver operator characteristic to determine the discrimination accuracy.
Visit 1- 202 samples
Visit 2- 222 samples

| Assay | Method of testing | Unit | Unpaired t test | Mann-Whitney | AUC |
|---|---|---|---|---|---|
| MMP9 | ELISA | ng/mL | 8.09987E-20 | <0.0001 | 0.7559 |
| MMP8 | ELISA | ng/mL | 5.60E-11 | <0.0001 | 0.75 |
| IL1b | ELISA | pg/mL | 4.40954E-10 | <0.0001 | 0.7344 |
| Fibrinogen | ELISA | ng/mL | 1.92498E-16 | <0.0001 | 0.7304 |
| H.S.A | ELISA | ng/mL | 3.62118E-21 | <0.0001 | 0.7302 |
| NGAL | ELISA | ng/mL | 1.29098E-18 | <0.0001 | 0.7264 |
| IL8 | ELISA | pg/mL | 2.6059E-11 | <0.0001 | 0.7222 |
| HNE | ELISA | ng/mL | 1.49814E-12 | <0.0001 | 0.7173 |
| Active MMP | Zymography | Arbitrary Unit | 1.73818E-10 | <0.0001 | 0.677 |
| IL6 | ELISA | pg/mL | 2.87862E-05 | <0.0001 | 0.6238 |
| RBP4 | ELISA | ng/mL | 0.012862881 | 0.0001 | 0.6076 |
| Desmosine | ELISA | ng/mL | 0.020936351 | 0.1453 | 0.5427 |

Urinary biomarkers were assessed in 112 patients that had both a visit 1 and a visit 2 sample and were analysed using a paired t test and receiver operator characteristic (see Figures 1-3). Amongst the markers that were highly significant in this context featured marker 1 and 3, with p<0.0001 in paired t-tests and with AUC values of 0.75-0.80 on their own suggesting that, in fact, a minimum number of at least one of the selected biomarkers is sufficient to detect a UTI.

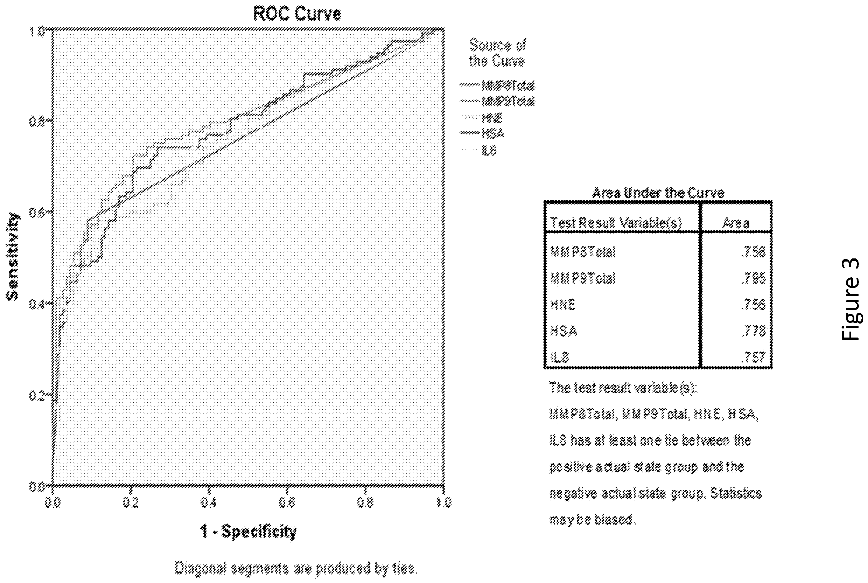

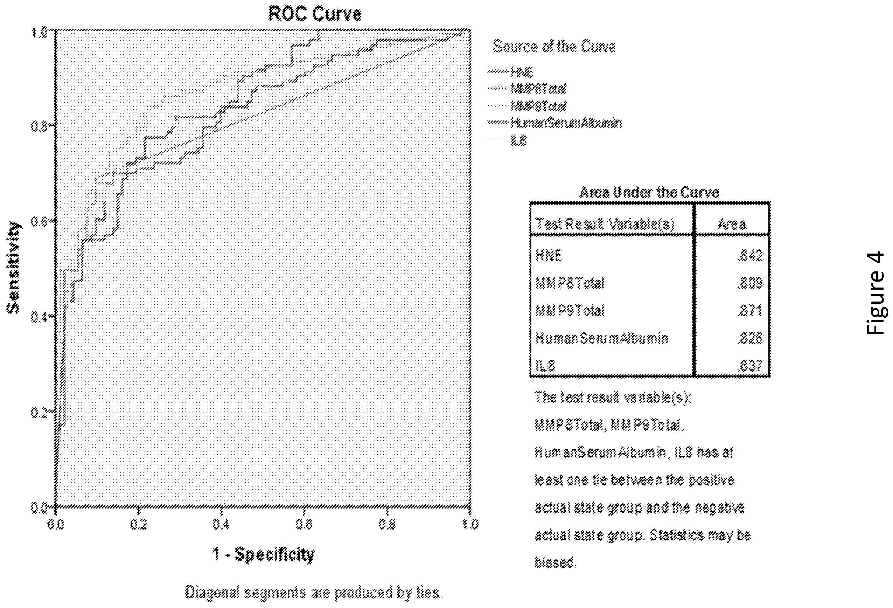

The AUC for each biomarker shown in Figure 3 was as follows: MMP8 = 0.756; MMP9 = 0.795; HNE = 0.756; HSA = 0.778; IL-8 = 0.757. In a further analysis, a ROC was plotted basing the results on HNE i.e. omission of samples with a negative HNE visit 1 (n = 93). This is shown in Figure 4. The AUC for each biomarker shown in Figure 4 was as follows: MMP8 = 0.809; MMP9 = 0.871; HNE = 0.842; HSA = 0.826; IL-8 = 0.837.

Amongst the markers that were highly significant between acute symptoms and recovery, Human Neutrophil Elastase (HNE) and Matrix Metalloproteinase-8 (MMP8) was selected with p<0.0001 from a paired t test and an ROCAUC of 0.76.

Based on this agreement between the two different analyses, one looking at discrimination between negative and positive samples at visit 1 and one looking at discrimination between pre-treatment and post-treatment, it was decided to take biomarkers forward that were identified by both methods.

### Example 2 - Development of lateral flow assays

The three selected biomarkers HNE, MMP8 and Cystatin C were taken forward for lateral flow development. The three lateral flow assays were developed and optimised enclosed into a single well plastic housing supplied by Bibby sterylin using a standard lateral flow form.

### HNE Lateral flow

Preparation of materials: The capture line, anti-HNE BSA-PEG Fab (Alere San Diego, Cat No 01241) and control line BSA-Biotin were immobilised onto the nitrocellulose membrane (Sartorius, CN140) at 1mg/mL in PBS + 1% Sucrose using the Imagene Isoflow dispenser. Membranes were subsequently dried in a tunnel dryer (Hedinair) at 60°C and stored with desiccant prior to use.

The gold conjugates were prepared according to known protocols. Anti-HNE BSA-PEG Fab (Alere San Diego, Cat No 01871) was conjugated to 40nm gold colloid in a suspension buffer of 20mM MES pH5.3 to a final concentration of 15µg/mL. Following a 10-minute incubation, any unbound colloid was blocked with a final concentration of 2mg/mL bovine serum albumin (200mg/mL stock in PBS). Both test conjugate and control gold: Goat anti Biotin gold 40nm (BBI) were mixed together in the presence of a gold drying buffer, Tris with BSA, 5% (w/v) sucrose and 2% (v/v) TritonX100. The gold conjugate was sprayed onto Ahlstrom 8951 glass fibre pads using the Imagene Isoflow dispenser deposited at 0.8µl/mm. The sprayed conjugate pads were dried in a tunnel dryer (Hedinair) at 60°C and stored with desiccant prior to use.

Both prepared membranes and conjugate pads were assembled into one-well plastic cassettes.

Running of the assay: The sample was diluted 1 in 100 in sample diluent (Tris with BSA, 1% (v/v) Tween20). After dilution, 80µL of the sample was added to the sample well of the device and the result was read after 10 minutes.

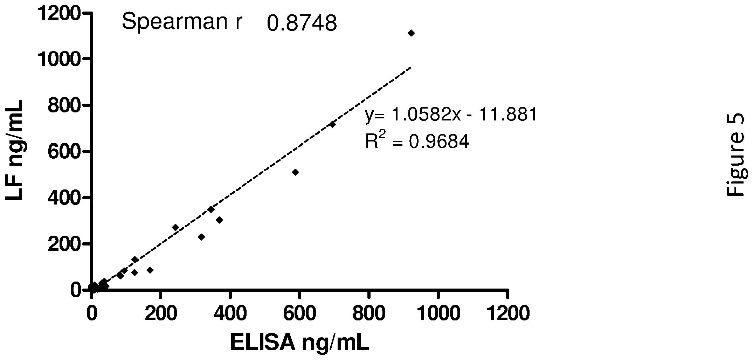

The HNE lateral flow assay performance was compared to a HNE ELISA (Mologic BHNEV1) by running a standard curve and 40 UTI urine samples run at a 1:100 dilution in TBST (tris-buffered saline with Tween 20). Data was analysed according to a Four Parameter Logistic Fit. The correlation between the lateral flow assay and ELISA assay in terms of HNE detection is shown in Figure 5. A strong positive correlation (R² = 0.9684) was observed.

### MMP8 Lateral flow

Preparation of materials: The capture line, anti-MMP8 BSA-PEG Fab (Alere San Diego, Cat No 99611) and control line BSA-Biotin were immobilised onto the nitrocellulose membrane (Sartorius, CN140) at 1mg/mL in PBS + 1% Sucrose using the Imagene Isoflow dispenser. Membranes were subsequently dried in a tunnel dryer (Hedinair) at 60°C and stored with desiccant prior to use.

The gold conjugates were prepared according to known protocols. Anti-MMP8 BSA-PEG Fab (Alere San Diego, Cat No 99651) was conjugated to 40nm gold colloid in a suspension buffer of 20mM BES pH6.6 to a final concentration of 15µg/mL. Following a 10-minute incubation, any unbound colloid was blocked with a final concentration of 2mg/mL bovine serum albumin (200mg/mL stock in PBS). Both test conjugate and control gold: Goat anti Biotin gold 40nm (BBI) were mixed together in the presence of a gold drying buffer, Tris with BSA, 5% (w/v) sucrose and 2% (v/v) TritonX100. The gold conjugate was sprayed onto Ahlstrom 8951 glass fibre pads using the Imagene Isoflow dispenser deposited at 0.8µl/mm. The sprayed conjugate pads were dried in a tunnel dryer (Hedinair) at 60°C and stored with desiccant prior to use.

Both prepared membranes and conjugate pads were assembled into one-well plastic cassettes.

Running of the assay: The sample was diluted 1 in 10 in sample diluent (Tris with BSA, 1% (v/v) Tween20). After dilution, 80µL of the sample was added to the sample well of the device and the result was read after 10 minutes.

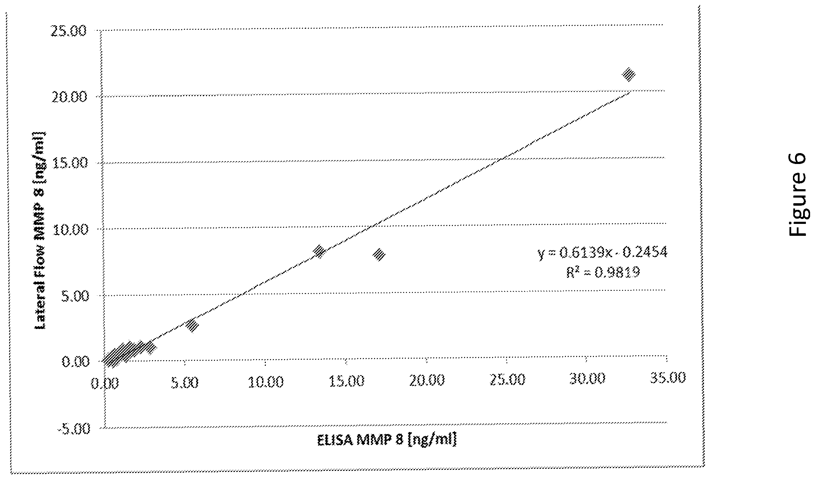

The MMP8 lateral flow assay performance was compared to the commercially available R&D duoset MMP8 ELISA by running a standard curve and 40 UTI urine samples run at a 1:10 dilution in TBST. Data was analysed according to a Four Parameter Logistic Fit. The correlation between the lateral flow assay and ELISA assay in terms of MMP8 detection is shown in Figure 6. A strong positive correlation (R² = 0.9819) was observed.

### Cystatin C Lateral flow

Preparation of materials: The capture line, anti-Cystatin C BSA-PEG Fab (Alere San Diego, Cat No 180) and control line BSA-Biotin were immobilised onto the nitrocellulose membrane (Sartorius, CN140) at 1mg/mL in PBS + 1% Sucrose using the Imagene Isoflow dispenser. Membranes were subsequently dried in a tunnel dryer (Hedinair) at 60°C and stored with desiccant prior to use.

The gold conjugates were prepared according to known protocols. Anti-Cystatin C (BBI, Cat No BP234-3) was conjugated to 40nm gold colloid in a suspension buffer of 20mM TAPS pH8.5 to a final concentration of 15µg/mL. Following a 10-minute incubation, any unbound colloid was blocked with a final concentration of 2mg/mL bovine serum albumin (200mg/mL stock in PBS). Both test conjugate and control gold: Goat anti Biotin gold 40nm (BBI) were mixed together in the presence of a gold drying buffer, Tris with BSA, 5% (w/v) sucrose and 2% (v/v) TritonX100. The gold conjugate was sprayed onto Ahlstrom 8951 glass fibre pads using the Imagene Isoflow dispenser deposited at 0.8µl/mm. The sprayed conjugate pads were dried in a tunnel dryer (Hedinair) at 60°C and stored with desiccant prior to use.

Both prepared membranes and conjugate pads were assembled into one-well plastic cassettes.

Running of the assay: The sample was diluted 1 in 20 in sample diluent (Tris with BSA, 1% (v/v) Tween20). After dilution, 80µL of the sample was added to the sample well of the device and the result was read after 10 minutes.

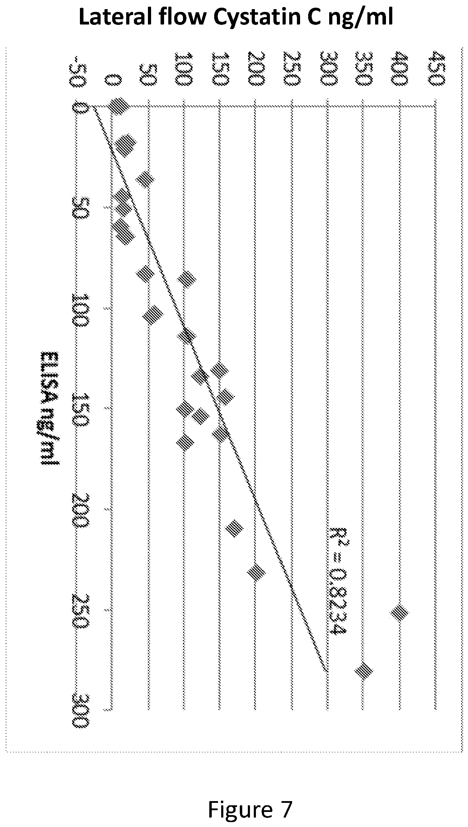

The Cystatin C lateral flow assay performance was compared to the commercially available R&D duoset Cystatin C ELISA by running a standard curve and 40 UTI urine samples run at a 1:20 dilution in PBST 1% BSA (phosphate buffered saline with Tween 20 + 1% bovine serum albumin). Data was analysed according to a Four Parameter Logistic Fit. The correlation between the lateral flow assay and ELISA assay in terms of Cystatin C detection is shown in Figure 7. A strong positive correlation (R² = 0.8234) was observed.

### Conclusion

The three biomarkers HNE, MMP8 and Cystatin C were selected based on testing of suspected UTI samples and subsequent recovery samples with reference ELISA's (Mologic in-house assays and R&D duoset ELISA). Lateral flow assays have been developed that produce equivalent results to the ELISA's (40 UTI samples tested (different samples used for each assay)) as determined by acceptable R² values.

### Example 3 - Statistical Analysis and Algorithm Development

In the Cardiff POETIC study (Bates 2014), 424 samples were collected in total: 204 were from a visit 1 and 220 were from a visit 2 samples (not all of these were matched samples). There were 93 matched visit 1 and visit 2 samples in total.

Sample information was available for a total of 194 samples; 175 from a visit 1 and 19 from a visit 2 samples. Of the 93 matched samples, 81 of the visit 1 samples had accompanying clinical information.

From the 81 visit 1 samples, 32 of the samples were UTI positive and 49 were UTI negative. The status was based on the microbiology results:
- No growth = No growth found
- No significant growth = growth of pure isolate <10⁵
- Mixed 2 orgs = Growth of 2 orgs at 10⁵ cfu/mL or more
- Mixed >2 orgs = Growth of 3 or more isolates at 10⁵ cfu/mL or more
- POS = either a pure growth at 10⁵ cfu/mL or more OR a growth of a predominant isolate at 10⁵ or more with growth of other isolates at 3 x log10 less.

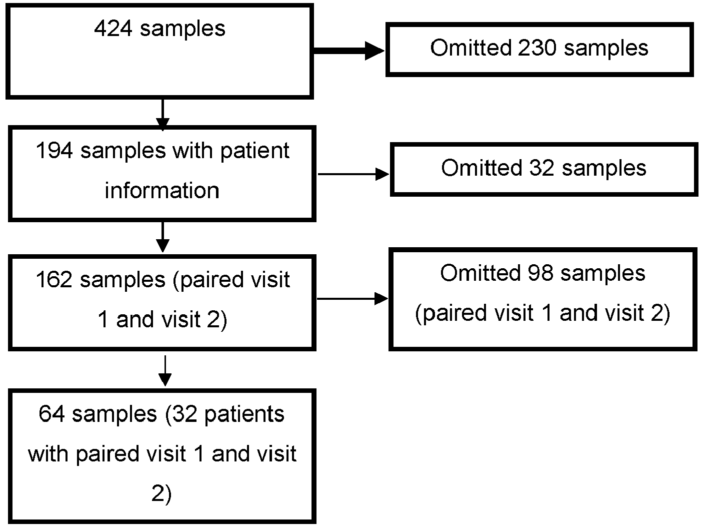

The urine samples were analysed by the inventors and logistic regression analysis was used to generate a statistical model for correlation of the levels of the three biomarkers HNE, MMP8 and Cystatin C with UTI status.

The model was derived from the 'positive' UTI visit 1 and subsequent visit 2 samples. It was assumed that at the time of collection of the visit 2 sample the patient had fully recovered. Two logistic regression models were generated.

### MMP8 only classification table

| MMP8 Only | | Predicted | | |
|---|---|---|---|---|
| | | Visit 1 | Visit 2 | % correct |
| Observed | Visit 1 | 27 | 5 | 84.4 |
| | Visit 2 | 3 | 29 | 90.6 |
| | Overall % | | | 87.5 |

### HNE only classification table

| HNE Only | | Predicted | | |
|---|---|---|---|---|
| | | Visit 1 | Visit 2 | % correct |
| Observed | Visit 1 | 24 | 8 | 75.0 |
| | Visit 2 | 2 | 30 | 93.8 |
| | Overall % | | | 84.4 |

### Cystatin C only classification table

| Cystatin C Only | | Predicted | | |
|---|---|---|---|---|
| | | Visit 1 | Visit 2 | % correct |
| Observed | Visit 1 | 23 | 9 | 71.9 |
| | Visit 2 | 26 | 6 | 18.8 |
| | Overall % | | | 45.3 |

### MMP8 and HNE only classification table - MODEL 1

| MMP8 and HNE only | | Predicted | | |
|---|---|---|---|---|
| | | Visit 1 | Visit 2 | % correct |
| Observed | Visit 1 | 28 | 4 | 87.5 |
| | Visit 2 | 1 | 31 | 96.9 |
| | Overall % | | | 92.2 |

### MMP8, HNE and Cystatin C classification table - MODEL 2

| MMP8, HNE and Cystatin C | | Predicted | | |
|---|---|---|---|---|
| | | Visit 1 | Visit 2 | % correct |
| Observed | Visit 1 | 28 | 4 | 87.5 |
| | Visit 2 | 1 | 31 | 96.9 |
| | Overall % | | | 92.2 |

These 2 models were used for the remaining analysis. It is noted that whilst Cystatin C did not increase the predictive accuracy when combined with MMP8 and HNE, it is expected that Cystatin C will increase the predictive accuracy when a larger number of biomarkers are analysed. The inclusion of Cystatin C is also predicted to diagnose renal infection.

### UTI visit 1 and visit 2

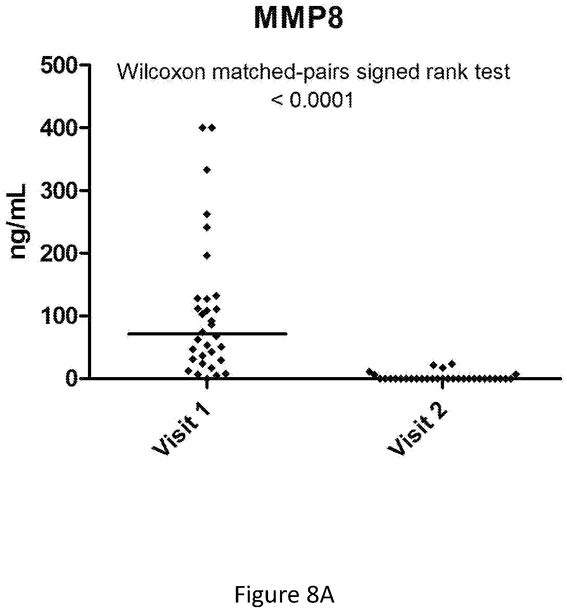

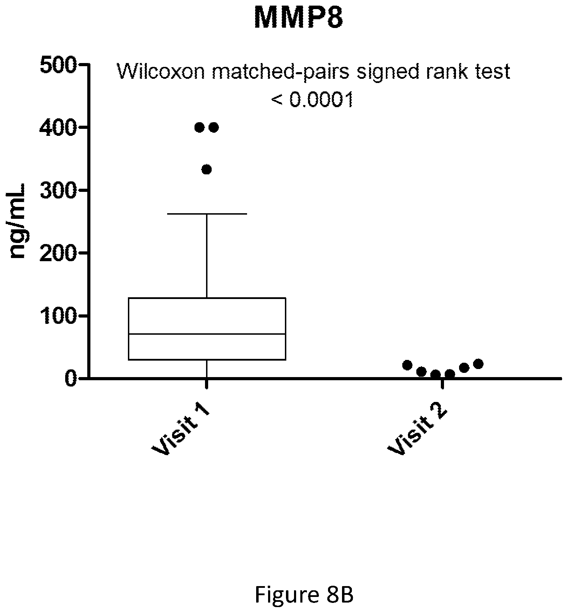

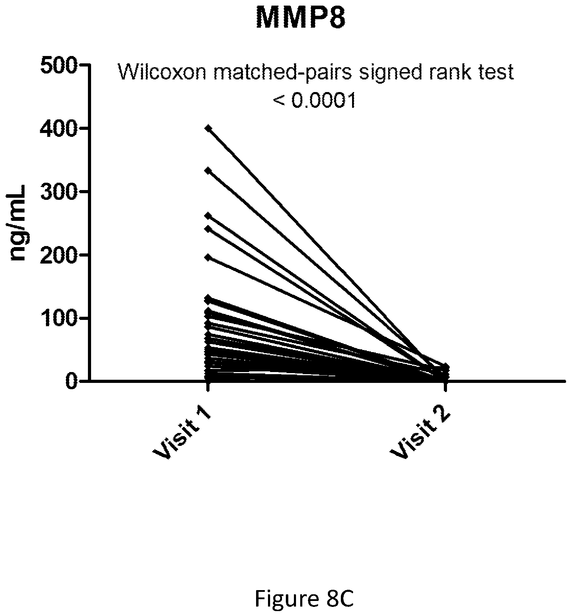

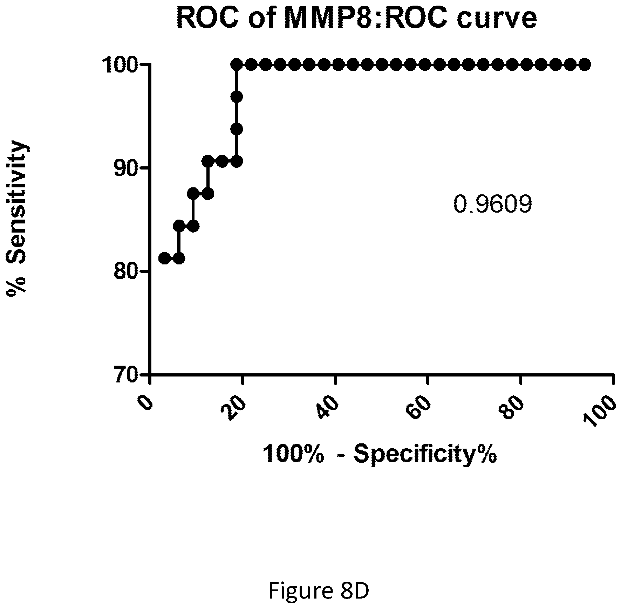

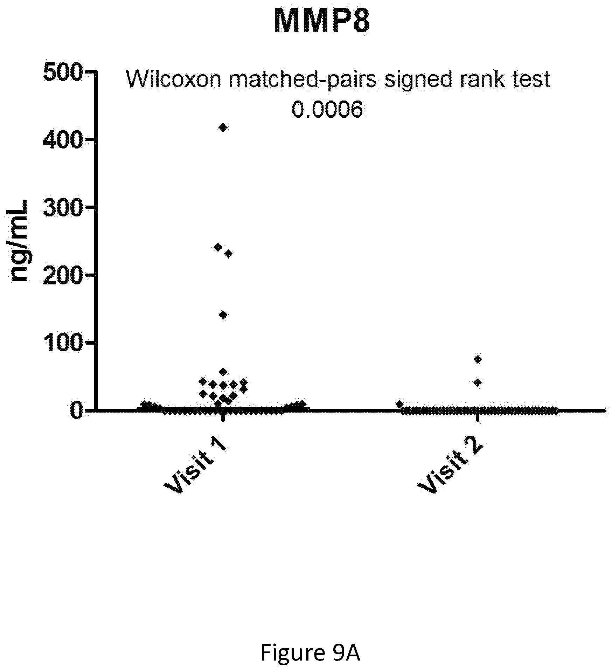

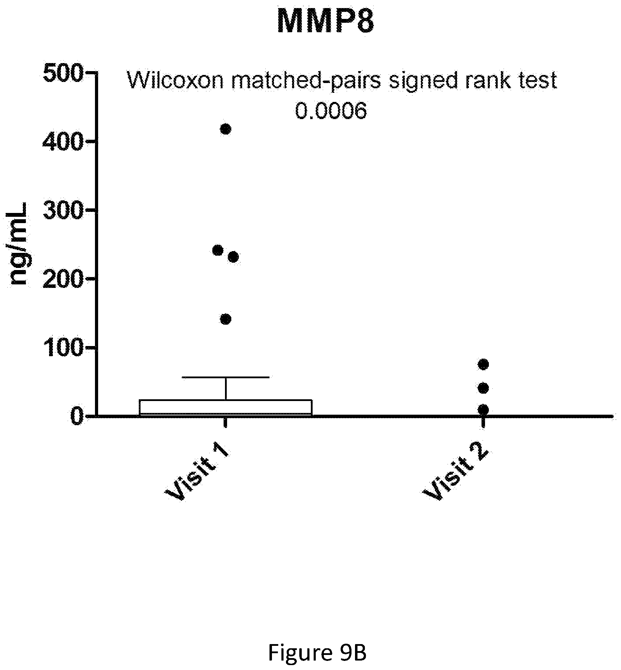

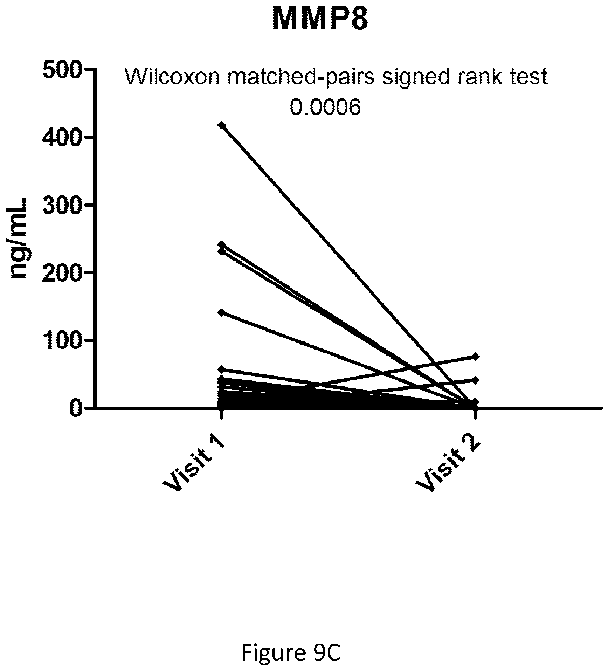

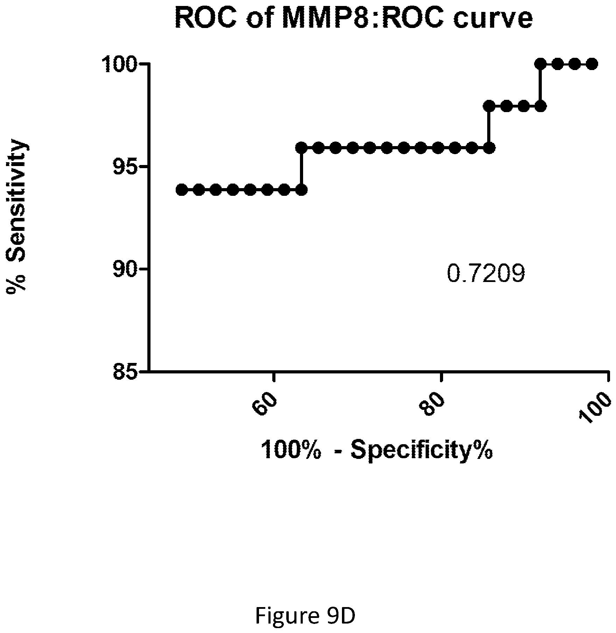

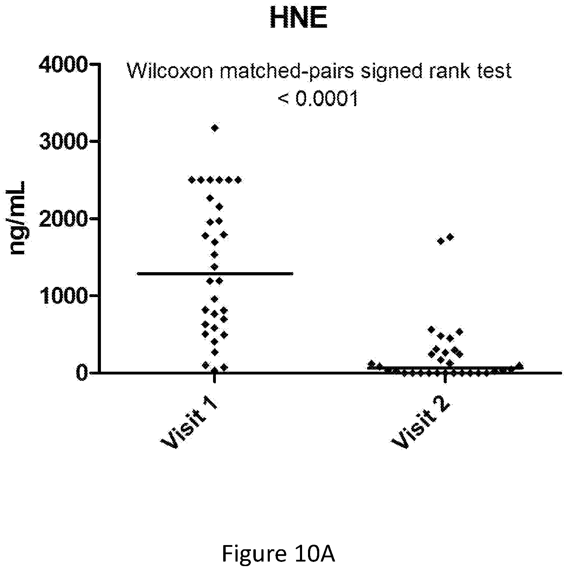

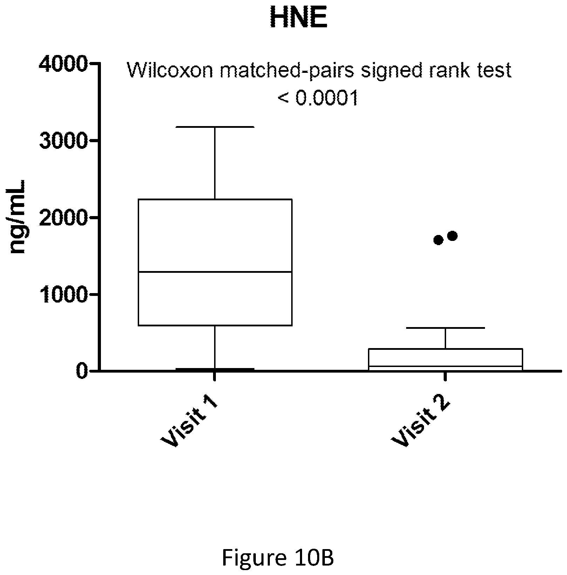

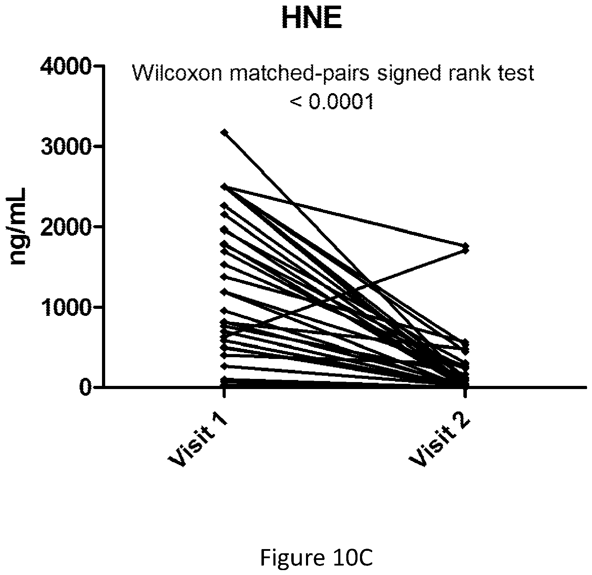

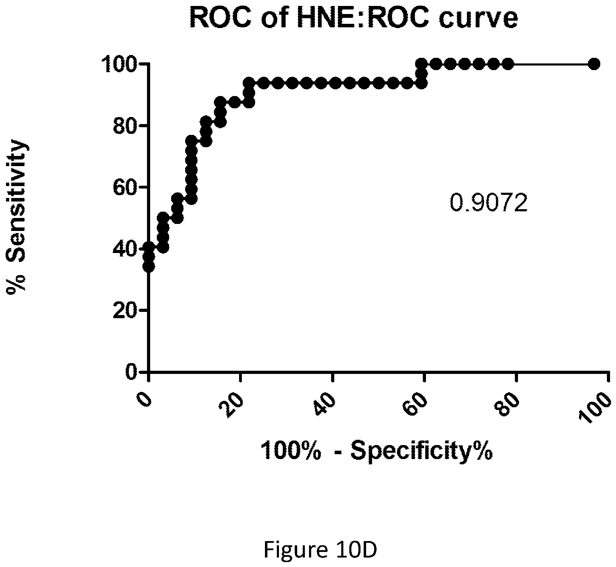

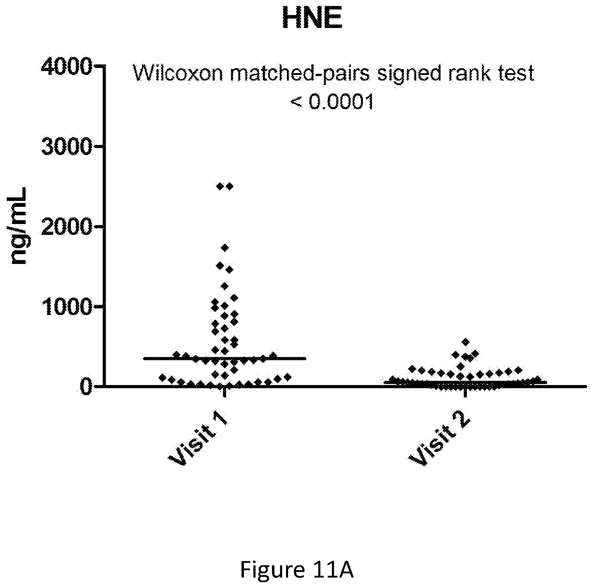

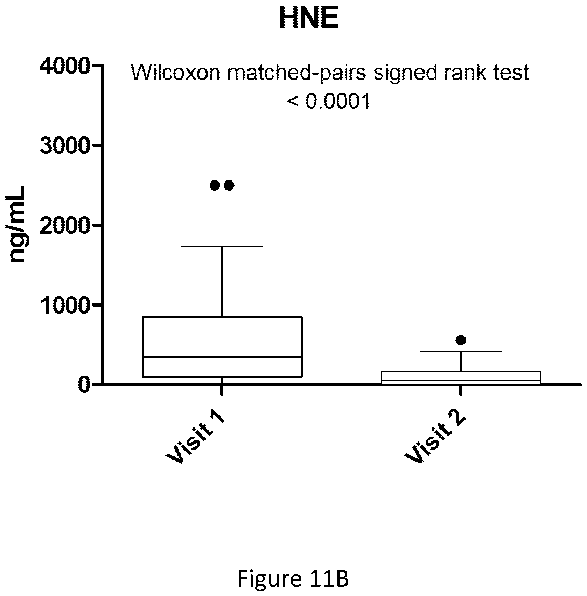

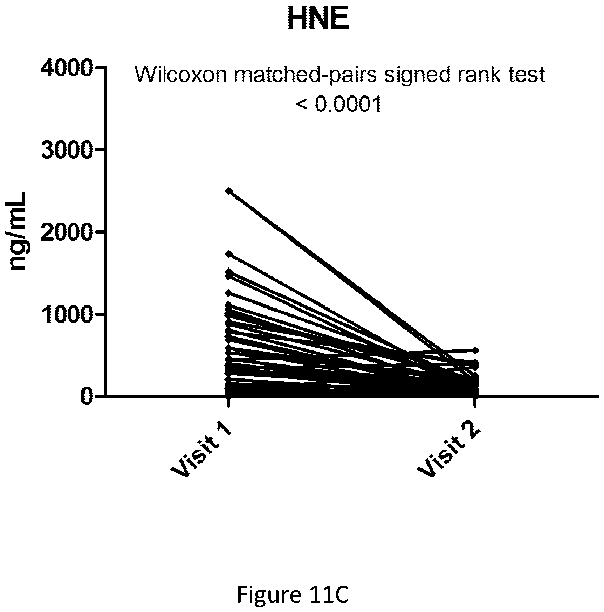

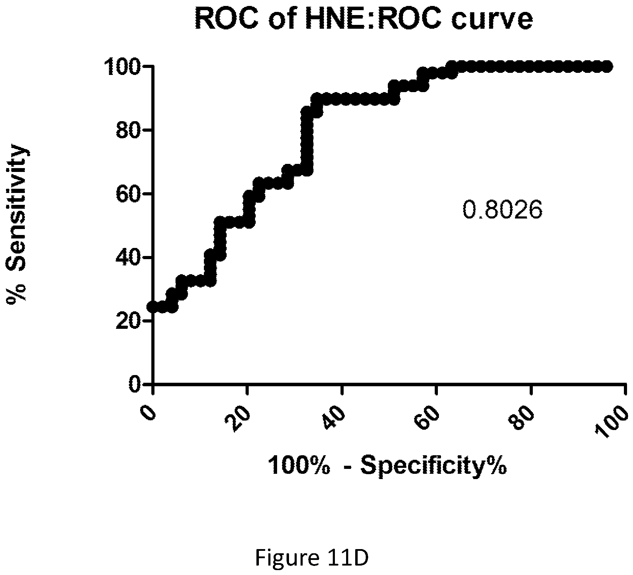

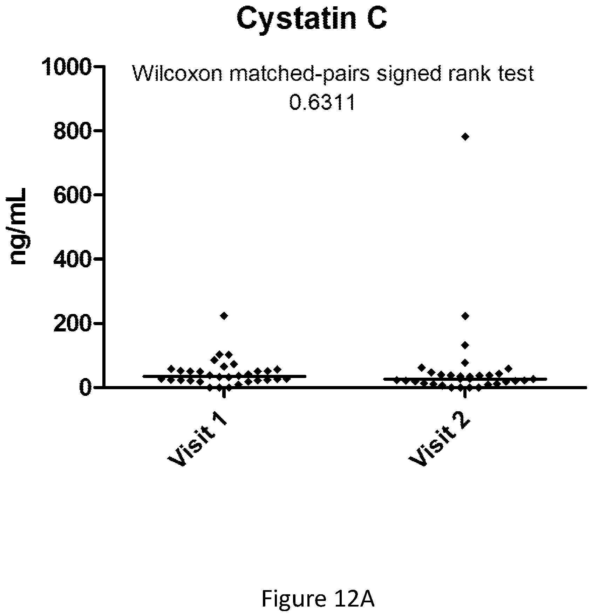

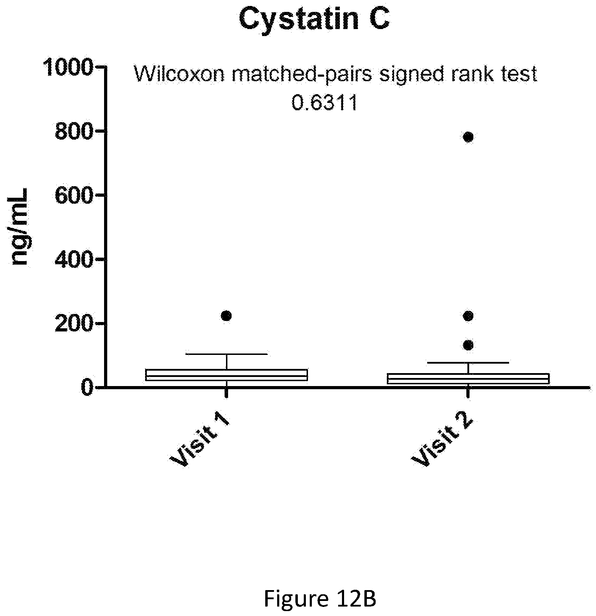

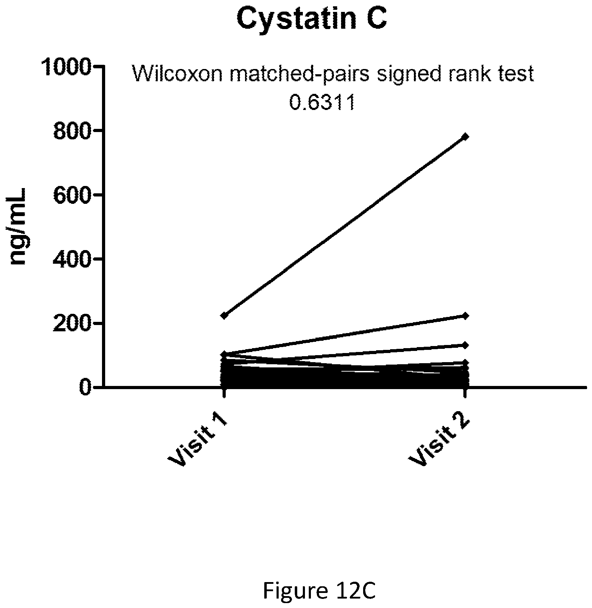

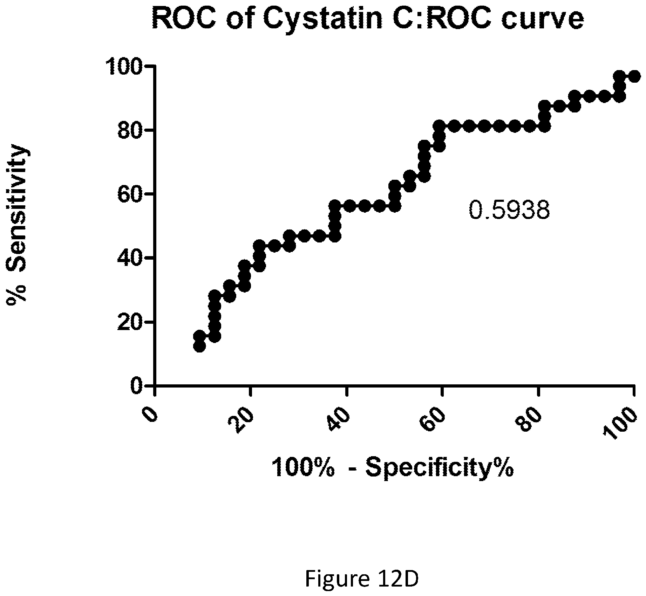

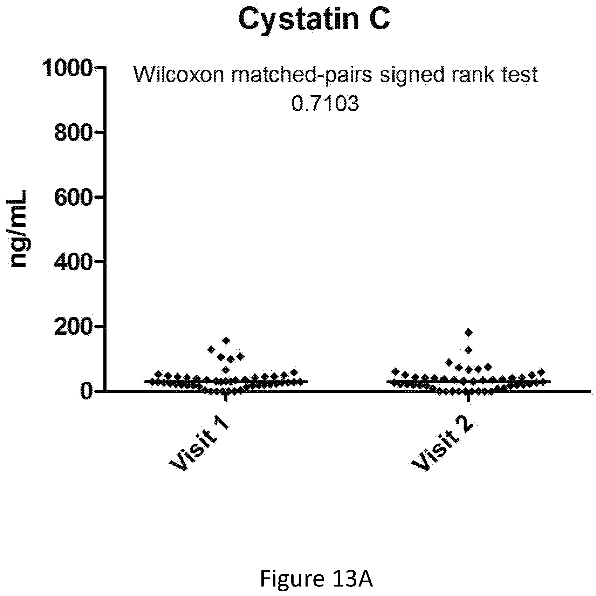

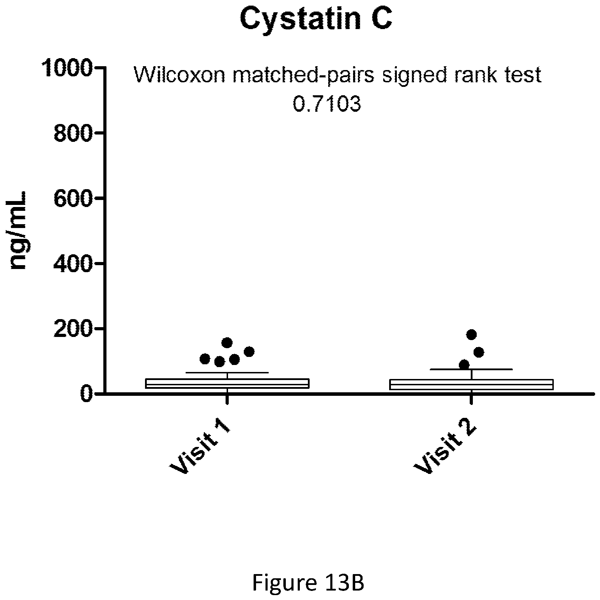

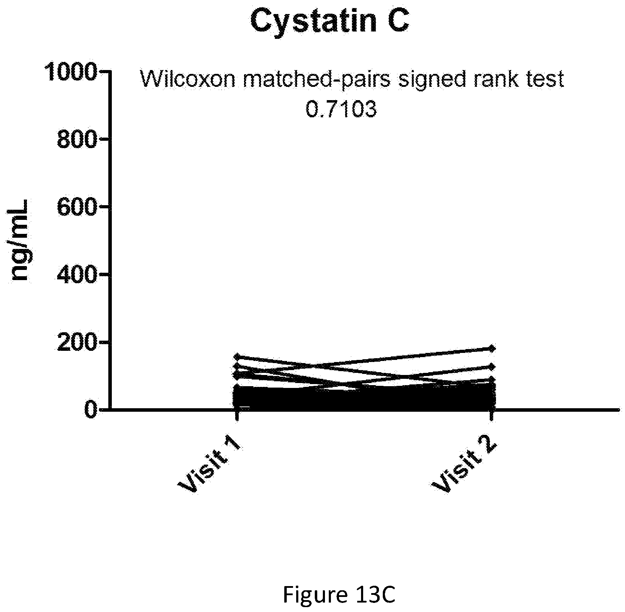

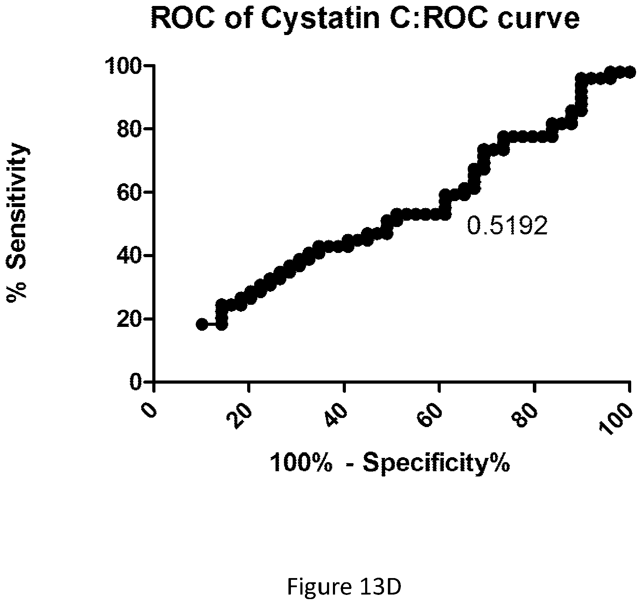

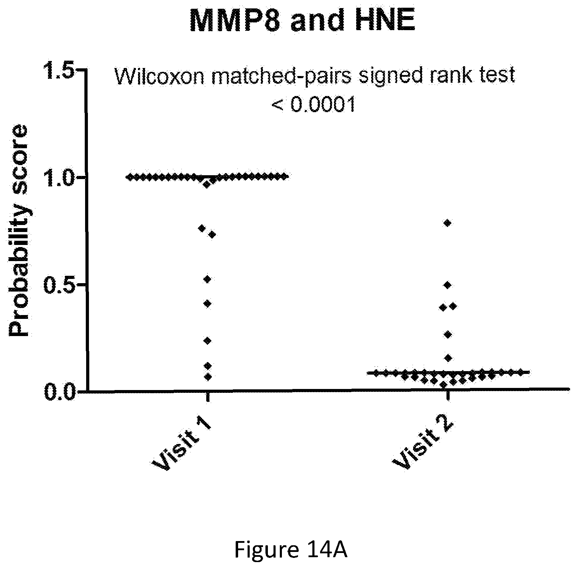

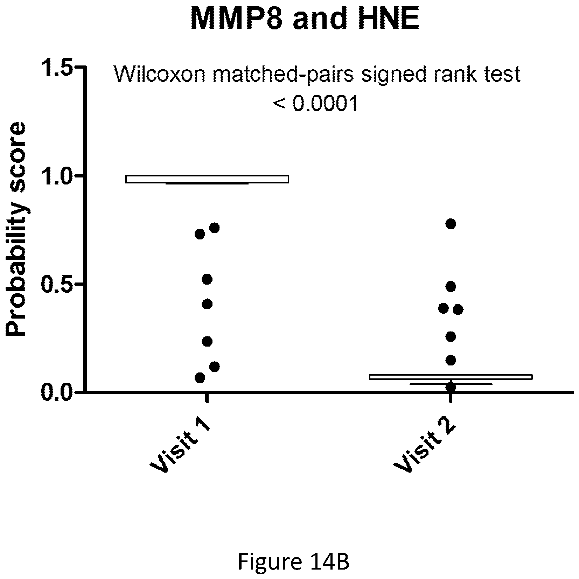

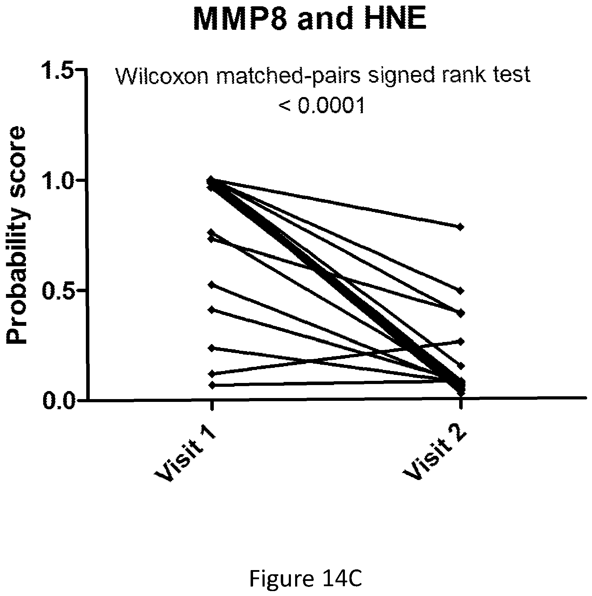

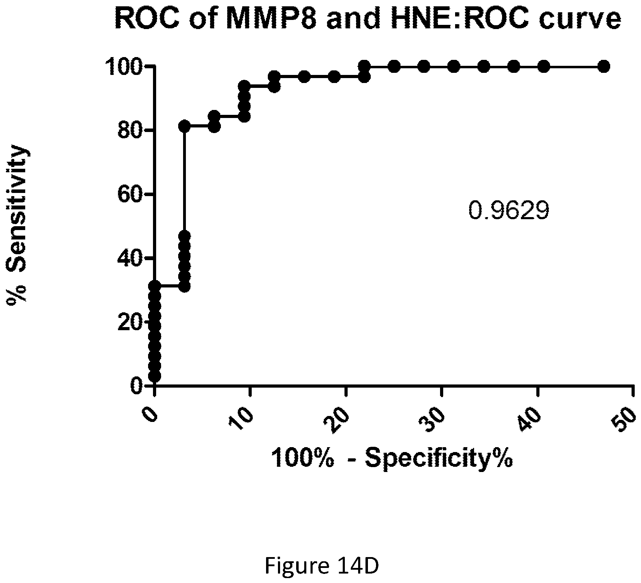

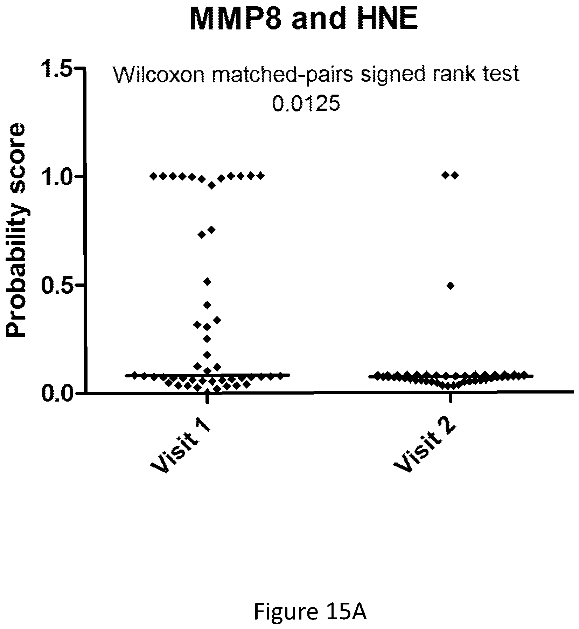

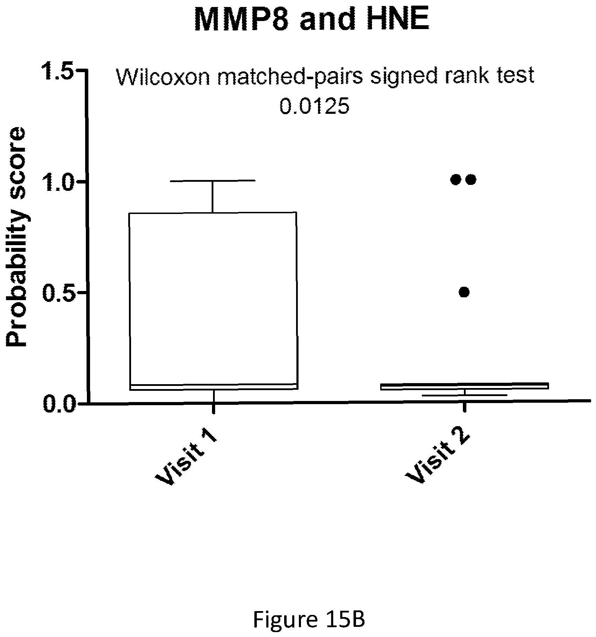

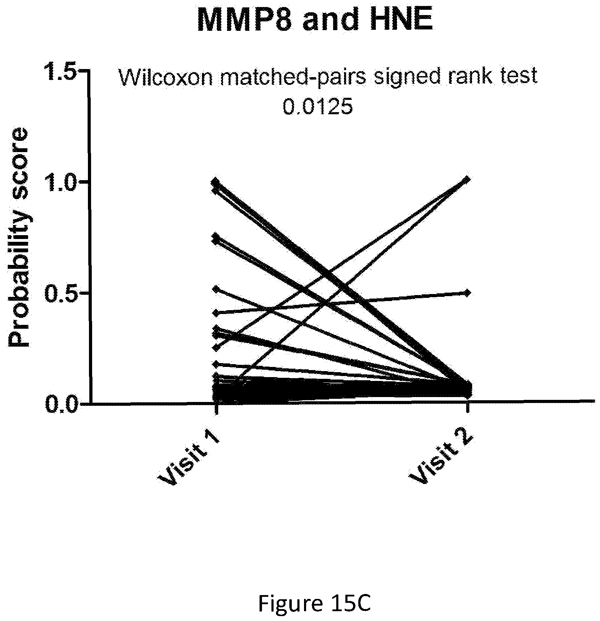

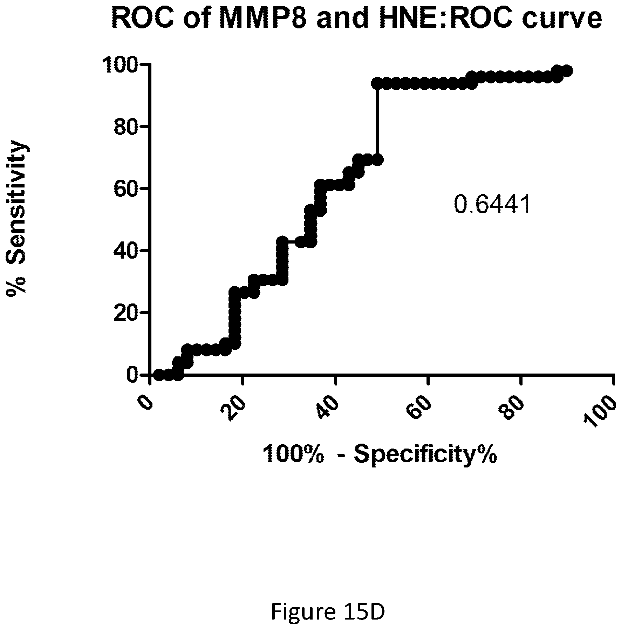

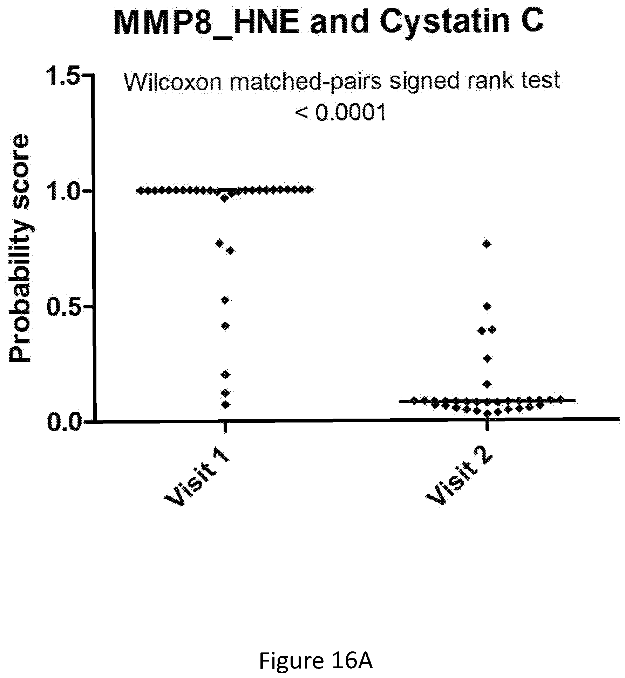

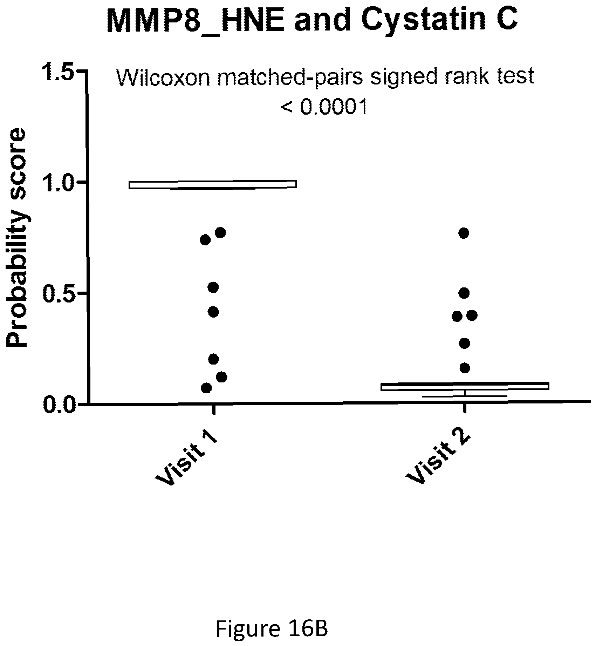

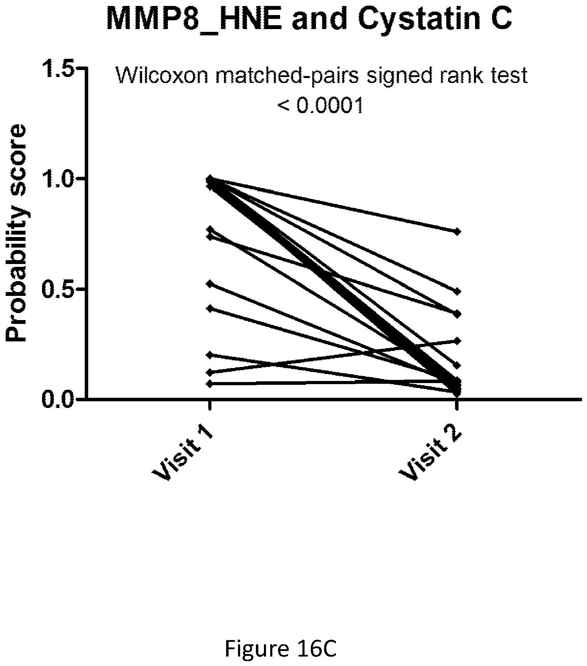

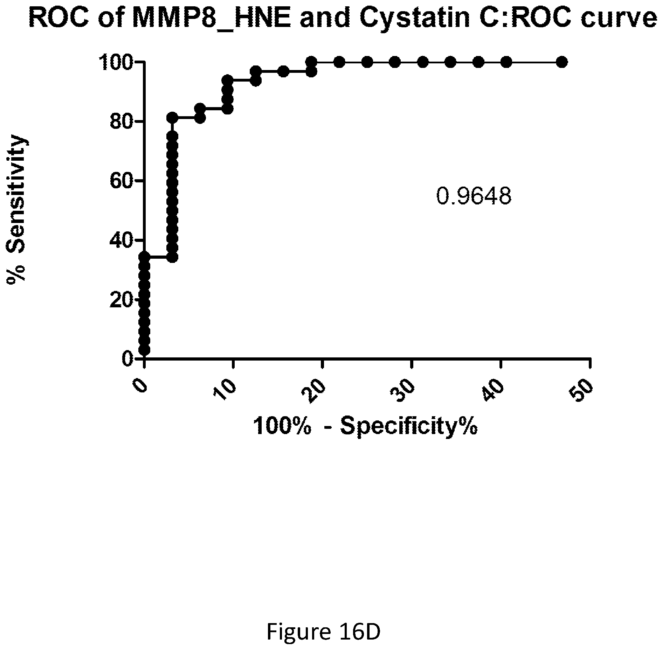

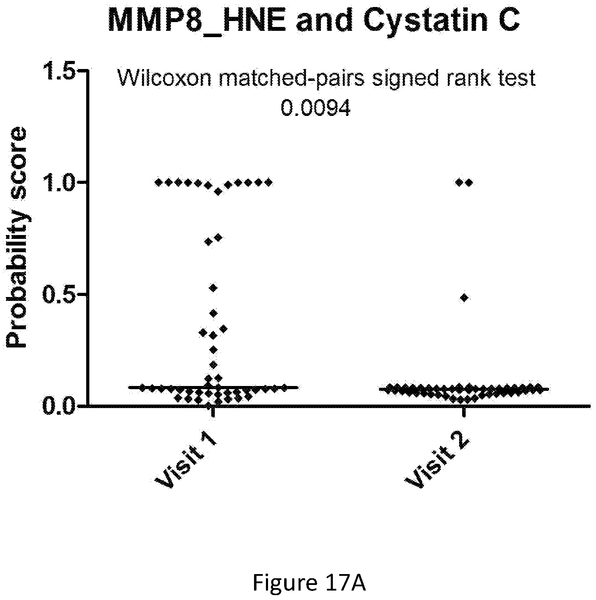

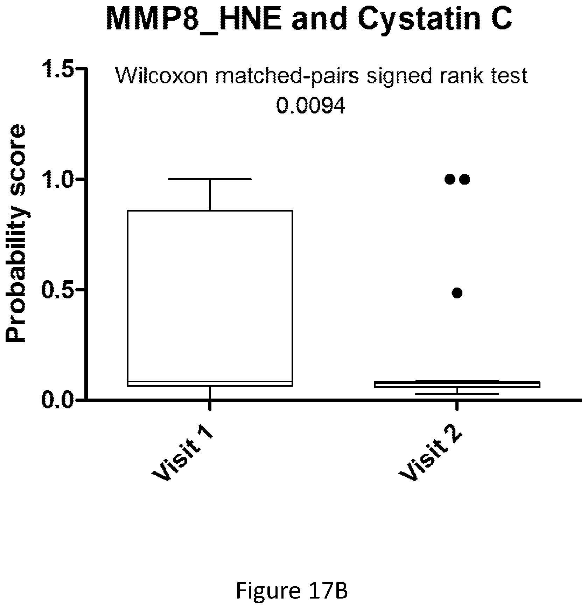

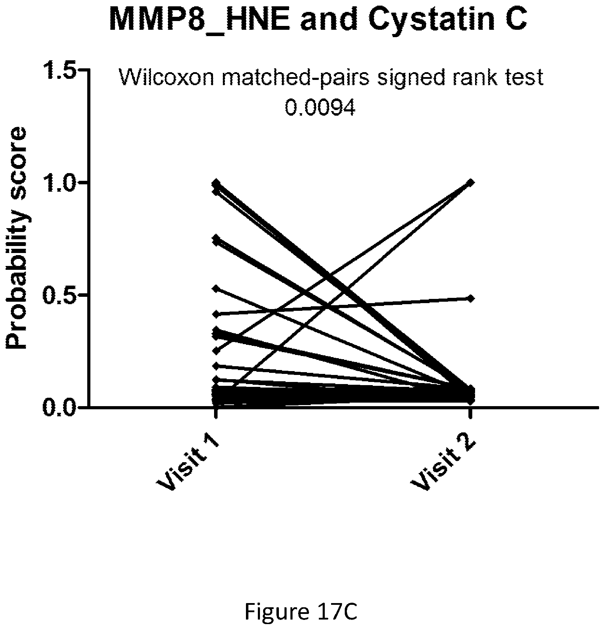

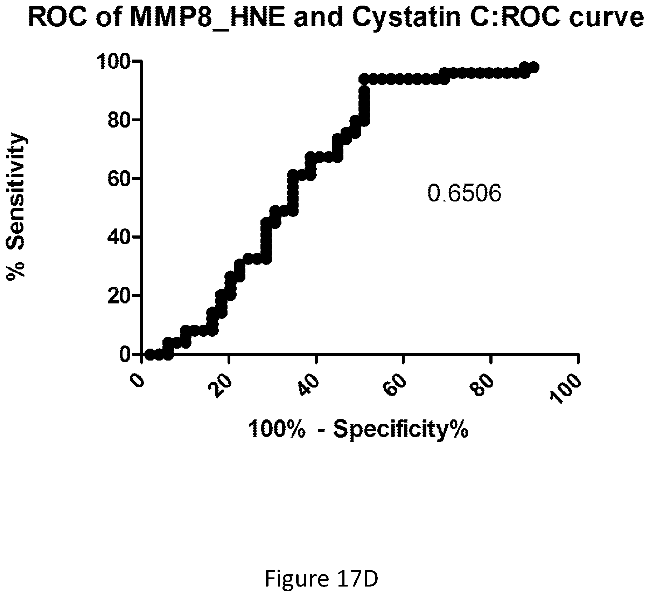

The individual biomarker levels and the 2 statistical models were evaluated for the ability to detect a UTI. Wilcoxon matched-pairs signed rank test was used to determine the discrimination between positive and negative detection of a UTI as they were paired samples. ROC analysis and AUC values were determined for each biomarker and the 2 statistic models. The results are shown in Figures 8-17. As shown in Figure 16, the combined markers produced an excellent AUC of 0.96 and thus are able to discriminate between culture positive confirmed UTI patients at visit 1 and visit 2 post treatment. This result is further strengthened by the data in Figure 17 whereby a poor AUC of 0.65 was obtained with the culture negative UTI patients indicating that the biomarkers were not significantly different pre and post treatment.

### UTI positive and negative

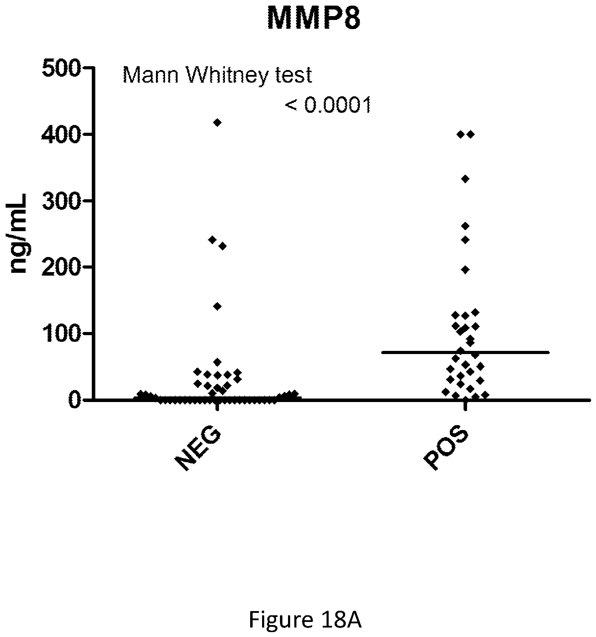

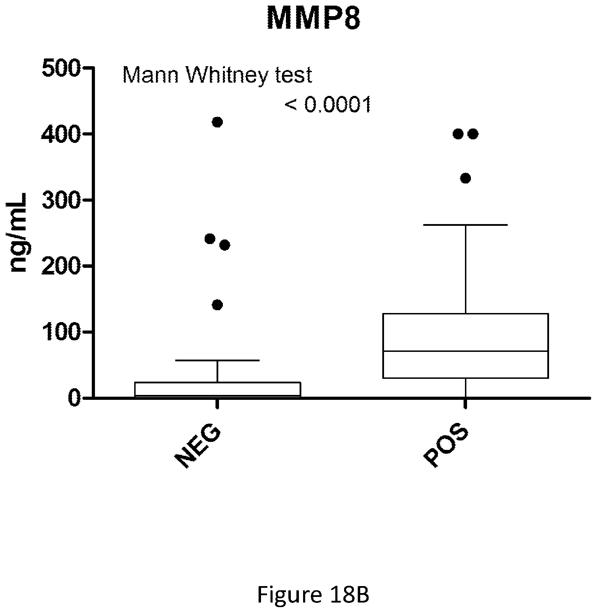

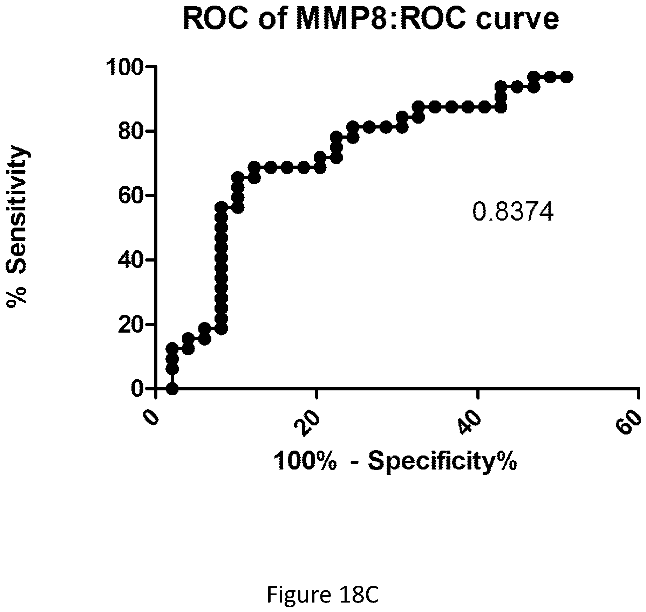

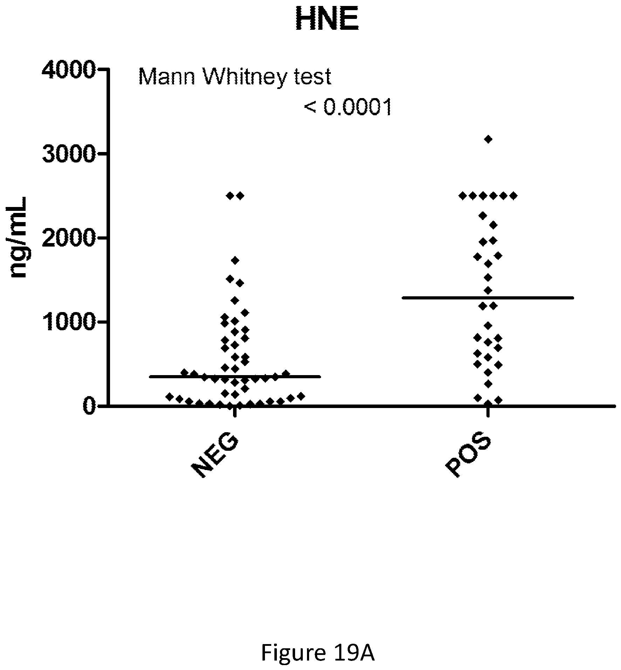

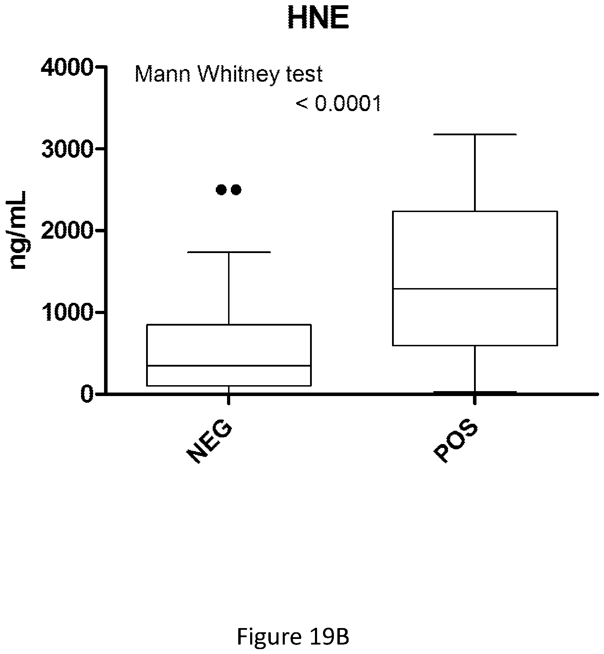

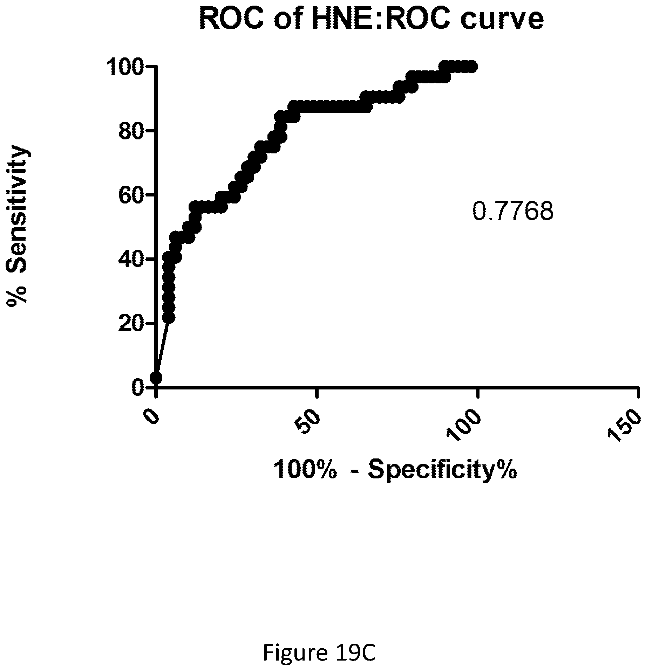

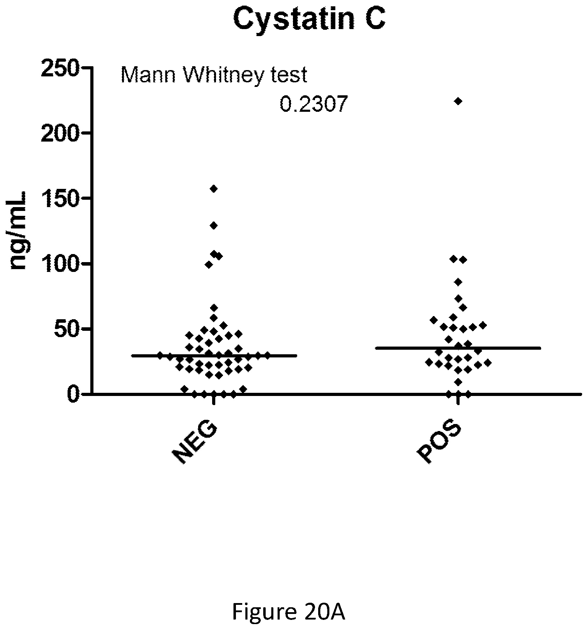

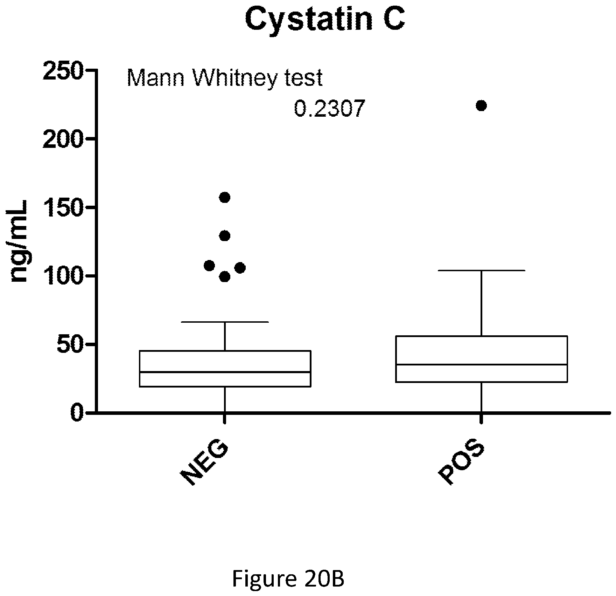

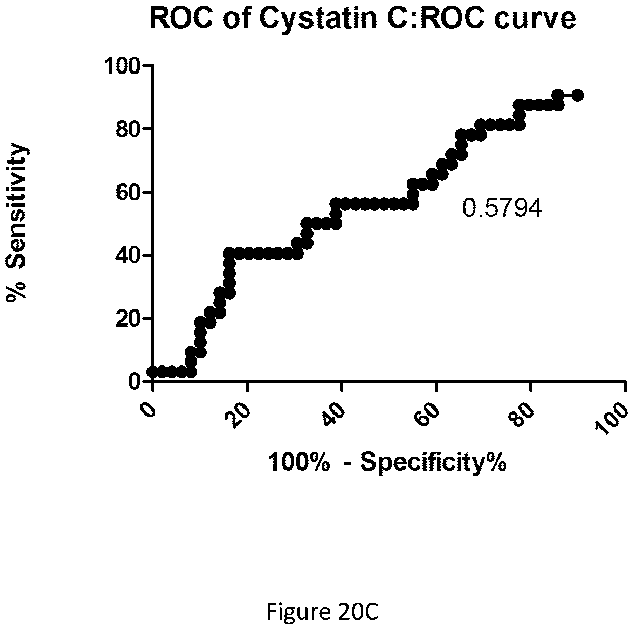

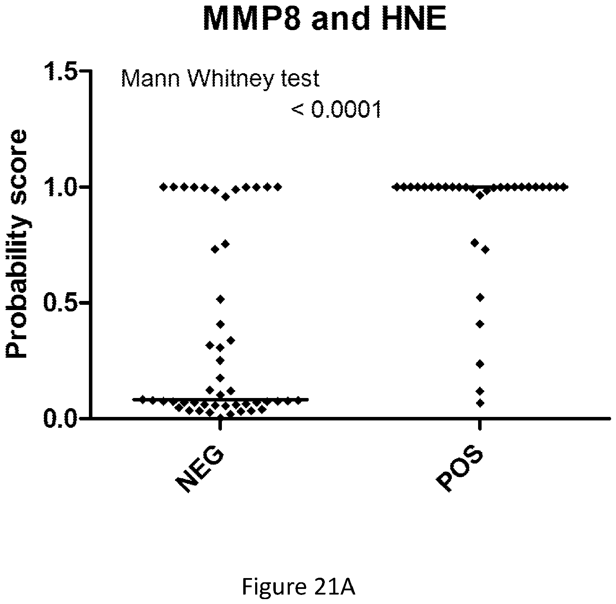

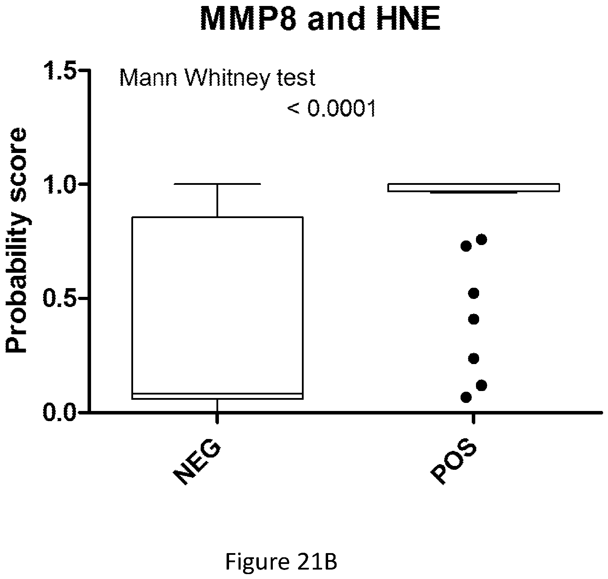

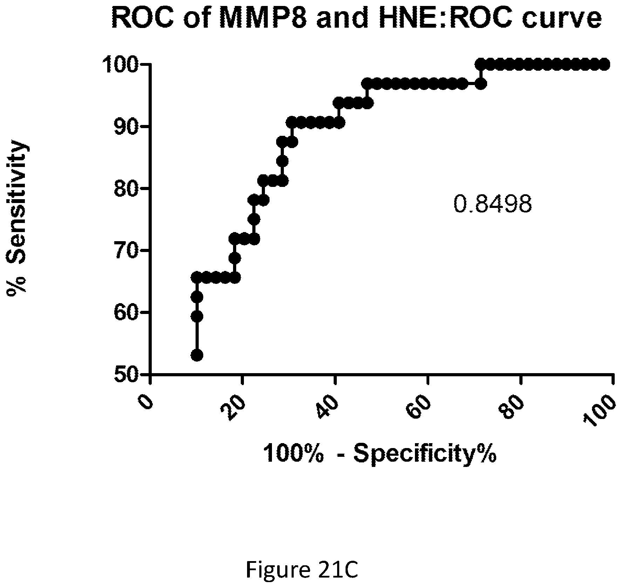

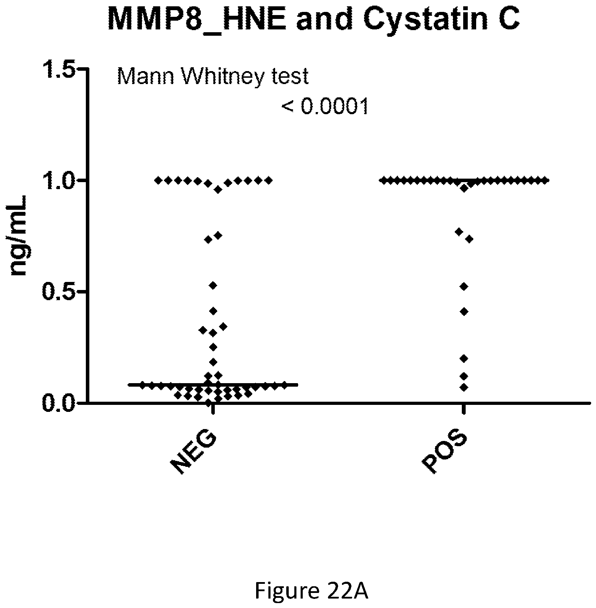

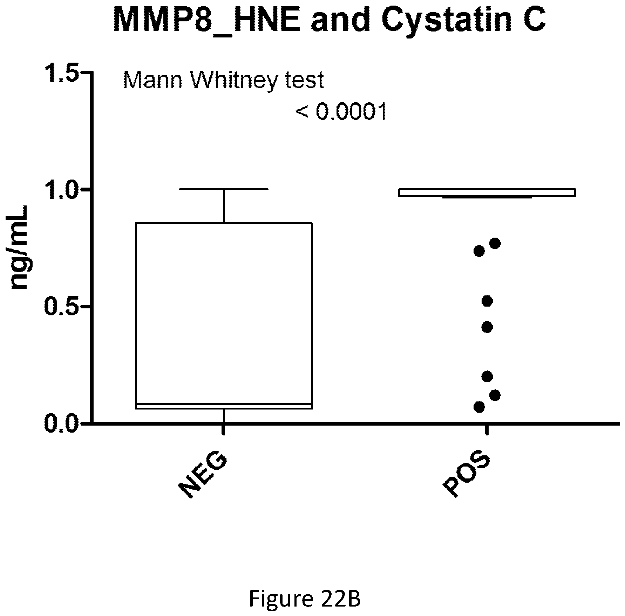

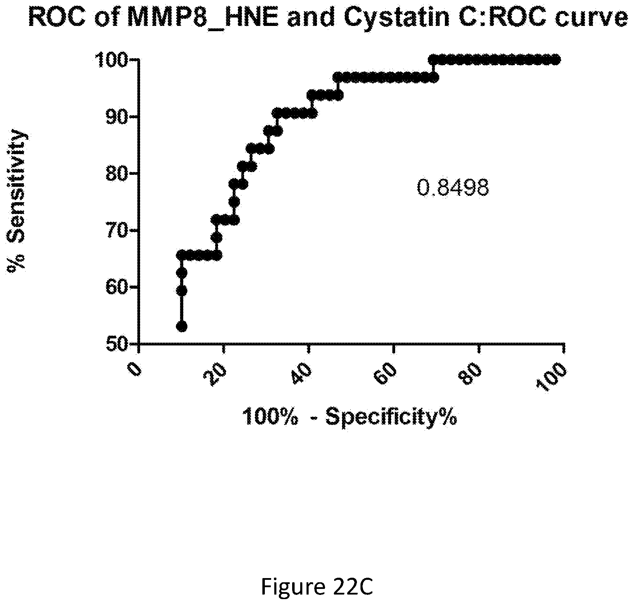

Although the algorithm was initially created using the visit 1 and visit 2 data, it was also applied to analyse the UTI confirmed positive and negative data. The individual biomarker levels and the 2 statistical models were evaluated. A Mann-Whitney test was used to determine the discrimination between the 2 groups. ROC analysis and AUC values were determined for each biomarker and the 2 statistical models. The results are shown in Figures 18-22.

### Conclusion

The samples with suspected UTI were poorly characterised according to the reference method used. Eighty-one patients with suspected samples were given antibiotics to treat UTI but, in fact, only 32 of these were positive based on microbiology results and 49 were negative. Thus 60% of these patients had been misdiagnosed. Using the combination of MMP8, HNE and Cystatin C, 28 of the 32 patients would have been correctly diagnosed with a sensitivity of 88% and 34 of the 49 negative UTI would have been given a negative result (specificity of 69%), therefore 19% of the patients would have been given unnecessary treatment compared to 60%. Analysis into the causative organisms may provide further insight. As there was no clinical information for the visit 2 samples, it is possible that some of these patients may have had a lingering infection.

### Example 4 - Exemplary test strip of the invention

The exemplary test strip detects MMP8, HNE and Cystatin C if present in a urine sample taken from a subject. The test strip first comprises a sample receiving zone to which the urine sample is added. This is connected via a liquid flow path to a conjugate zone comprising an anti-HNE antibody (bovine serum albumin, BSA linked) and an anti-MMP8 antibody (BSA linked) which are each complexed to gold particles and an anti-Cystatin C polyclonal antibody (complexed to gold particles). Alternatively, the sample receiving zone and conjugate zone may be combined as a single zone. Connected to the conjugate zone is a solid support defining a liquid flow path for the sample and comprising corresponding test lines for each of MMP8, HNE and Cystatin C. The HNE and MMP8 test lines comprise an immobilised anti-HNE antibody (BSA linked) and an immobilised anti-MMP8 antibody (BSA linked) respectively. The Cystatin C test line comprises immobilised Cystatin C or an analogue thereof to which the anti-Cystatin C polyclonal antibody is still able to bind. All three test lines are perpendicular to the direction of flow of the sample along the test strip flowing away from the sample receiving zone.

The HNE and MMP8 tests are sandwich assays. When HNE and MMP8 are present in a urine sample added to the sample receiving zone, they travel in the liquid along the liquid flow path to the conjugate zone and are bound by the anti-HNE antibody (bovine serum albumin, BSA linked) and an anti-MMP8 antibody (BSA linked) which are each complexed to gold particles. The HNE-antibody-gold complexes and MMP8-antibody-gold complexes then travel along the liquid flow path and are bound at the HNE and MMP8 test lines by the immobilised anti-HNE antibody (BSA linked) and immobilised anti-MMP8 antibody (BSA linked) respectively. As a consequence, a coloured test line appears on the test strip by virtue of the immobilised gold particles to indicate the presence of HNE and MMP8 respectively in the sample.

The Cystatin C test is a competition assay. When Cystatin C is present in a urine sample added to the sample receiving zone, it travels in the liquid along the liquid flow path to the conjugate zone and is bound by the anti-Cystatin C polyclonal antibody (complexed to gold particles). The Cystatin C-antibody-gold complexes then travel along the liquid flow path. As the anti-Cystatin C polyclonal antibody is already complexed with Cystatin C present in the sample it cannot bind to the Cystatin C or an analogue thereof to which the anti-Cystatin C polyclonal antibody is still able to bind immobilised at the test line. Thus, when Cystatin C is present in the sample, a weak or no test line appears on the test strip proportional to the amount of Cystatin C present in the sample.

This is shown in Figure 23. This exemplary test strip may be referred to as the UTRiPLEX test strip assay, UTRiPLEX test strip, UTRiPLEX assay or simply UTRiPLEX. The sequential order of HNE, MMP8 and Cystatin C along the test strip is not important and can be in any order.

A procedural control line (C) may be included downstream of the test lines (relative to the flow of sample) to indicate that the assay has been performed correctly, that is to say the control line provides a positive signal once contacted with the sample indicating that the sample has flowed past, and therefore been in contact with, the test lines. Thus, when a negative result on the test lines indicating an absence of MMP, HNE and/or Cystatin C in the sample is observed, the user can have confidence that this is a true result and not simply because for some reason the sample did not flow along the flow path and come into contact with the test lines. An exemplary control line comprises immobilised BSA-Biotin. The conjugate zone further comprises an anti-Biotin antibody complexed with gold particles.

### Reagents and pilot manufacture

The test lines Cystatin C antigen (BBI P713-1), anti-HNE BSA-PEG Fab (Alere San Diego, Cat No 01241), anti-MMP8 BSA-PEG Fab (Alere San Diego, Cat No 99611) and BSA-Biotin (Mologic) were immobilised onto a nitrocellulose membrane at 0.75mg/mL, 0.5mg/mL, 0.5mg/mL and 0.5mg/mL respectively in PBS + 1% w/v Sucrose using the Imagene Isoflow dispenser. Cystatin C, HNE, MMP8 and BSA-Biotin lines were plotted at 12mm, 16mm, 20mm and 24mm onto a 40mm CN95 nitrocellulose membrane and subsequently dried in a tunnel dryer (Hedinair) at 60°C and stored with desiccant prior to use.

The gold conjugates' preparation:
- Anti-Cystatin C goat polyclonal (BBI, BP234-3) was conjugated to 40nm gold colloid in a suspension buffer of 20mM aqueous borate at pH8.5, to a final concentration of 15µg/mL. Following a 10 min incubation, any unbound colloid was blocked with a final concentration of 2mg/mL bovine serum albumin (200mg/mL stock in PBS).
- Anti-HNE BSA-PEG Fab (Alere San Diego, Cat No 01871) was conjugated to 40nm gold colloid in a suspension buffer of 20mM aqueous MES at pH5.3 to a final concentration of 15µg/mL. Following a 10 min incubation, any unbound colloid was blocked with a final concentration of 2mg/mL bovine serum albumin (200mg/mL stock in PBS).
- Anti-MMP8 BSA-PEG Fab (Alere San Diego, Cat No 99651) was conjugated to 40nm gold colloid in a suspension buffer of 20mM aqueous BES at pH6.6 to a final concentration of 15µg/mL. Following a 10 min incubation, any unbound colloid was blocked with a final concentration of 2mg/mL bovine serum albumin (200mg/mL stock in PBS).
- Control gold: Goat anti Biotin gold 40nm (BBI)

The gold conjugates were mixed together with a final optical density of 5 (Cystatin C), 2 (HNE), 3 (MMP8) and 1 (Control) in gold drying buffer 1M Tris pH 9, 3% (w/v) BSA, 5% (w/v) sucrose and 2% (v/v) TritonX100. The gold conjugate was sprayed onto 27mm Ahlstrom 8951 glass fibre pads using the Imagene Isoflow dispenser deposited at 0.8µl/mm. The sprayed conjugate pads were dried in a tunnel dryer (Hedinair) at 60°C and stored with desiccant prior to use.

Both prepared membranes and conjugate pads were assembled into strips and laminated onto a backing card. The strip includes a terminal region downstream of the control line which the user can grip by hand or otherwise. Thus, the test strip is very convenient for the user to use.

### Sample collection and storage

Collect a sample of urine "mid-stream" in a sterile screw-top container (avoid the first urine of the day).

The sample can be tested directly from the collection pot or transferred to a clean dry, container.

If testing cannot be done within an hour, refrigerate the specimen and let it return to room temperature before testing.

For storage of samples, aliquot and store at -20°C/-4°F or below. Avoid repeated freezethaw cycles.

### Assay procedure

Equilibrate urine to room temperature prior to use.

Testing should be done at room temperature (15 - 30°C / 59 - 86°F)
1) Collect a midstream urine sample in a clean, dry container as described above
2) Remove one UTRiPLEX^{™} test strip from the container and immediately replace the cap. Do not touch areas of the test strip apart from the terminal grip region indicated (this may be a red coloured terminal region of the strip that says 'hold')
3) Dip the sample receiving zone portion of the strip into the sample for approximately 10 seconds. The boundary of the sample receiving zone may be marked for the user's convenience; for instance, with arrows. The user should dip the sample receiving zone portion of the strip into the sample up to the arrows.
4) Place strip in adaptor for reader - ensure correct orientation (see Figure 24A)
5) Place reader on top of the adaptor and read after 10 minutes (see Figure 24B)

### Reader instructions

The instructions are for single use mode (as proposed for use by healthcare professional) using the Cube reader's automatic timer.
- To turn the test reader on, press the button briefly. A beep sounds and the display shows 'ON'. If not- see error guide for instructions on how to change the batteries.
- Press and hold the button for longer than 1 second. The display will show `RFID'.
- Place the supplied RFID card onto the top of the reader. A beep sounds and the display shows 'TEST'. (Remove the RFID card after the beep has been heard).
- Start the timer by pressing the button. The display will show a countdown from 10 minutes.
- After the countdown has finished a beep sounds and the result is displayed and saved automatically. For the single use mode, an alarm will indicate when the 10 minutes is up. The strip can be disposed of once complete.

### Standard curves

Standard curves can be used to quantify unknown levels of each biomarker in urine samples.

Cystatin C, HNE and MMP8 were diluted in the sample diluent (with PBS at pH7.2), to give concentrations between:
MMP8: 1.95-125ng/mL
HNE: 62.5-2000ng/mL
Cystatin C: 78.12 - 2500ng/mL

The results were as follows:

| **MMP8 (ng/mL)** | **Reader value** | **HNE (ng/mL)** | **Reader value** | **Cystatin C (ng/mL)** | **Reader value** |
|---|---|---|---|---|---|
| 125 | 255.25 | 2000 | 352.55 | 2500 | 23.45 |
| 62.5 | 230.35 | 1000 | 272.625 | 1250 | 54.775 |
| 31.25 | 200.1 | 500 | 169.65 | 625 | 106.15 |
| 15.625 | 151.975 | 250 | 83.075 | 312.5 | 154.775 |
| 7.8125 | 100.5 | 125 | 41.4 | 156.25 | 188.525 |
| 3.90625 | 56.275 | 62.5 | 18.1 | 78.125 | 204.1 |
| 1.953125 | 30.725 | 0 | 2.2 | 0 | 231.125 |
| 0 | 0.925 | - | - | - | - |

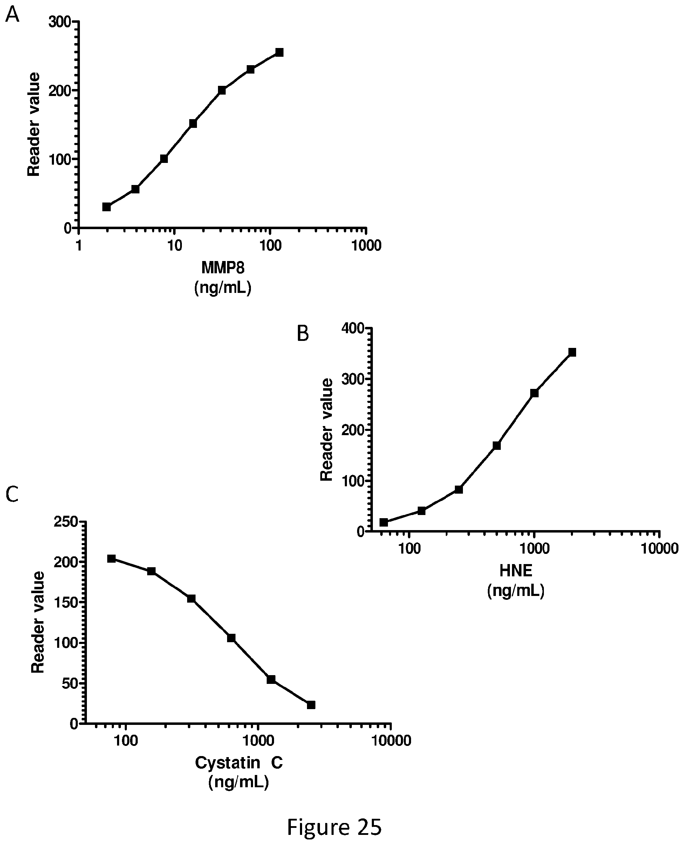

The results are shown graphically in Figure 25.

### Example 5 - Reference assay validation

### MMP8

The reference ELISA for MMP8 (Human Total MMP8 Duoset ELISA, R&D Systems) was validated according to FDA standards and specifications were met for the following criteria:
- Sensitivity (lowest limit of detection; LLOD)
- Standard curve
- Intra-plate reproducibility
- Inter-plate reproducibility
- Spike recovery
- Linearity
- Cross reactivity

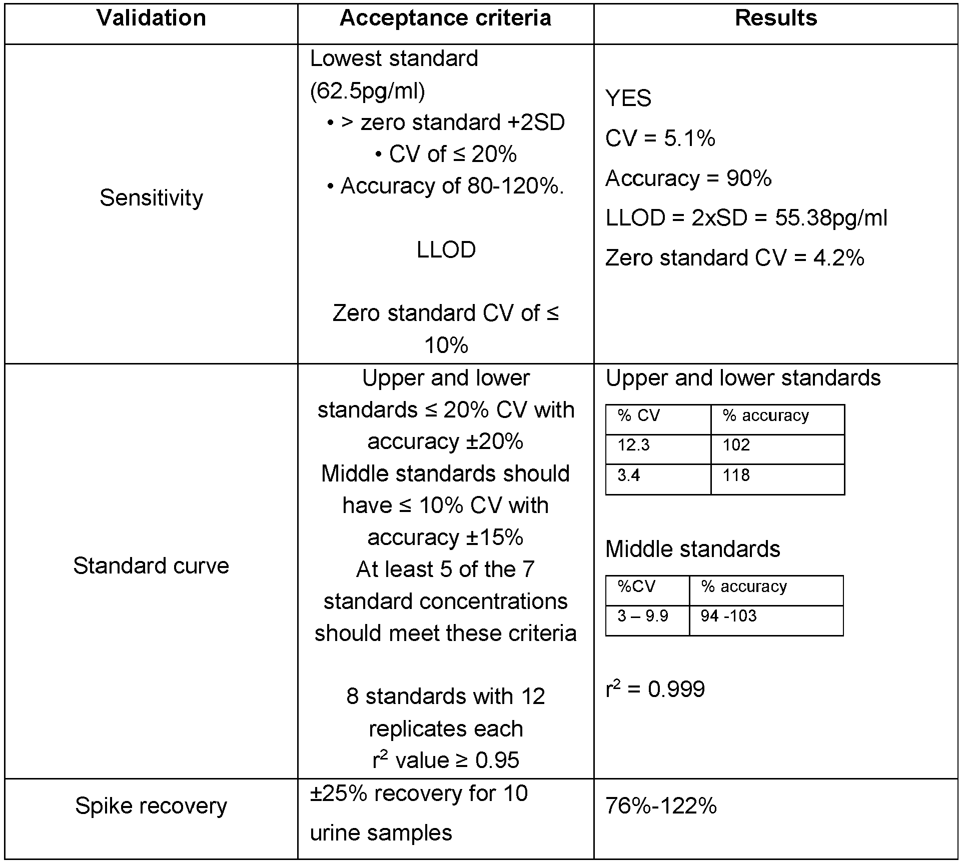

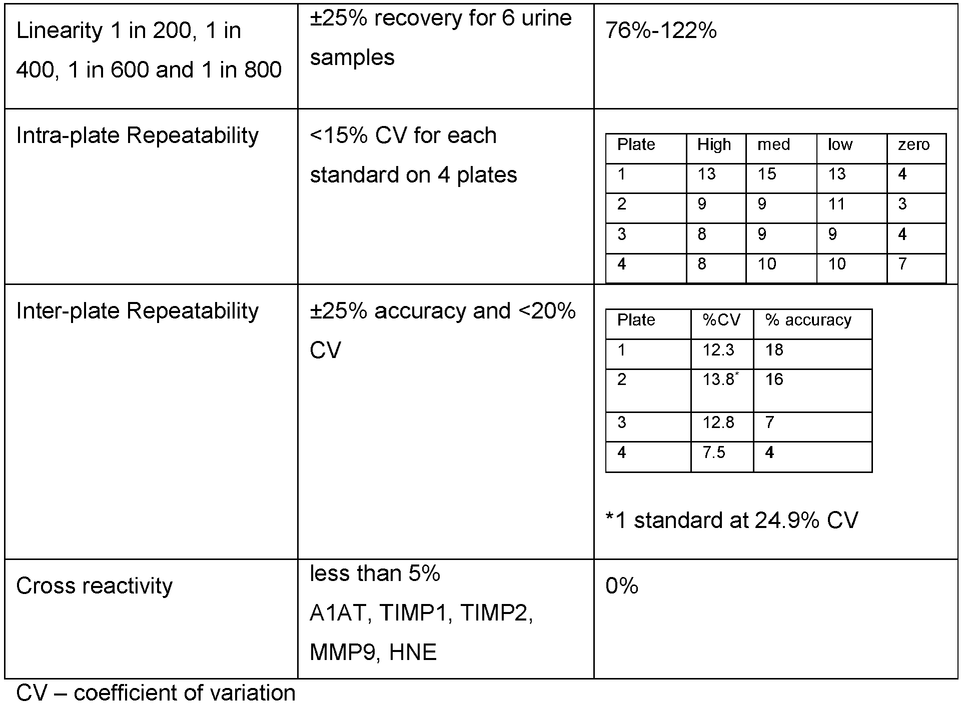

### HNE

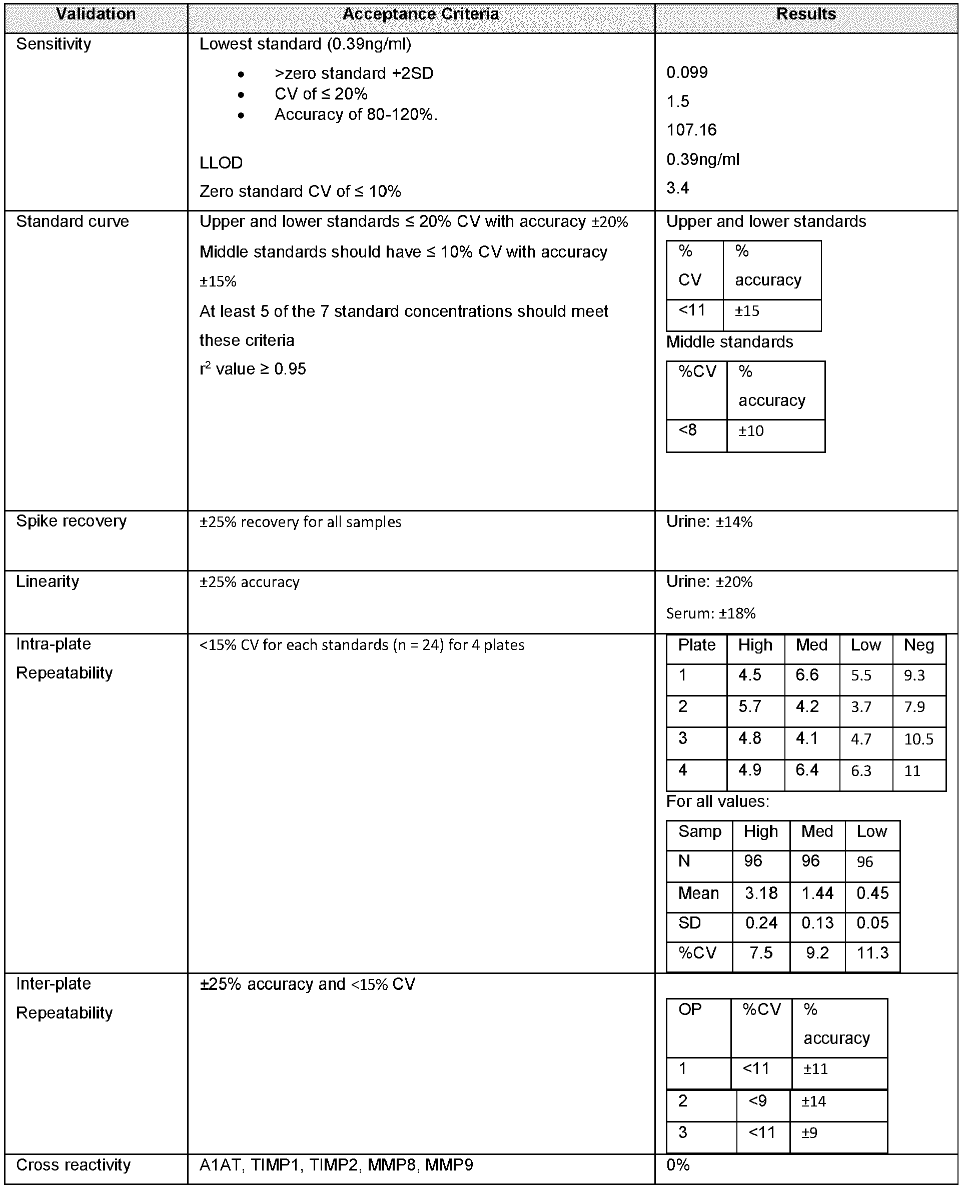

### Cystatin C

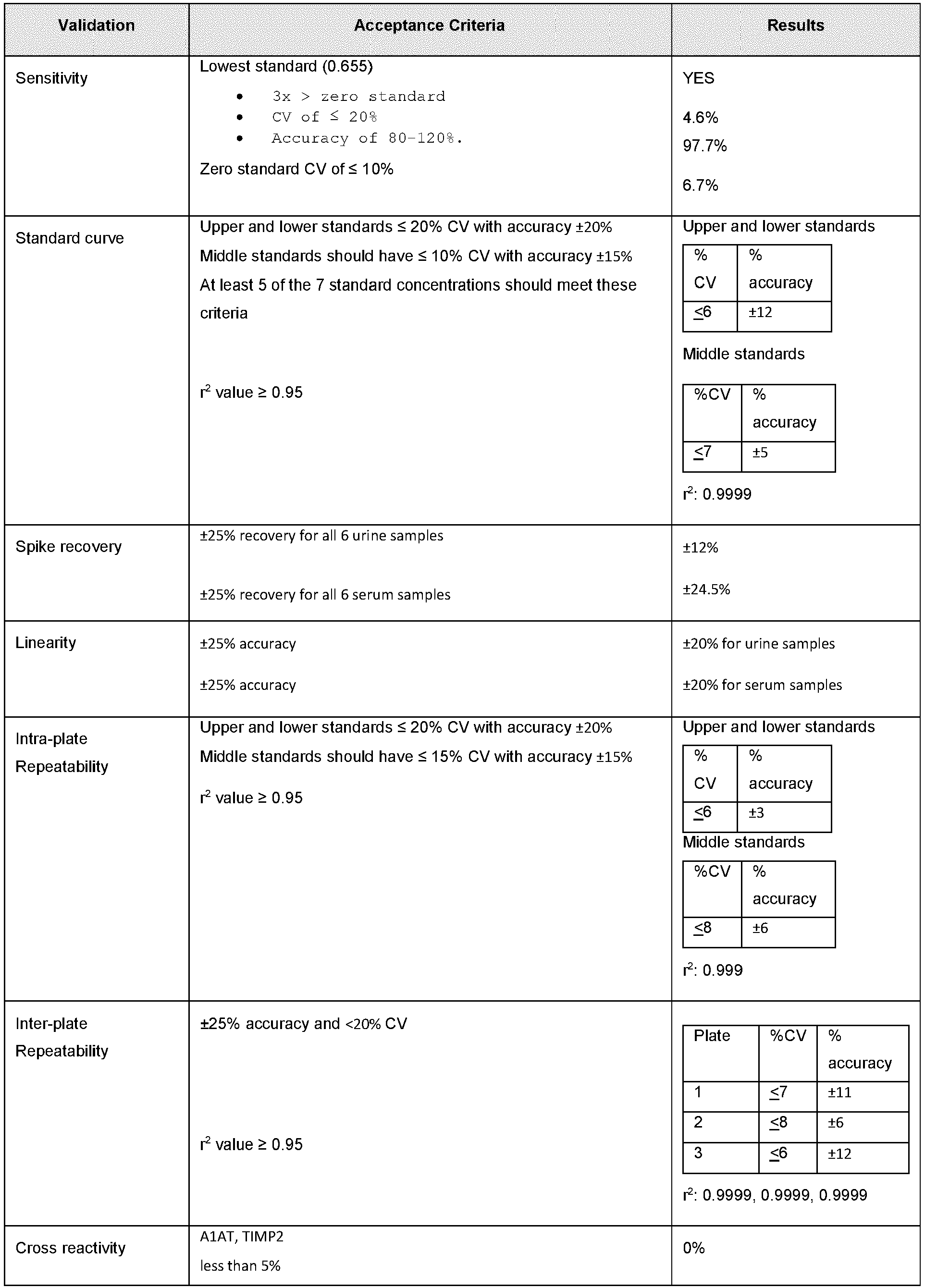

### Example 6 - UTRiPLEX validation

### Sensitivity

For HNE and MMP8 the lowest level of detection (LLOD) was determined by adding two standard deviations to the mean reader value of twenty zero standard replicates and calculating the corresponding concentration.

For Cystatin C the lowest level of detection (LLOD) was determined by subtracting two standard deviations from the mean reader value of twenty zero standard replicates and calculating the corresponding concentration.

LLOD is typically 64.8ng/mL for Cystatin C, 29.2ng/mL for HNE and 0.16ng/mL for MMP8.

### Precision

### Intra-assay precision

Six or seven samples of known concentrations were tested twelve times on one batch of strips by one operator.

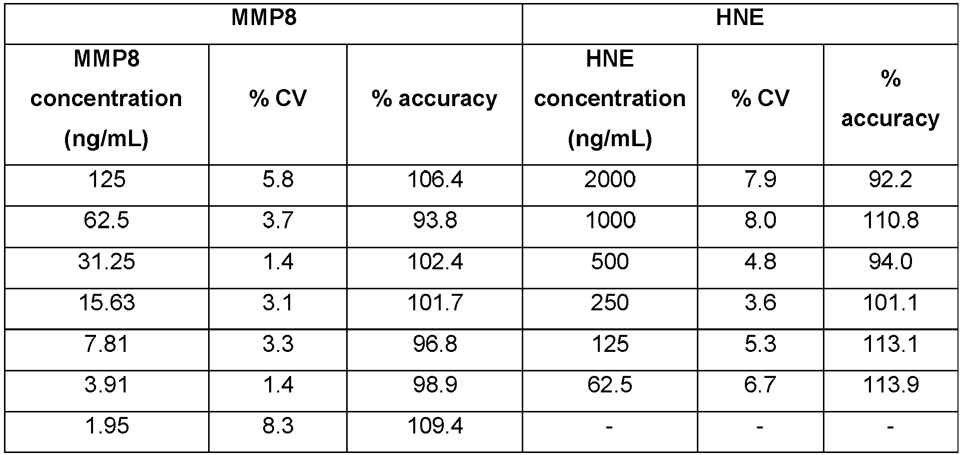

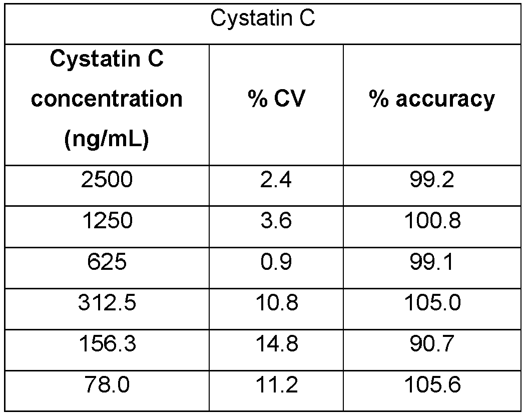

### MMP8

- Average CV: 3.9% (1.4 - 8.3)
- Average accuracy: 101.3% (93.8 - 109.4)

### HNE

- Average CV: 6.0% (3.6 - 8.0)
- Average accuracy: 104.2% (92.2 - 113.9)

### Cystatin C

- Average CV: 7.3% (0.9 - 14.8)
- Average accuracy: 100.1% (90.7 - 106.6)

### Inter-assay precision

Six or seven samples of known concentrations were tested four times on three different batches of strips by two different operators.

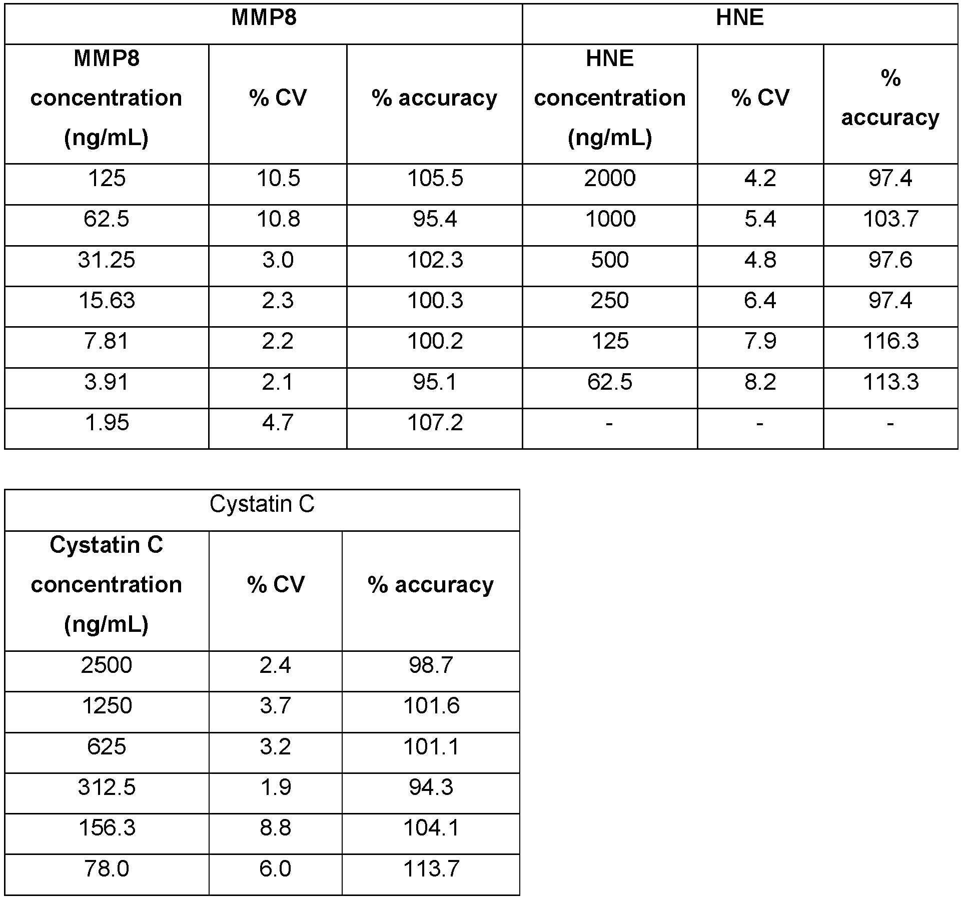

### MMP8

- Average CV: 5.1% (2.1 - 10.8)
- Average accuracy: 100.9% (95.1 - 107.2)

### HNE

- Average CV: 6.2% (4.2 - 8.2)
- Average accuracy: 104.3% (97.4 - 116.3)

### Cystatin C

- Average CV: 4.3% (1.9 - 8.8)
- Average accuracy: 102.3% (94.3 - 113.7)

### Recovery

The recovery of MMP8 spiked into 6 urine samples was evaluated.
- Standard added value: 50ng/mL
- Recovery %: 84.9-110.9
- Average recovery %: 98.0

The recovery of HNE spiked into 6 urine samples was evaluated.
- Standard added value: 750ng/mL
- Recovery %: 86.0-115.4
- Average recovery %: 101.2

The recovery of Cystatin C spiked into 6 urine samples was evaluated.
- Standard added value: 800ng/mL
- Recovery %: 106.7-117.0
- Average recovery %: 111.9

### Specificity

The specificity of the assay was evaluated by measuring the degree of cross-reactivity to various compounds.

| **Cross reactant** | **Concentration (ng/mL)** | **% cross reactivity** | | |
|---|---|---|---|---|
| | | **MMP8** | **HNE** | **Cystatin C** |
| Cystatin C | 2500 | 0 | 0 | N/A |
| HNE | 2000 | 0 | N/A | 0 |
| MMP8 | 500 | N/A | 0 | 0 |
| A1AT | 500 | 0 | 0 | 0 |
| MMP9 | 500 | 0 | 0 | 0 |
| TIMP1 | 500 | 0 | 0 | 0 |
| TIMP2 | 500 | 0 | 0 | 0 |
| CRP | 500 | 0 | 0 | 0 |

### Example 7 -Verification studies

### Fresh and frozen samples

Twelve samples freshly collected were tested on the UTRiPLEX^{™} test and then frozen and tested after 3 days. The results were as follows:

| **MMP8** | **Reader value** | | **Concentration ng/mL** | |
|---|---|---|---|---|
| | *Fresh* | *Frozen* | *Fresh* | *Frozen* |
| Sample 1 | 29.5 | 17.1 | 3.35 | 1.585 |
| Sample 2 | 67 | 21.1 | 7.467 | 2.019 |
| Sample 3 | 136.2 | 83.3 | 18.75 | 9.543 |
| Sample 4 | 214.3 | 187.5 | 63.52 | 57.52 |
| Sample 5 | 227 | 201 | 91.17 | 91.72 |
| Sample 6 | 126 | 61.8 | 16.52 | 6.589 |
| Sample 7 | 48.4 | 44.3 | 4.989 | 4.526 |
| Sample 8 | 63.2 | 76.3 | 6.765 | 8.516 |
| Sample 9 | 45.5 | 59.4 | 4.66 | 6.291 |
| Sample 10 | 111.5 | 108.8 | 14.62 | 14.05 |
| Sample 11 | 111.3 | 118.4 | 14.58 | 16.19 |
| Sample 12 | 10.7 | 14.3 | 0.8512 | 1.272 |

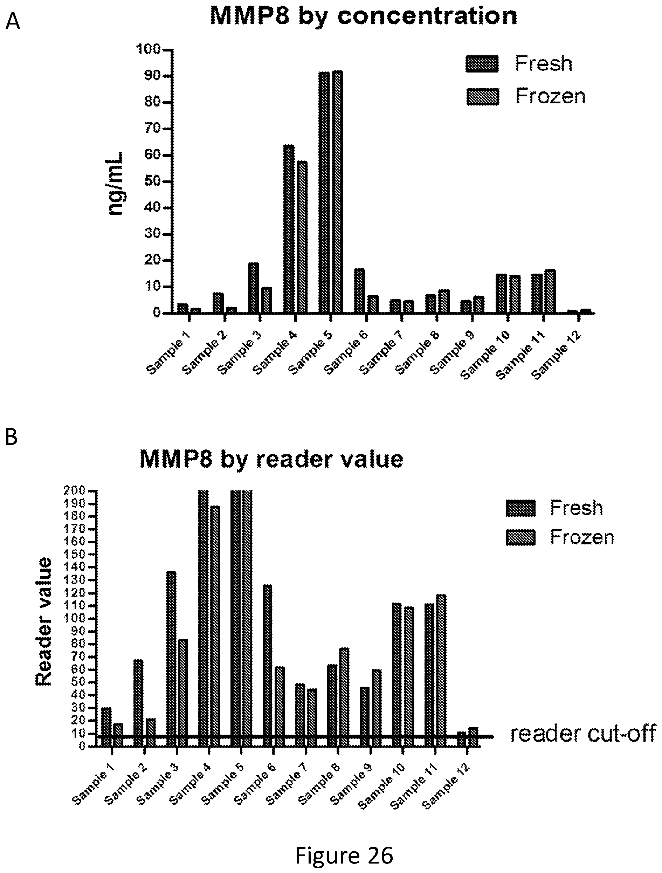

The results are shown graphically in Figure 26.

| **HNE** | **Reader value** | | **Concentration ng/mL** | |
|---|---|---|---|---|
| | *Fresh* | *Frozen* | *Fresh* | *Frozen* |
| Sample 1 | 29.5 | 17.3 | 121.4 | 53.84 |
| Sample 2 | 51.5 | 22.4 | 175.4 | 71.93 |
| Sample 3 | 7.3 | 4.3 | 0 | 0 |
| Sample 4 | 8.7 | 3.1 | 0 | 0 |
| Sample 5 | 30.4 | 19.1 | 124 | 60.45 |
| Sample 6 | 3.1 | 3.5 | 0 | 0 |
| Sample 7 | 3.9 | 1 | 0 | 0 |
| Sample 8 | 3.3 | 4.1 | 0 | 0 |
| Sample 9 | 3.3 | 4.8 | 0 | 0 |
| Sample 10 | 3.9 | 1 | 0 | 0 |
| Sample 11 | 12.4 | 24.3 | 33.73 | 78.24 |
| Sample 12 | 2 | 1.3 | 0 | 0 |

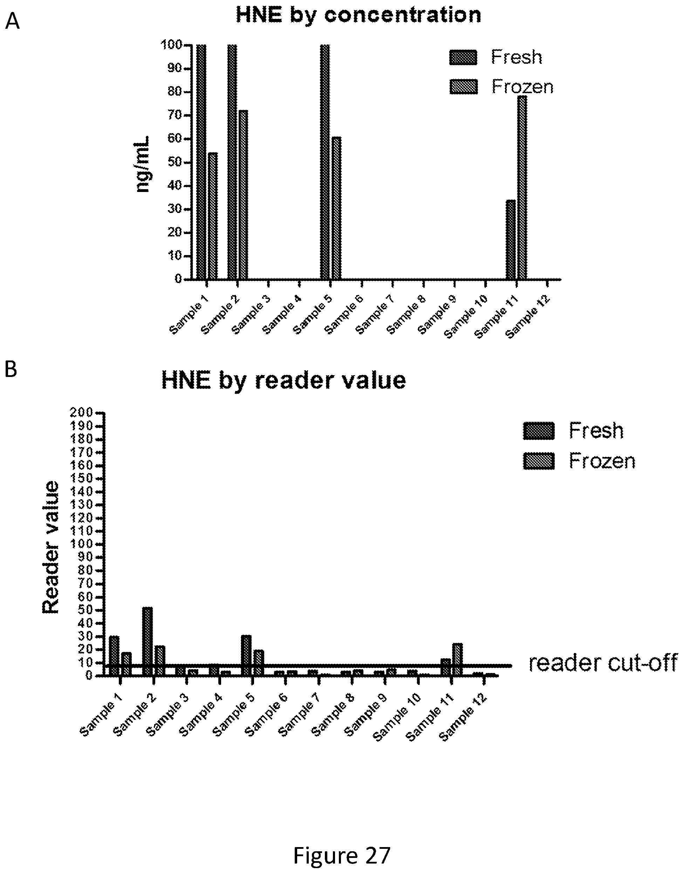

The results are shown graphically in Figure 27.

| **Cystatin C** | **Reader value** | | **Concentration ng/mL** | |
|---|---|---|---|---|
| | *Fresh* | *Frozen* | *Fresh* | *Frozen* |
| Sample 1 | 119 | 106 | 370.1 | 248.2 |
| Sample 2 | 168.5 | 104.6 | 218.3 | 256.2 |
| Sample 3 | 124.5 | 86.5 | 352 | 381.7 |
| Sample 4 | 156.5 | 116.6 | 254.3 | 194.9 |
| Sample 5 | 161.5 | 110 | 239.4 | 226.8 |
| Sample 6 | 151 | 95.1 | 270.6 | 316.1 |
| Sample 7 | 148.5 | 140 | 85.07 | 108.5 |
| Sample 8 | 85.8 | 84.5 | 387.6 | 398.8 |
| Sample 9 | 65 | 81.6 | 615.2 | 425 |
| Sample 10 | 161 | 159 | 56.5 | 60.65 |
| Sample 11 | 143.5 | 180 | 98.41 | 24.09 |
| Sample 12 | 140.4 | 148 | 107.3 | 86.35 |

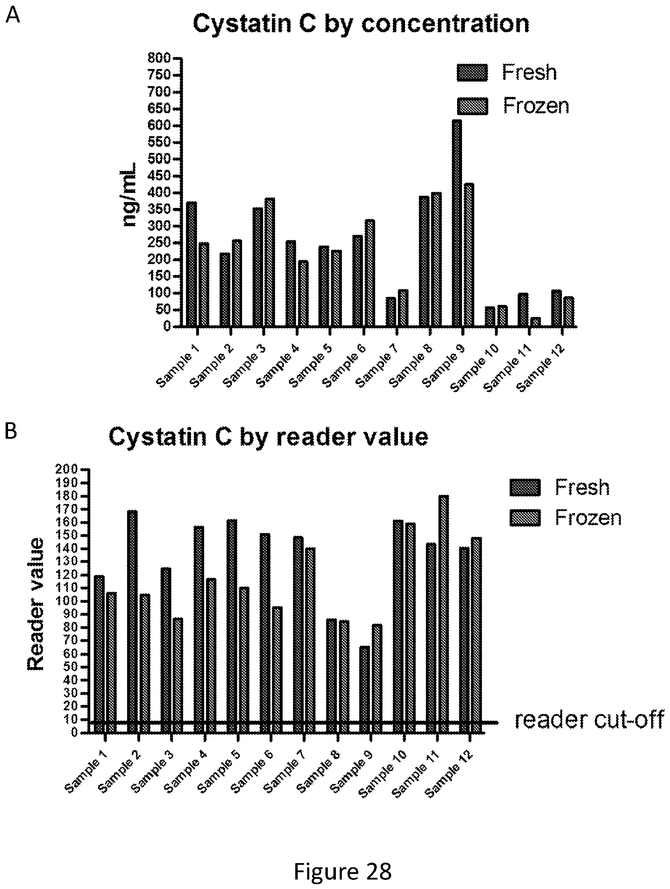

The results are shown graphically in Figure 28.

In general, the results appear to be largely reproducible and stable when frozen.

### UTRiPLEX assay variability with 'healthy' urine over 5 days with multiple operators

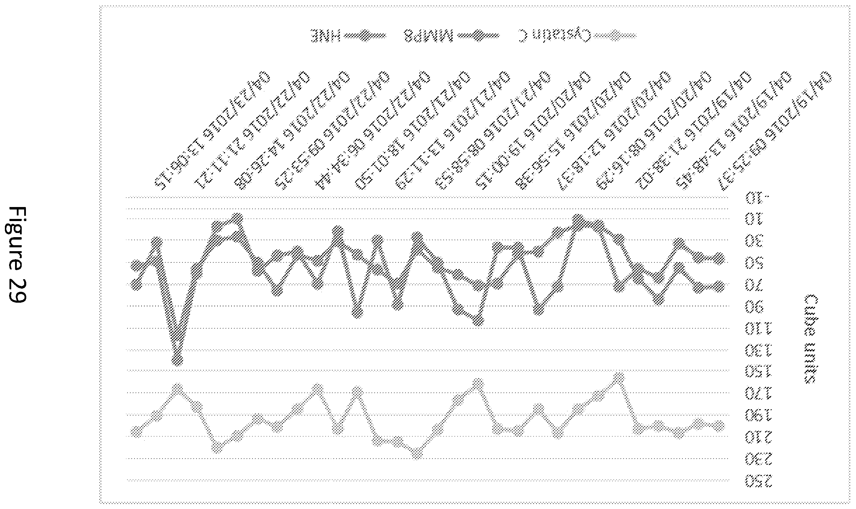

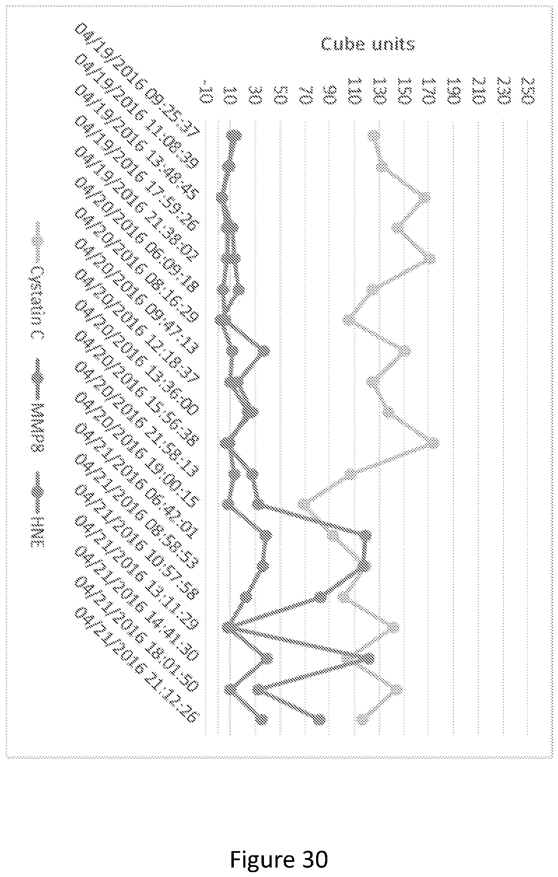

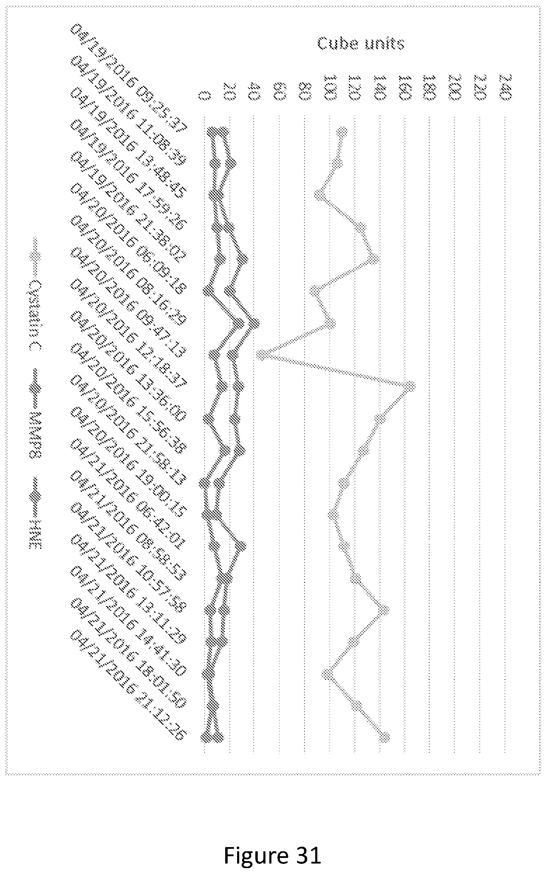

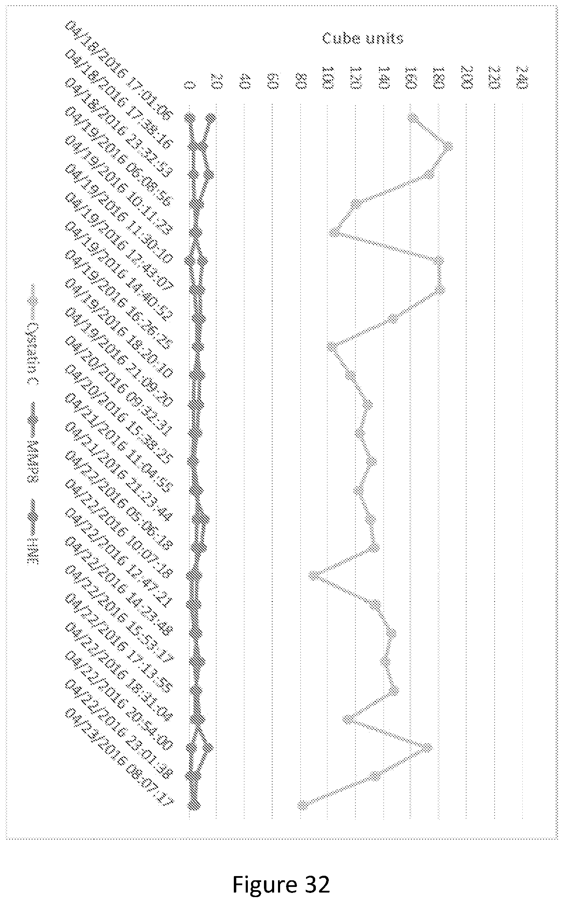

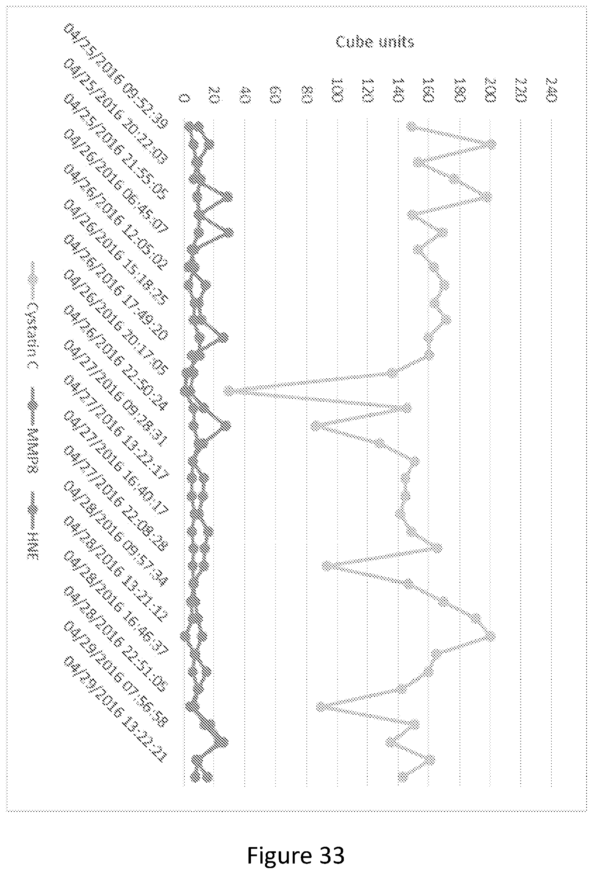

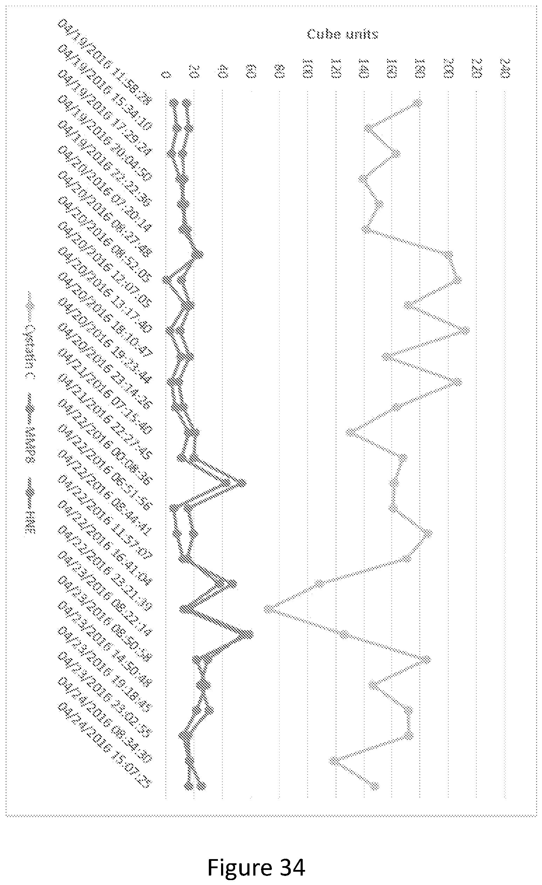

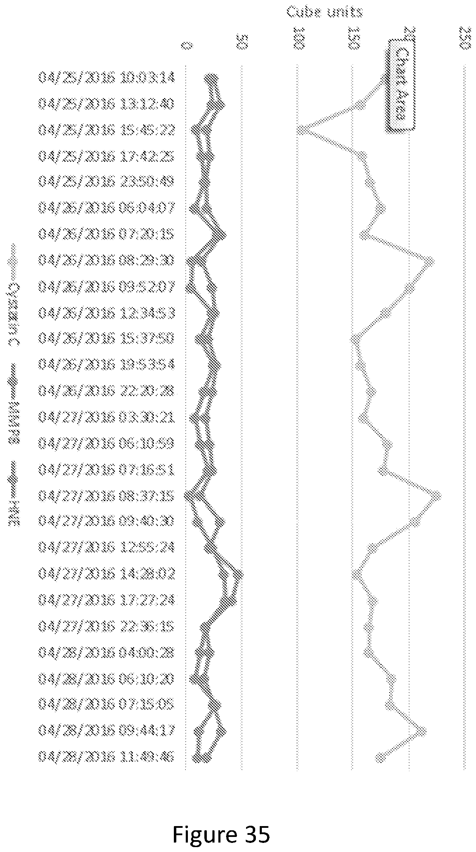

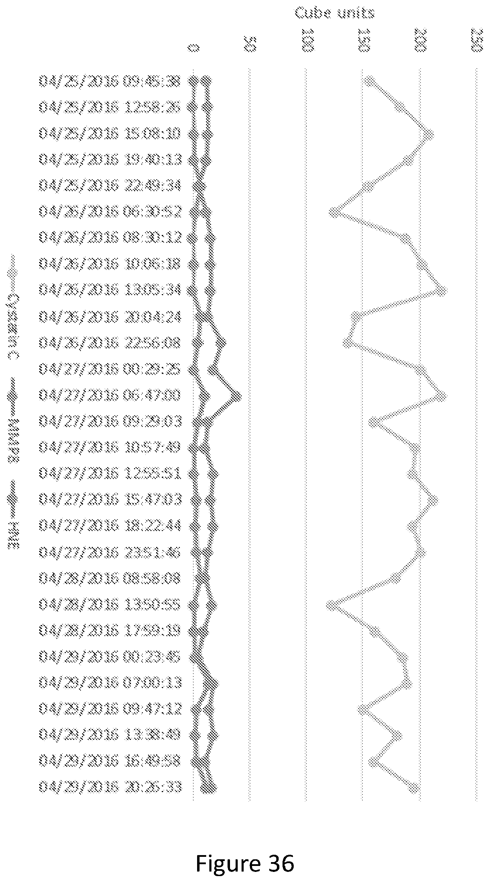

Samples were collected from every urination by 8 'healthy' local volunteers over 5 days, and were tested using UTRiPLEX^{™} strips to explore test performance and results. The results are shown in Figures 29-36 corresponding to volunteers 1-8 respectively. Volunteers 1, 2, 6 and 7 were female. The rest were male. Some variability observed throughout the day, in particular late evening. All variability within acceptable limits.

### Stability of urine over 1 day with multiple operators

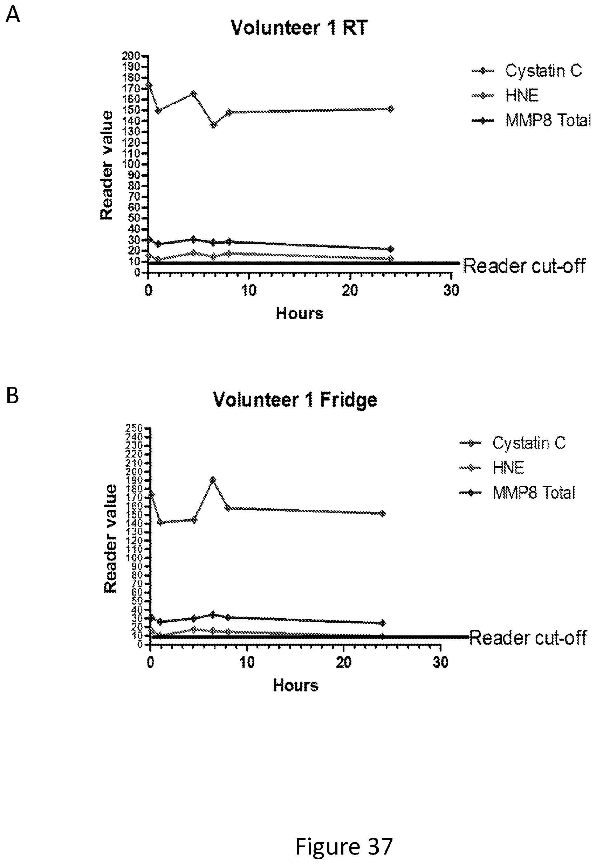

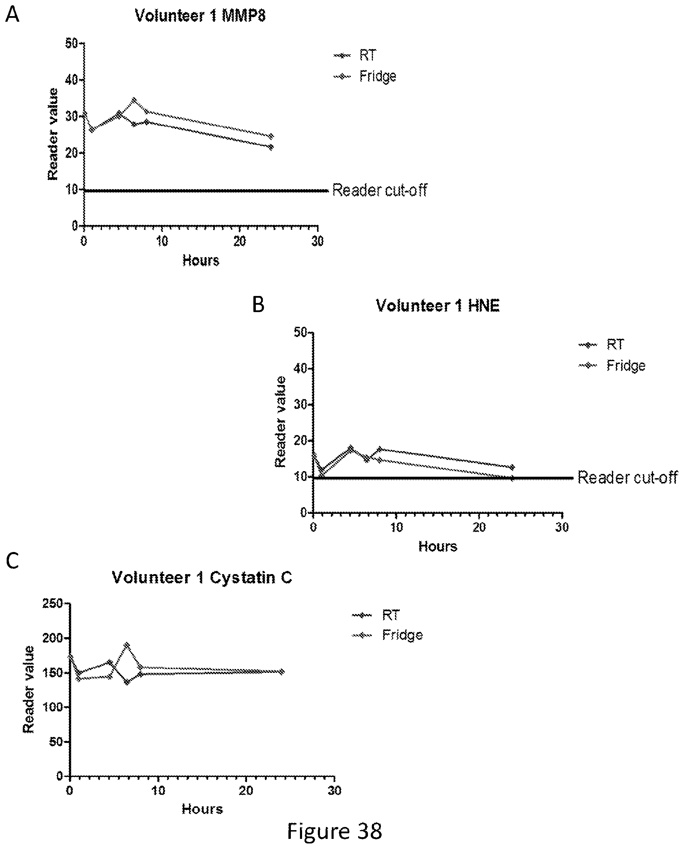

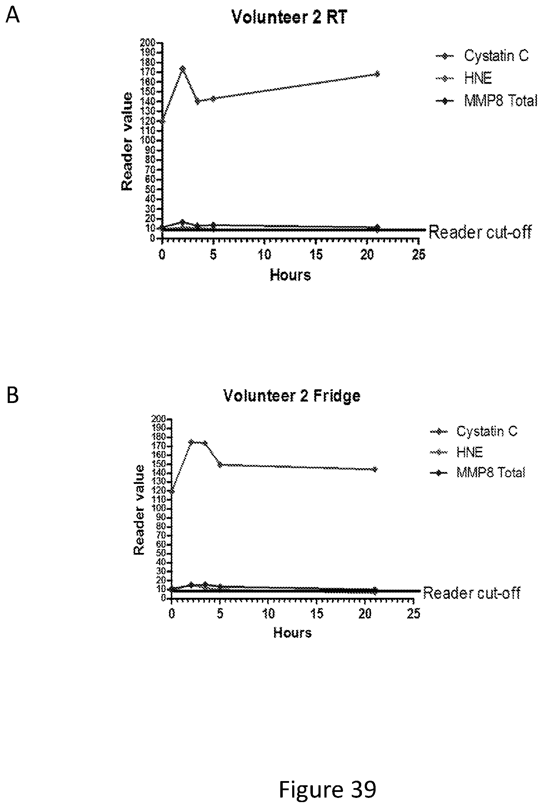

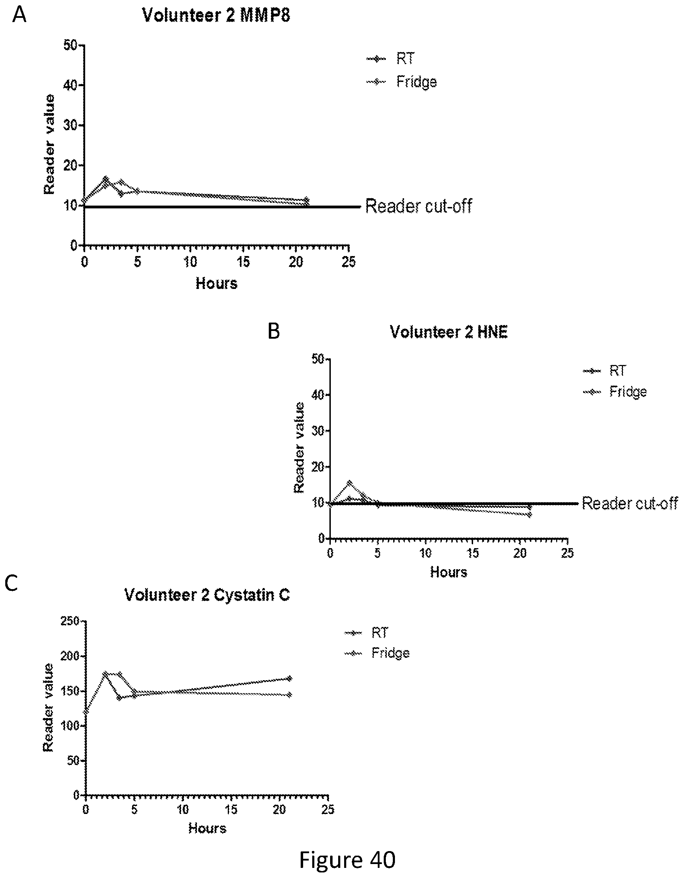

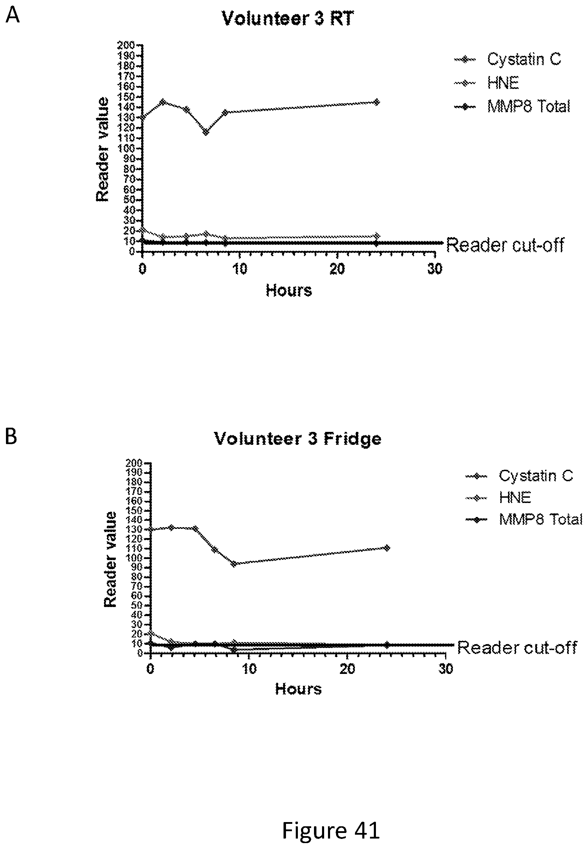

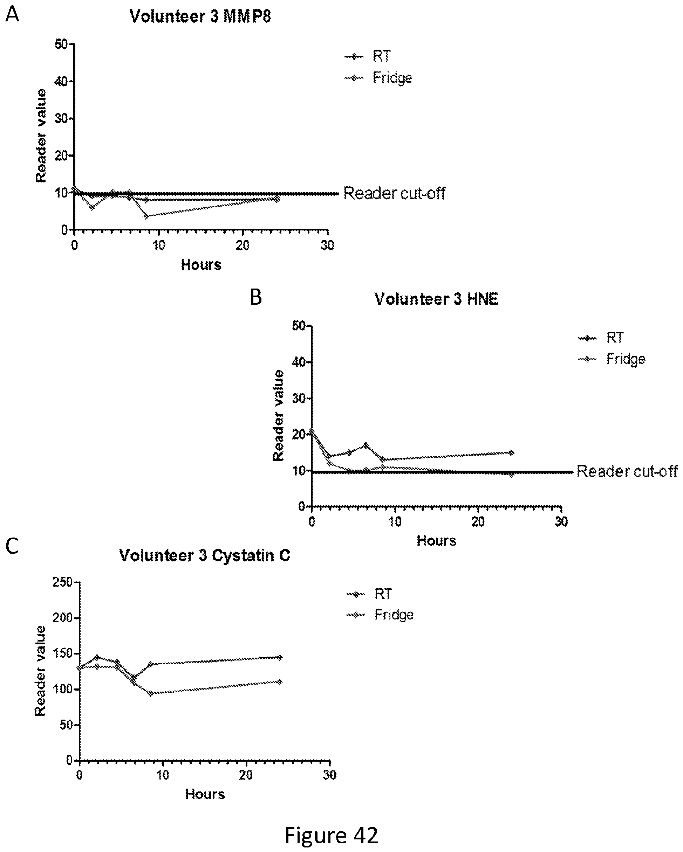

Urine samples may be collected off-site and shipped for next day testing. Therefore an investigation into the stability of the urine samples and the biomarkers levels contained therein was conducted over a 24-hr period from sampling. Urine was collected from 3 local volunteers in the morning and measured straight away using the UTRiPLEX^{™} test assay. The sample was then split into 2 aliquots, and stored either at room temperature or at 4°C (fridge). The samples were tested throughout the day. The results are shown in Figures 37 and 38, 39 and 40, and 41 and 42 corresponding to volunteers 1-3 respectively. All 3 volunteers were female. Samples were deemed stable over a 24 period at room temperature. Next day testing is acceptable including overnight shipping of samples at RT or 2-8°C.

### Stability of the UTRiPLEX test strip over time in storage

One batch of UTI strips was manufactured. The strips were stored in sealed pouches for up to 104 weeks at 3 storage temperatures, 4°C, Room Temperature and 37°C. More strips were placed in the sealed pots which will be used as packaging: one pot opened and then re-sealed until the next test time point and other pots opened only at one specific time point. These pots were stored at room temperature. The data obtained were used to confirm the shelf life of the UTRiPLEX^{™} test strips and evidence >6 month stability.

### Conclusion

The UTRiPLEX test has been successfully validated and is ready for clinical studies. It is a 5-10 minute dip test measuring three biomarkers: Cystatin C, MMP8 and HNE. In the current state it is accompanied with a cube reader until the statistical model is validated.

The table below summarizes the performance of the UTRiPLEX test.

| | **Cystatin C** | **HNE** | **MMP8** |
|---|---|---|---|
| **Standard curve** | 78.12-2500ng/mL | 62.5-2000ng/mL | 1.95-125ng/mL |
| **LLOD** | 64.8ng/mL | 29.2ng/mL | 0.16ng/mL |
| **Intra-assay precision** | CV: 7.3% | CV: 6.0% | CV: 3.9% |
| | Accuracy: 100.1% | Accuracy: 104.2% | Accuracy: 101.3% |
| **Inter-assay precision** | CV: 4.3% | CV: 6.2% | CV: 5.1% |
| | Accuracy: 102.3% | Accuracy: 104.3% | Accuracy: 100.9% |
| **Spike recovery** | 111.9% | 101.2% | 98.0% |
| **(6 urine samples)** | (106.7-117.0) | (86.0-115.4%) | (84.9-110.9%) |
| **Cross reactivity** | 0% | 0% | 0% |
| **Stability** | 2 months at RT | 2 months at RT | 2 months at RT |

With the combined three markers, the sensitivity of the test was 87.7% and the specificity of the test was 96.9%. Four samples from 32 samples that were culture positive were missed using the diagnostic. Only one sample of the 32 negative samples gave a false positive result, whereas all 32 samples gave a false positive result with the method that was used to put them in the 'suspected' UTI group (presumed to be Multistix urinalysis strips and clinical symptoms). Using ROC analysis an AUC of 0.96 was obtained and a p value of <0.0001 using Wilcoxon matched-pairs signed rank test.

### Example 8 - UTI monitoring using the UTRiPLEX test assay

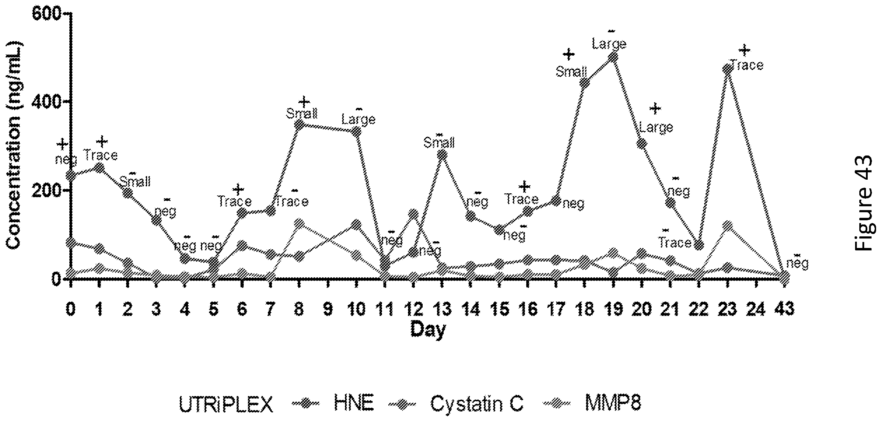

From 1 volunteer, samples were tested daily using UTRiPLEX alongside known Multistix leukocyte and nitrite assays. The results are shown in Figure 43. All samples were deemed to be UTI positive based on culture results tested periodically throughout the period. At day 43, UTRiPLEX markers and nitrite/leukocyte results were negative indicating the end of infection. Throughout the 3-4 week period, the UTRiPLEX markers indicated that the infection was not resolved whilst leukocytes and nitrite levels were positive for a UTI only sporadically. Correlation was good between the UTRiPLEX and Leukocytes (small and large) with all 3 biomarkers positive for these samples.

Thus, the UTRiPLEX assay more reliably and easily detects a UTI than known methods.

### Example 9 - UTRiPLEX assay stability studies

### HNE

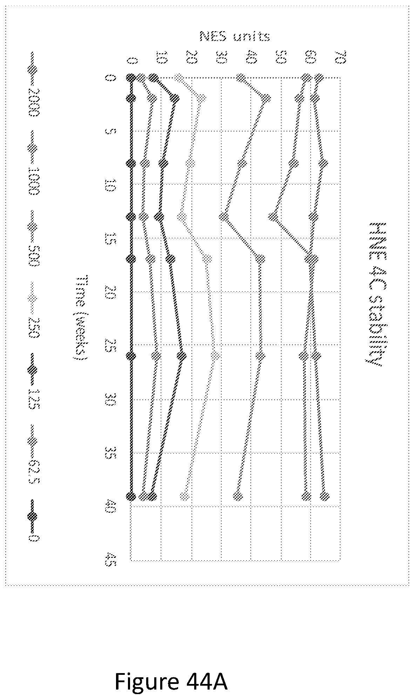

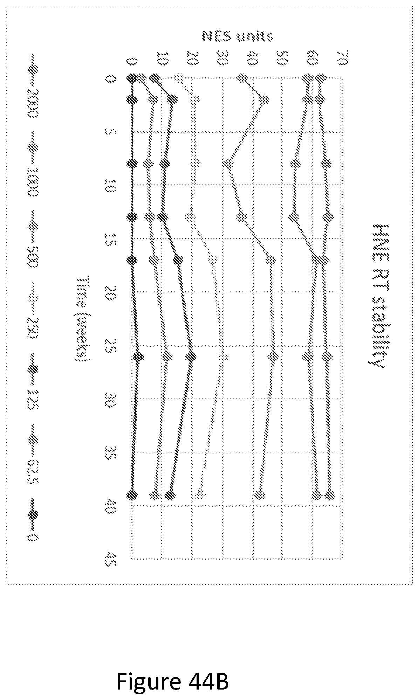

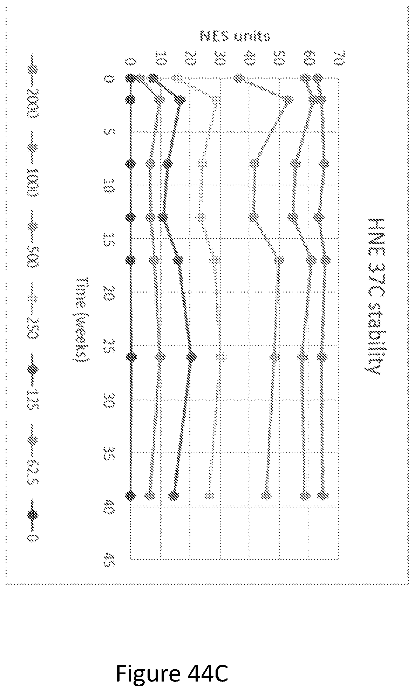

Seven standards at known concentrations (2000ng/mL down to 62.5ng/mL) and one negative were run in duplicate for each storage condition 4°C, Room Temperature (RT) and 37°C and at each time point (weeks). The average reader values were calculated and graphically represented in Figure 44.

### MMP8

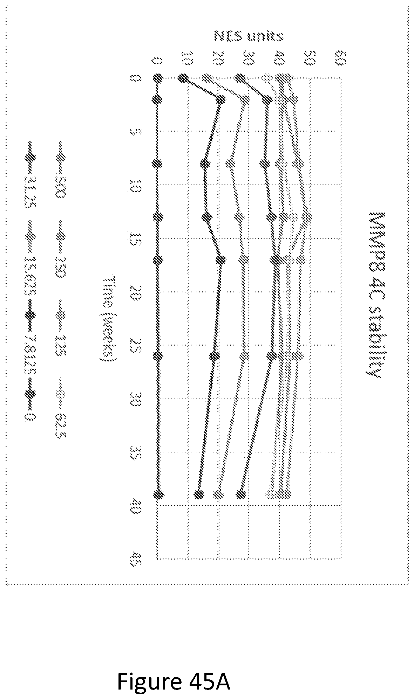

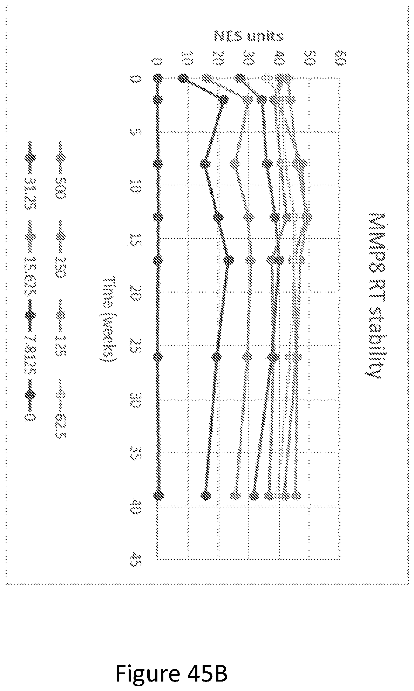

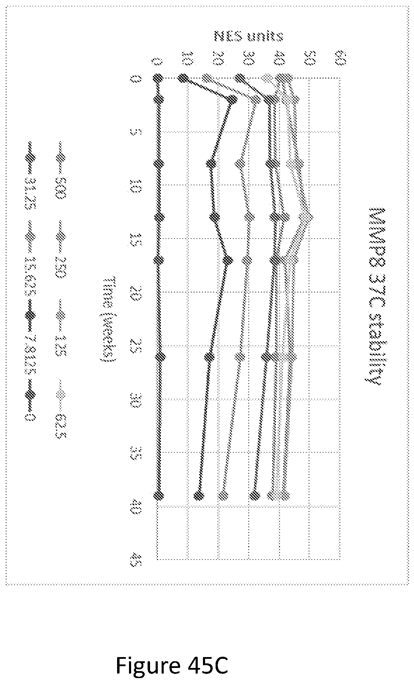

Seven standards at known concentrations (500ng/mL down to 7.8ng/mL) and one negative were run in duplicate for each storage condition 4°C, Room Temperature (RT) and 37°C and at each time point (weeks). The average reader values were calculated and graphically represented in Figure 45.

### Cystatin C (competition)

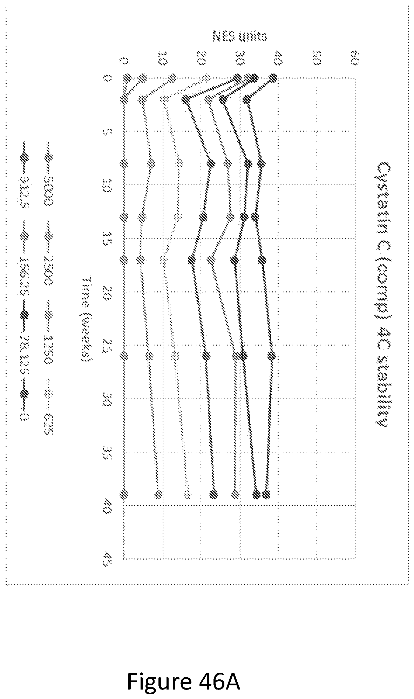

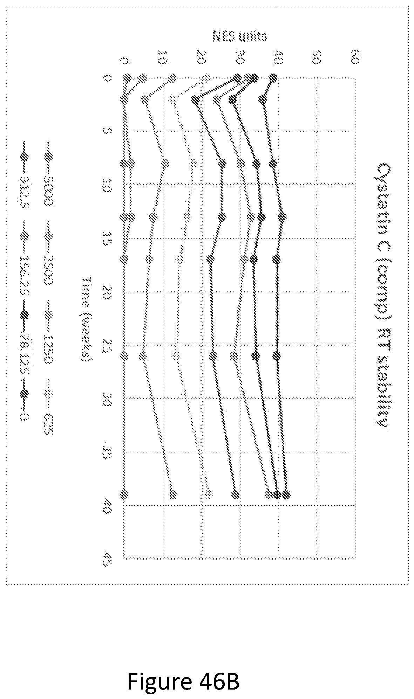

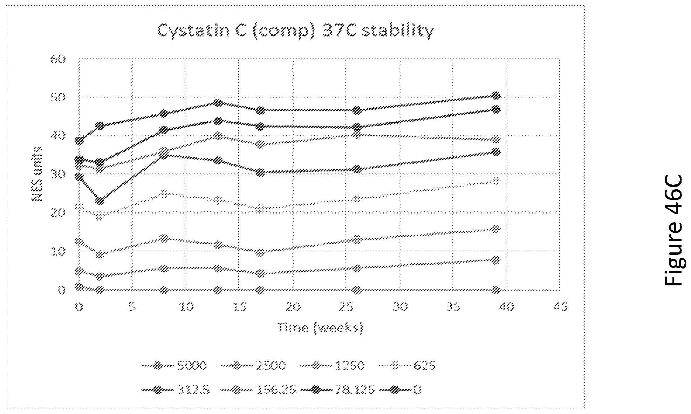

Seven standards at known concentrations (5000ng/mL down to 78.125ng/mL) and one negative were run in duplicate for each storage condition 4°C, Room Temperature (RT) and 37°C and at each time point (weeks). The average reader values were calculated and graphically represented in Figure 46.

### Conclusion

Results met all stability specifications: Accuracy, non-specific binding and signal reduction over time (within acceptable limits). The data obtained were used to confirm the shelf life of the UTRiPLEX^{™} test strips for 9 months at up to 37°C.

### Example 10 - Performance evaluation of the UTRiPLEX strips in a clinical setting

The scope of this performance evaluation was to determine the sensitivity and specificity of the UTRiPLEX^{®} test with urines collected from suspected UTI patients, clinically confirmed by microbiological culture (Gold Standard). Results obtained from the UTRiPLEX^{®} test (using the combined results from cystatin C, HNE and MMP8) for both reader values and visual readings after the recommended time of 6 minutes were used to determine the performance characteristics. Multistix^{®} results were used as GP surgery reference standard.

For this performance evaluation, urines obtained from patients with suspected UTI, that were subsequently clinically confirmed, were evaluated with the UTRiPLEX^{®} strip. The urines were collected from individuals presenting at a GP surgery. These urines were then transferred to a central lab and tested on the UTRiPLEX MUV1 strips and sent off for lab confirmatory tests including culture.

The UTRiPLEX^{®} strips used in this study were manufactured under controlled production processes and conditions which met the QC release specifications.

A total of 38 urines from patients with suspected UTI were available to evaluate the UTRiPLEX strip
- 38 urines have clinical status confirmed by bacterial culture.
   ∘ 10 (26%) had no significant bacterial growth i.e. UTI negative
   ∘ 28 (74%) had bacterial growth i.e. UTI positive

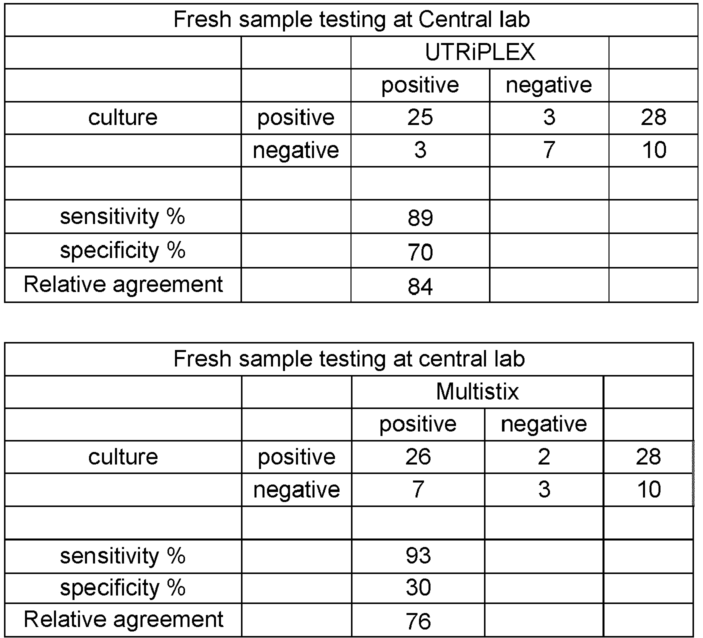

Preliminary results show improved specificity with UTRiPLEX compared to the multistix results and comparable sensitivity.

### Example 11 - Further biomarker development

25 urine samples taken from subjects with culture positive results and 24 urine samples from subjects with culture negative results were analysed with 2 different test strips (per the approach described herein), each consisting of different biomarker combinations;
a. MMP8 + HNE + Fibrinogen
b. MMP8 + HNE + CRP

Both test strips measure three biomarkers, all three biomarker assays are in a sandwich assay format (as described elsewhere herein). In summary, one anti analyte antibody is positioned on the capture line and the second anti analyte antibody is conjugated to gold particles (which may, for instance, be sprayed onto a conjugate glass fibre pad depending on the structure of the specific test strip). Presence of analyte in the sample creates a complex on the capture line which can be seen visually as a red line or measured by a line reader device.

### Visual readings were taken by 1 operator. Reader values obtained using a lateral flow reader (Cube, Optricon)

In the culture positive group, 12% were males. In the culture negative group, 38% were males.

Samples were tested pre- and post-boric acid addition.

### Results

### Individual biomarker analysis (using OD readings provided by a immunochromatographic reader)

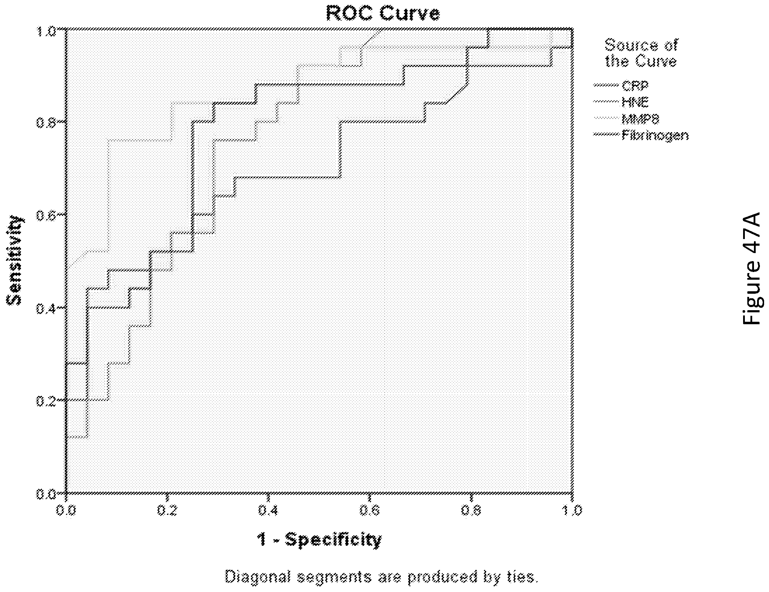

Figure 47A shows (pre-boric acid addition to the samples) the Individual ROC curve for each biomarker. The corresponding AUC was 0.702, 0.769, 0.869 and 0.788 for CRP, HNE, MMP8 and Fibrinogen respectively.

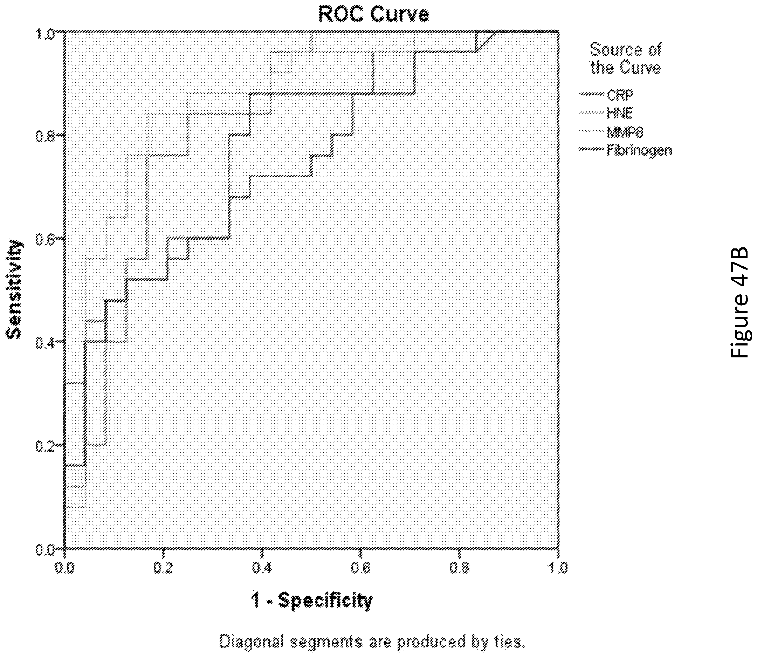

Figure 47B shows (post-boric acid addition to the samples) the Individual ROC curve for each biomarker. The corresponding AUC was 0.756, 0.837, 0.872 and 0.773 for CRP, HNE, MMP8 and Fibrinogen respectively.

### Combined biomarker analysis - visual readings

For these specific experiments, in order to increase confidence in the result, to be considered a UTI positive result, 2 of the 3 lines needed to show a positive result (i.e. presence of a detectable line). To be considered a UTI negative result, 2 of the 3 lines needed to show a negative result (i.e. absence of a detectable line).

The table below shows the diagnostic accuracy of the MMP8 + HNE + Fibrinogen test strip (pre-boric acid) - visual scoring

| MMP8 + HNE + Fibrinogen (pre-boric acid) | Lateral flow strip positive (2/3 biomarkers) | Lateral flow strip negative (2/3 biomarkers) | Total |
|---|---|---|---|
| Culture positive samples | 21 | 4 | 25 |
| Culture negative samples | 6 | 18 | 24 |
| Total | 27 | 22 | 49 |

The results demonstrate that this combination of markers (pre-boric acid addition) has:

| | |
|---|---|
| Sensitivity | 84% |
| Specificity | 75% |
| Relative agreement | 80% |
| Negative Predictive Value (NPV) | 82% |
| Positive Predictive Value (PPV) | 78% |

The table below shows the diagnostic accuracy of the MMP8 + HNE + Fibrinogen test strip (post-boric acid) - visual scoring

| MMP8 + HNE + Fibrinogen (post-boric acid) | Lateral flow strip positive (2/3 biomarkers) | Lateral flow strip negative (2/3 biomarkers) | Total |
|---|---|---|---|
| Culture positive samples | 22 | 3 | 25 |
| Culture negative samples | 5 | 19 | 24 |
| Total | 27 | 22 | 49 |

The results demonstrate that this combination of markers (post-boric acid addition) has:

| | |
|---|---|
| Sensitivity | 88% |
| Specificity | 79% |
| Relative agreement | 84% |
| NPV | 86% |
| PPV | 81% |

The table below shows the diagnostic accuracy of the MMP8 + HNE + CRP test strip (pre-boric acid) - visual scoring

| MMP8 + HNE + CRP (pre-boric acid) | Lateral flow strip positive (2/3 biomarkers) | Lateral flow strip negative (2/3 biomarkers) | Total |
|---|---|---|---|
| Culture positive samples | 21 | 4 | 25 |
| Culture negative samples | 6 | 18 | 24 |
| Total | 27 | 22 | 49 |

The results demonstrate that this combination of markers (pre-boric acid addition) has:

| | |
|---|---|
| Sensitivity | 84% |
| Specificity | 75% |
| Relative agreement | 80% |
| NPV | 82% |
| PPV | 78% |

The table below shows the diagnostic accuracy of the MMP8 + HNE + CRP test strip (post-boric acid) - visual scoring

| MMP8 + HNE + CRP (post-boric acid) | Lateral flow strip positive (2/3 biomarkers) | Lateral flow strip negative (2/3 biomarkers) | Total |
|---|---|---|---|
| Culture positive samples | 21 | 4 | 25 |
| Culture negative samples | 6 | 18 | 24 |
| Total | 27 | 22 | 49 |

The results demonstrate that this combination of markers (post-boric acid addition) has:

| | |
|---|---|
| Sensitivity | 84% |
| Specificity | 75% |
| Relative agreement | 80% |
| NPV | 82% |
| PPV | 78% |

### Combined biomarker analysis - Decision tree analysis using OD readings provided by a immunochromatographic reader

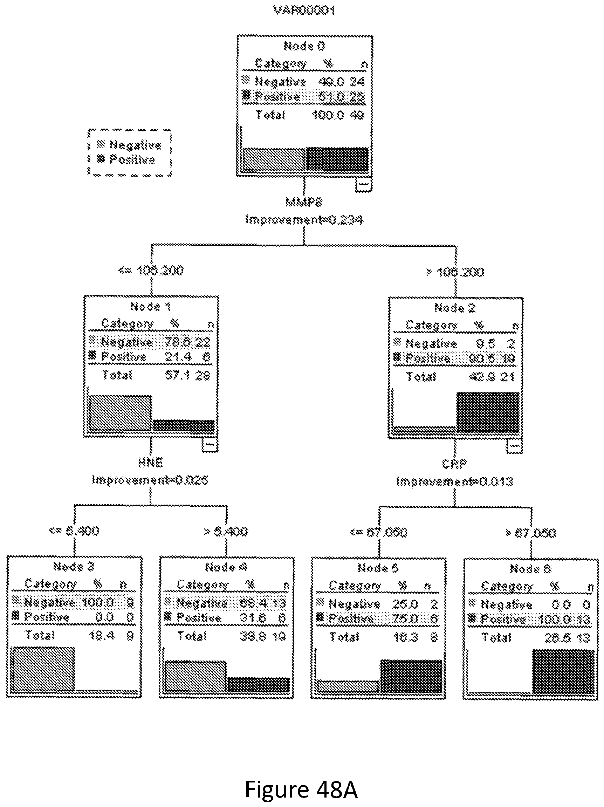

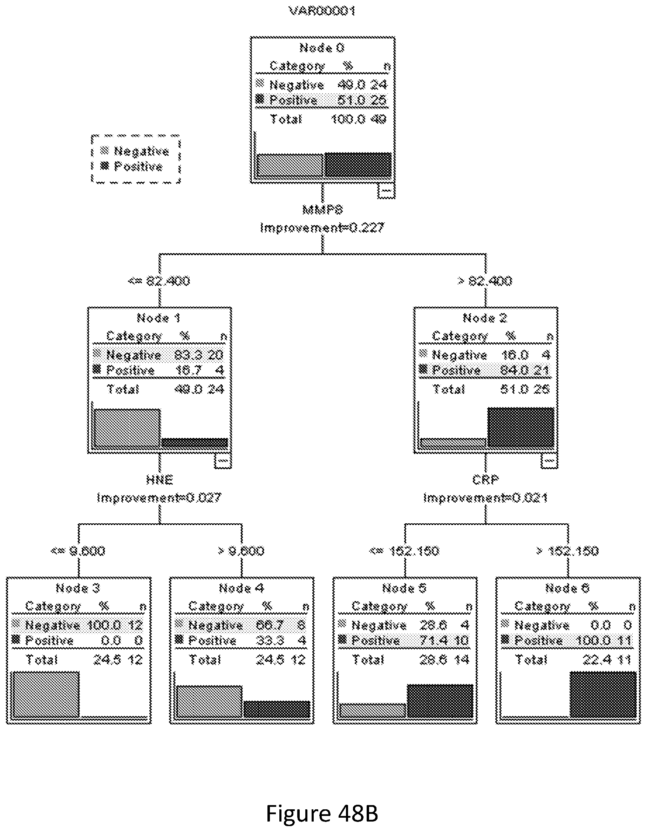

Using decision tree analysis, combination of all three biomarkers CRP + HNE + MMP8 give a sensitivity and specificity of 91.7% and 76% respectively pre-boric acid (Figure 48A) and 83.3% and 84% respectively post-boric acid addition (Figure 48B).

### Summary of the results

ROC AUC for the individual biomarkers ranges from 0.702 - 0.872. The ROC AUC is an indication of the utility of each marker in its ability to discriminate between culture negative and culture positive urine samples. This supports the validity of the use of these biomarkers individually and in combination within the UTI diagnostic panel.

Using a simple visual positive or negative score to provide a diagnostic result, both combinations of biomarkers (MMP8+HNE+Fibrinogen & MMP8+HNE+CRP) provide results with good sensitivity and specificity >75% and NPV >80%. In this current study, presence of boric acid has little effect on the final results.

The Decision tree analysis indicates the cut-off levels for each marker when separating them into culture negative and culture positive groups. This analysis further strengthens the diagnostic utility of these markers in UTI using complex statistical analysis and reader values.

For the avoidance of doubt, the term "comprises" is used in this specification to mean that the subject of the phrase to which comprises relates has the following features but may include other features not stated. That is to say, it is used as an open term. Conversely, the term "consists of" is used in this specification to mean that the subject of the phrase to which consists of relates has the following features and only those features. That is to say, it is used as a closed term.

## Claims

1. A method for detecting a urinary tract infection in a subject comprising:
i) determining the protein level of at least MMP8 in a urine sample obtained from the subject; and
ii) comparing the determined protein level of at least MMP8 with a threshold level wherein an increased protein level of at least MMP8 in the urine sample relative to the threshold level is indicative of the presence of a urinary tract infection.

2. A method for monitoring a urinary tract infection in a subject comprising:
i) determining the protein level of at least MMP8 in urine samples obtained from the subject at multiple time points; and
ii) comparing the determined protein level of at least MMP8 with a threshold level wherein the continued presence of the non-decreased or increased protein level of at least MMP8 relative to the threshold level or relative to the level measured in a sample taken from an earlier time point is indicative that the urinary tract infection persists and/or that treatment has not been effective and/or the decreased protein level of at least MMP8 relative to the threshold level or relative to the level measured in a sample taken from an earlier time point is indicative of recovery from, or successful treatment of, a urinary tract infection.

3. A method for detecting and monitoring a urinary tract infection in a subject comprising:
i) performing the method of claim 1; and, where a urinary tract infection is detected
ii) determining the protein level of at least MMP8 in a further urine sample obtained from the subject at a later time point
wherein a non-decreased or increased protein level of at least MMP8 relative to the threshold level or to the level measured in step (i) is indicative that the urinary tract infection persists and/or a decreased protein level of at least MMP8 relative to the threshold level or to the level measured in step (i) is indicative of recovery from, or successful treatment of, the urinary tract infection.

4. A method for monitoring treatment of a urinary tract infection in a subject comprising:
i) determining the protein level of at least MMP8 in a urine sample obtained from the subject prior to treatment of the urinary tract infection in order to set a threshold level;
ii) determining the protein level of at least MMP8 in a further urine sample obtained from the subject following treatment of the urinary tract infection
wherein a non-decreased or increased protein level of at least MMP8 relative to the threshold level is indicative that the treatment has not been effective and/or a decreased protein level of at least MMP8 relative to the threshold level is indicative of successful treatment of the urinary tract infection.

5. The method of any one of claims 1-4 wherein the protein levels of at least two or three biomarkers are determined, optionally wherein one or more of the biomarkers are selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP.

6. The method of claim 5 wherein non-decreased or increased protein levels of at least two or three, optionally all, of the biomarkers in the urine sample relative to the threshold level or relative to the levels measured in a sample taken from an earlier time point are indicative of the presence of a urinary tract infection.

7. The method of any one of claims 1-6 wherein:
A) at least 2, 3, 4, 5, 6, 7, 8, 9, 10, 11, 12, 13 or 14 or more samples have been taken from the subject at different times and the protein levels of the one or more biomarkers is determined; and/or
B) the samples have been taken every 6 to 24 hours, such as daily, or every 3, 4, 5, 6, 7 or 14 days.

8. A method of selecting a subject for treatment with an antibiotic comprising performing the method of any one of claims 1-7 and selecting the subject for treatment where a urinary tract infection is detected or persists.

9. A method of predicting responsiveness of a subject to treatment with an antibiotic comprising performing the method of any one of claims 1-7 and predicting responsiveness of the subject to treatment where a urinary tract infection is detected or persists.

10. A system or test kit for detecting a urinary tract infection in a subject, comprising:
a. One or more testing devices for determining protein levels of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a urine sample obtained from the subject
b. A processor; and
c. A storage medium comprising a computer application that, when executed by the processor, is configured to:
i. Access and/or calculate the determined protein level of at least MMP8 in the sample on the one or more testing devices
ii. Calculate a test score from the protein level of at least MMP8 in the sample that detects a urinary tract infection; and
iii. Output from the processor the detected result for the subject.

11. A system or test kit for monitoring a urinary tract infection in a subject, comprising:
a. One or more testing devices for determining the protein level of at least MMP8 and optionally one or more biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP in a urine sample obtained from the subject at multiple time points
b. A processor; and
c. A storage medium comprising a computer application that, when executed by the processor, is configured to:
i. Access and/or calculate the determined protein level of at least MMP8 in the sample on the one or more testing devices
ii. Calculate a test score from the protein level of at least MMP8 in the sample, optionally including a comparison of the level with those taken at one or more earlier time points, that detects a urinary tract infection; and
Output from the processor the detected result for the subject.

12. The system or test kit for of claim 10 or 11, wherein the testing device comprises:
a. A sample receiving zone to which a urine sample from a subject is added
b. A conjugate zone comprising at least one labelled binding reagent which specifically binds to MMP8 and optionally one or two labelled binding reagents, each of which specifically binds to one of the biomarkers selected from HNE, Cystatin C, MMP9, HSA, IL-8, interleukin-6 (IL-6), interleukin-1 beta (IL-1b), fibrinogen, RBP4, active MMP9 and MMP2, NGAL, Desmosine, MPO and CRP
c. A solid support defining a liquid flow path for the sample and comprising a corresponding test line for at least MMP8, the test line comprising:
i. an immobilised further binding reagent that also specifically binds to MMP8 thereby immobilising MMP8 at the test line to produce a signal via the labelled binding reagent also specifically bound to MMP8; or
ii. an immobilised version of MMP8 or an analogue thereof able to compete with MMP8 in the sample for specific binding to the labelled binding reagent;
optionally wherein the testing device further comprises:
at least one labelled control binding reagent that binds to a binding partner immobilised at a control line downstream of the test line for MMP8 and thus confirms that the test has completed successfully;
and/or further comprising:
an absorbent material downstream of the test (and control, where present) lines to absorb excess sample.

13. The system or test kit for of claim 12, wherein:
A) the solid support comprises a chromatographic medium;
B) the solid support comprises a capillary flow device;
C) the testing device is a test strip;
D) the testing device further comprises a vessel for collecting a urine sample;
E) the testing device further comprises a visual aid displaying different test line intensity patterns from which the user can interpret the observed test line results; and/or
F) the testing device further comprises a reader to determine protein levels of the markers at the respective test lines.

14. The method of any one of claims 1-9 or the system or test kit of any one of claims 10-13 wherein the biomarkers comprise:
a. MMP8 and HNE;
b. MMP8 and HNE and Cystatin C;
c. MMP8, HNE and fibrinogen;
d. MMP8, HNE and fibrinogen and Cystatin C;
e. MMP8, HNE and CRP; or
f. MMP8, HNE and CRP and Cystatin C.

15. The method, system or test kit of claim 14 wherein the test line for each biomarker respectively comprises an immobilised further binding reagent that also specifically binds to each biomarker respectively thereby immobilising each biomarker at the respective test line to produce a signal via the labelled binding reagent also specifically bound to each biomarker.

16. The method, system or test kit of claim 14 or 15, wherein the test line for Cystatin C comprises an immobilised version of Cystatin C or an analogue thereof able to compete with Cystatin C in the sample for specific binding to the labelled binding reagent.

17. The method of any one of claims 1-9 or 14-16, wherein the protein level of at least MMP8 is detected using a lateral flow assay.

## Patentansprüche

1. Verfahren zum Nachweis einer Harnwegsinfektion bei einer Patienten, umfassend:
i) Ermitteln des Proteinspiegels von mindestens MMP8 in einer von dem Patienten erhaltenen Urinprobe; und
ii) Vergleichen des ermittelten Proteinspiegels von mindestens MMP8 mit einem Schwellenwert, wobei ein erhöhter Proteinspiegel von mindestens MMP8 in der Urinprobe im Verhältnis zum Schwellenwert auf das Vorliegen einer Harnwegsinfektion hinweist.

2. Verfahren zum Überwachen einer Harnwegsinfektion bei einem Patienten, mit den Schritten:
i) Ermitteln des Proteinspiegels von mindestens MMP8 in Urinproben, die von dem Patienten zu mehreren Zeitpunkten erhalten wurden; und
ii) Vergleichen des ermittelten Proteinspiegels von mindestens MMP8 mit einem Schwellenwert, wobei das fortgesetzte Vorhandensein des in Bezug auf den Schwellenwert oder in Bezug auf den in einer zu einem früheren Zeitpunkt entnommenen Probe gemessenen Spiegel nicht verringerten oder erhöhten Proteinspiegels von mindestens MMP8 ein Anzeichen dafür ist, dass die Harnwegsinfektion fortbesteht und/oder dass die Behandlung nicht wirksam war, und/oder der in Bezug auf den Schwellenwert oder in Bezug auf den in einer zu einem früheren Zeitpunkt entnommenen Probe gemessenen Spiegel verringerte Proteinspiegel von mindestens MMP8 ein Anzeichen für die Genesung von einer Harnwegsinfektion oder für deren erfolgreiche Behandlung ist.

3. Verfahren zum Nachweisen und Überwachen einer Harnwegsinfektion bei einem Patienten, umfassend:
i) Durchführen des Verfahrens nach Anspruch 1; und, wenn eine Harnwegsinfektion nachgewiesen wird
ii) Ermitteln des Proteinspiegels von mindestens MMP8 in einer weiteren, von dem Patienten zu einem späteren Zeitpunkt erhaltenen Urinprobe,
wobei ein im Vergleich zum Schwellenwert oder zu dem in Schritt (i) gemessenen Wert nicht verringerter oder erhöhter Proteinspiegel von mindestens MMP8 anzeigt, dass die Harnwegsinfektion fortbesteht, und/oder ein im Vergleich zum Schwellenwert oder zu dem in Schritt (i) gemessenen Wert verringerter Proteinspiegel von mindestens MMP8 eine Erholung von der Harnwegsinfektion oder eine erfolgreiche Behandlung derselben anzeigt.

4. Verfahren zum Überwachen der Behandlung einer Harnwegsinfektion bei einem Patienten, mit den Schritten:
i) Ermitteln des Proteinspiegels von mindestens MMP8 in einer Urinprobe, die von dem Patienten vor der Behandlung der Harnwegsinfektion erhalten wurde, um einen Schwellenwert festzulegen;
ii) Bestimmen des Proteinspiegels von mindestens MMP8 in einer weiteren, von dem Patienten nach der Behandlung der Harnwegsinfektion erhaltenen Urinprobe,
wobei ein nicht verringerter oder erhöhter Proteinspiegel von mindestens MMP8 relativ zum Schwellenwert anzeigt, dass die Behandlung nicht wirksam war, und/oder ein verringerter Proteinspiegel von mindestens MMP8 relativ zum Schwellenwert eine erfolgreiche Behandlung der Harnwegsinfektion anzeigt.

5. Verfahren nach einem der Ansprüche 1 bis 4, bei dem die Proteinkonzentrationen von mindestens zwei oder drei Biomarkern bestimmt werden, wobei optional ein oder mehrere der Biomarker ausgewählt werden aus HNE, Cystatin C, MMP9, HSA, IL-8, Interleukin-6 (IL-6), Interleukin-1beta (IL-1b), Fibrinogen, RBP4, aktivem MMP9 und MMP2, NGAL, Desmosin, MPO und CRP.

6. Verfahren nach Anspruch 5, bei dem nicht verringerte oder erhöhte Proteinkonzentrationen von mindestens zwei oder drei, optional allen, der Biomarker in der Urinprobe relativ zum Schwellenwert oder relativ zu den in einer zu einem früheren Zeitpunkt entnommenen Probe gemessenen Konzentrationen auf das Vorliegen einer Harnwegsinfektion hinweisen.

7. Verfahren nach einem der Ansprüche 1 bis 6, bei dem:
A) mindestens 2, 3, 4, 5, 6, 7, 8, 9, 10, 11, 12, 13 oder 14 oder mehr Proben von dem Patienten zu unterschiedlichen Zeitpunkten entnommen wurden und die Proteinspiegel der ein oder mehreren Biomarker ermittelt werden; und/oder
B) die Proben alle 6 bis 24 Stunden, z. B. täglich, oder alle 3, 4, 5, 6, 7 oder 14 Tage entnommen worden sind.

8. Verfahren zum Auswählen eines Patienten für die Behandlung mit einem Antibiotikum, umfassend die Durchführung des Verfahrens nach einem der Ansprüche 1 bis 7 und das Auswählen des Patienten für die Behandlung, wenn eine Harnwegsinfektion festgestellt wird oder andauert.

9. Verfahren zum Vorhersagen des Ansprechens eines Patienten auf die Behandlung mit einem Antibiotikum, umfassend die Durchführung des Verfahrens nach einem der Ansprüche 1 bis 7 und das Vorhersagen des Ansprechens des Patienten auf die Behandlung, wenn eine Harnwegsinfektion festgestellt wird oder andauert.

10. System oder Testkit zum Nachweisen einer Harnwegsinfektion bei einem Patienten, umfassend:
a. eine oder mehrere Testvorrichtungen zum Ermitteln der Proteinkonzentrationen von mindestens MMP8 und optional eines oder mehrerer aus HNE, Cystatin C, MMP9, HSA, IL-8, Interleukin-6 (IL-6), Interleukin-1beta (IL-1b), Fibrinogen, RBP4, aktivem MMP9 und MMP2, NGAL, Desmosin, MPO und CRP ausgewählter Biomarker in einer von dem Patienten erhaltenen Urinprobe;
b. einen Prozessor; und
c. ein Speichermedium, das eine Computeranwendung umfasst, die, wenn sie von dem Prozessor ausgeführt wird, konfiguriert ist zum:
i. Zugreifen auf den ermittelten Proteinspiegel von mindestens MMP8 in der Probe auf den ein oder mehreren Testvorrichtungen und/oder Berechnen desselben;
ii. Berechnen eines Testwertes aus dem Proteinspiegel von mindestens MMP8 in der Probe, der eine Harnwegsinfektion nachweist; und
iii. Ausgeben des für den Patienten festgestellten Ergebnisses durch den Prozessor.

11. System oder Testkit zum Überwachen einer Harnwegsinfektion bei einem Patienten, umfassend:
a. ein oder mehrere Testvorrichtungen zum Ermitteln des Proteinspiegels von mindestens MMP8 und optional eines oder mehrerer Biomarker, ausgewählt aus HNE, Cystatin C, MMP9, HSA, IL-8, Interleukin-6 (IL-6), Interleukin-1beta (IL-1b), Fibrinogen, RBP4, aktivem MMP9 und MMP2, NGAL, Desmosin, MPO und CRP in einer von dem Patienten zu mehreren Zeitpunkten erhaltenen Urinprobe
b. einen Prozessor; und
c. ein Speichermedium, das eine Computeranwendung umfasst, die, wenn sie von dem Prozessor ausgeführt wird, konfiguriert ist zum:
i. Zugreifen auf den ermittelten Proteinspiegel von mindestens MMP8 in der Probe auf den ein oder mehreren Testvorrichtungen und/oder Berechnen desselben;
ii. Berechnen eines Testergebnisses aus dem Proteinspiegel von mindestens MMP8 in der Probe, optional einschließlich eines Vergleichs des Spiegels mit denen, die zu einem oder mehreren früheren Zeitpunkten genommen wurden, das eine Harnwegsinfektion nachweist; und
Ausgeben des für den Patienten ermittelten Ergebnisses durch den Prozessor.

12. System oder Testkit nach Anspruch 10 oder 11, bei dem die Testvorrichtung umfasst:
a. eine Probenaufnahmezone, in die eine Urinprobe von einem Patienten gegeben wird
b. eine Konjugatzone, die mindestens ein markiertes Bindungsreagenz, das spezifisch an MMP8 bindet, und optional ein oder zwei markierte Bindungsreagenzien umfasst, von denen jedes spezifisch an einen der Biomarker bindet, die aus HNE, Cystatin C, MMP9, HSA, IL-8, Interleukin-6 (IL-6), Interleukin-1beta (IL-1b), Fibrinogen, RBP4, aktivem MMP9 und MMP2, NGAL, Desmosin, MPO und CRP ausgewählt sind,
c. einen festen Träger, der einen Fluiddurchflussweg für die Probe definiert und eine entsprechende Testlinie für mindestens MMP8 umfasst, wobei die Testlinie umfasst:
i. ein immobilisiertes weiteres Bindungsreagenz, das ebenfalls spezifisch an MMP8 bindet, wodurch MMP8 an der Testlinie immobilisiert wird, um über das ebenfalls spezifisch an MMP8 gebundene markierte Bindungsreagenz ein Signal zu erzeugen; oder
ii. eine immobilisierte Version von MMP8 oder eines Analogons davon, die mit MMP8 in der Probe um die spezifische Bindung an das markierte Bindungsreagenz konkurrieren kann;
optional, wobei die Testvorrichtung ferner umfasst:
mindestens ein markiertes Kontrollbindungsreagenz, das an einen Bindungspartner bindet, der an einer Kontrolllinie stromabwärts der Testlinie für MMP8 immobilisiert ist, und somit bestätigt, dass der Test erfolgreich abgeschlossen wurde;
und/oder ferner umfasst:
ein absorbierendes Material stromabwärts der Test- (und, sofern vorhanden, Kontroll-) -linie, um überschüssige Probe zu absorbieren.

13. System oder Testkit nach Anspruch 12, bei dem:
A) der feste Träger ein chromatographisches Medium umfasst;
B) der feste Träger eine Kapillarflussvorrichtung umfasst;
C) die Testvorrichtung ein Teststreifen ist;
D) die Testvorrichtung ferner ein Gefäß zum Sammeln einer Urinprobe umfasst;
E) die Testvorrichtung ferner ein visuelles Hilfsmittel umfasst, das verschiedene Testlinienintensitätsmuster anzeigt, anhand derer der Benutzer die beobachteten Testlinienergebnisse interpretieren kann; und/oder
F) die Testvorrichtung ferner ein Lesegerät umfasst, um die Proteinspiegel der Marker an den jeweiligen Testlinien zu bestimmen.

14. Verfahren nach einem der Ansprüche 1-9 oder System oder Testkit nach einem der Ansprüche 10-13, bei dem die Biomarker umfassen:
a. MMP8 und HNE;
b. MMP8 und HNE und Cystatin C;
c. MMP8, HNE und Fibrinogen;
d. MMP8, HNE und Fibrinogen und Cystatin C;
e. MMP8, HNE und CRP; oder
f. MMP8, HNE und CRP und Cystatin C.

15. Verfahren, System oder Testkit nach Anspruch 14, bei dem die Testlinie für jeden Biomarker jeweils ein immobilisiertes weiteres Bindungsreagenz umfasst, das ebenfalls spezifisch an jeden Biomarker bindet, wodurch jeder Biomarker an der jeweiligen Testlinie immobilisiert wird, um ein Signal über das ebenfalls spezifisch an jeden Biomarker gebundene markierte Bindungsreagenz zu erzeugen.

16. Verfahren, System oder Testkit nach Anspruch 14 oder 15, bei dem die Testlinie für Cystatin C eine immobilisierte Version von Cystatin C oder einem Analogon davon umfasst, die mit Cystatin C in der Probe um die spezifische Bindung an das markierte Bindungsreagenz konkurrieren kann.

17. Verfahren nach einem der Ansprüche 1-9 oder 14-16, bei dem der Proteinspiegel zumindest von MMP8 unter Verwendung eines Lateral-Flow-Assays nachgewiesen wird.

## Revendications

1. Procédé de détection d'une infection des voies urinaires chez un sujet comprenant :
i) la détermination du taux protéique d'au moins la MMP8 dans un échantillon d'urine obtenu chez le sujet ; et
ii) la comparaison du taux protéique déterminé d'au moins la MMP8 avec un taux seuil, dans lequel un taux protéique augmenté d'au moins la MMP8 dans l'échantillon d'urine par rapport au taux seuil indique la présence d'une infection des voies urinaires.

2. Procédé de surveillance d'une infection des voies urinaires chez un sujet comprenant :
i) la détermination du taux protéique d'au moins la MMP8 dans des échantillons d'urine obtenus chez le sujet à plusieurs moments ; et
ii) la comparaison du taux protéique déterminé d'au moins la MMP8 avec un taux seuil, dans lequel la présence continue du taux protéique non réduit ou augmenté d'au moins la MMP8 par rapport au taux seuil ou par rapport au taux mesuré dans un échantillon prélevé à un moment antérieur indique que l'infection des voies urinaires persiste et/ou que le traitement n'a pas été efficace, et/ou du taux protéique réduit d'au moins la MMP8 par rapport au taux seuil ou par rapport au taux mesuré dans un échantillon prélevé à un moment antérieur indique la guérison, ou le succès du traitement, d'une infection des voies urinaires.

3. Procédé de détection et de surveillance d'une infection des voies urinaires chez un sujet comprenant :
i) la réalisation du procédé selon la revendication 1 ; et, lorsqu'une infection des voies urinaires est détectée
ii) la détermination du taux protéique d'au moins la MMP8 dans un échantillon d'urine supplémentaire obtenu chez le sujet à un moment ultérieur
dans lequel un taux protéique non réduit ou augmenté d'au moins la MMP8 par rapport au taux seuil ou au taux mesuré à l'étape (i) indique que l'infection des voies urinaires persiste et/ou un taux protéique réduit d'au moins la MMP8 par rapport au taux seuil ou au taux mesuré à l'étape (i) indique la guérison, ou le succès du traitement, de l'infection des voies urinaires.

4. Procédé de surveillance d'un traitement d'une infection des voies urinaires chez un sujet comprenant :
i) la détermination du taux protéique d'au moins la MMP8 dans un échantillon d'urine obtenu chez le sujet avant le traitement de l'infection des voies urinaires afin de définir un taux seuil ;
ii) la détermination du taux protéique d'au moins la MMP8 dans un échantillon d'urine supplémentaire obtenu chez le sujet après le traitement de l'infection des voies urinaires
dans lequel un taux protéique non réduit ou augmenté d'au moins la MMP8 par rapport au taux seuil indique que le traitement n'a pas été efficace et/ou un taux protéique réduit d'au moins la MMP8 par rapport au taux seuil indique le succès du traitement de l'infection des voies urinaires.

5. Procédé selon l'une quelconque des revendications 1 à 4, dans lequel les taux protéiques d'au moins deux ou trois biomarqueurs sont déterminés, facultativement dans lequel un ou plusieurs des biomarqueurs sont sélectionnés parmi la HNE, la cystatine C, la MMP9, la HSA, l'IL-8, l'interleukine-6 (IL-6), l'interleukine-1 bêta (IL-1b), le fibrinogène, la RBP4, les MMP9 et MMP2 actives, la NGAL, la desmosine, la MPO et la CRP.

6. Procédé selon la revendication 5, dans lequel des taux protéiques non réduits ou augmentés d'au moins deux ou trois, facultativement de l'ensemble, des biomarqueurs dans l'échantillon d'urine par rapport au taux seuil ou par rapport aux taux mesurés dans un échantillon prélevé à un moment antérieur indiquent la présence d'une infection des voies urinaires.

7. Procédé selon l'une quelconque des revendications 1 à 6, dans lequel :
A) au moins 2, 3, 4, 5, 6, 7, 8, 9, 10, 11, 12, 13 ou 14 échantillons ou plus ont été prélevés chez le sujet à différents moments et les taux protéiques des un ou plusieurs biomarqueurs sont déterminés ; et/ou
B) les échantillons ont été prélevés toutes les 6 à 24 heures, tel que quotidiennement, ou tous les 3, 4, 5, 6, 7 ou 14 jours.

8. Procédé de sélection d'un sujet pour recevoir un traitement par un antibiotique comprenant la réalisation du procédé selon l'une quelconque des revendications 1 à 7 et la sélection du sujet pour recevoir un traitement lorsqu'une infection des voies urinaires est détectée ou persiste.

9. Procédé de prédiction de la réponse d'un sujet à un traitement par un antibiotique comprenant la réalisation du procédé selon l'une quelconque des revendications 1 à 7 et la prédiction de la réponse du sujet au traitement lorsqu'une infection des voies urinaires est détectée ou persiste.

10. Système ou kit de test pour détecter une infection des voies urinaires chez un sujet, comprenant :
a. un ou plusieurs dispositifs de test pour déterminer les taux protéiques d'au moins la MMP8 et facultativement d'un ou de plusieurs biomarqueurs sélectionnés parmi la HNE, la cystatine C, la MMP9, la HSA, l'IL-8, l'interleukine-6 (IL-6), l'interleukine-1 bêta (IL-1b), le fibrinogène, la RBP4, les MMP9 et MMP2 actives, la NGAL, la desmosine, la MPO et la CRP dans un échantillon d'urine obtenu chez le sujet
b. un processeur ; et
c. un support de stockage comprenant une application informatique qui, lorsqu'elle est exécutée par le processeur, est configurée pour :
i. accéder à et/ou calculer le taux protéique déterminé d'au moins la MMP8 dans l'échantillon sur le un ou plusieurs dispositifs de test
ii. calculer un score de test à partir du taux protéique d'au moins la MMP8 dans l'échantillon qui détecte une infection des voies urinaires ; et
iii. générer, à partir du processeur, le résultat détecté pour le sujet.

11. Système ou kit de test pour surveiller une infection des voies urinaires chez un sujet, comprenant :
a. un ou plusieurs dispositifs de test pour déterminer le taux protéique d'au moins la MMP8 et facultativement d'un ou de plusieurs biomarqueurs sélectionnés parmi la HNE, la cystatine C, la MMP9, la HSA, l'IL-8, l'interleukine-6 (IL-6), l'interleukine-1 bêta (IL-1b), le fibrinogène, la RBP4, les MMP9 et MMP2 actives, la NGAL, la desmosine, la MPO et la CRP dans un échantillon d'urine obtenu chez le sujet à plusieurs moments
b. un processeur ; et
c. un support de stockage comprenant une application informatique qui, lorsqu'elle est exécutée par le processeur, est configurée pour :
i. accéder à et/ou calculer le taux protéique déterminé d'au moins la MMP8 dans l'échantillon sur le un ou plusieurs dispositifs de test
ii. calculer un score de test à partir du taux protéique d'au moins la MMP8 dans l'échantillon, comprenant facultativement une comparaison du taux avec ceux prélevés à un ou plusieurs moments antérieurs, qui détecte une infection des voies urinaires ; et
générer, à partir du processeur, le résultat détecté pour le sujet.

12. Système ou kit de test selon la revendication 10 ou 11, dans lequel le dispositif de test comprend :
a. une zone recevant un échantillon à laquelle est ajouté un échantillon d'urine d'un sujet
b. une zone de conjugué comprenant au moins un réactif de liaison marqué qui se lie spécifiquement à la MMP8 et facultativement un ou deux réactifs de liaison marqués, chacun d'entre eux se liant spécifiquement à l'un des biomarqueurs sélectionnés parmi la HNE, la cystatine C, la MMP9, la HSA, l'IL-8, l'interleukine-6 (IL-6), l'interleukine-1 bêta (IL-1b), le fibrinogène, la RBP4, les MMP9 et MMP2 actives, la NGAL, la desmosine, la MPO et la CRP
c. un support solide définissant un trajet d'écoulement de liquide pour l'échantillon et comprenant une ligne de test correspondante pour au moins la MMP8, la ligne de test comprenant :
i. un réactif de liaison supplémentaire immobilisé qui se lie également spécifiquement à la MMP8, en immobilisant ainsi la MMP8 au niveau de la ligne de test afin de produire un signal via le réactif de liaison marqué qui est également spécifiquement lié à la MMP8 ; ou
ii. une version immobilisée de la MMP8 ou d'un analogue de celle-ci capable d'entrer en compétition avec la MMP8 dans l'échantillon pour une liaison spécifique au réactif de liaison marqué ;
facultativement dans lequel le dispositif de test comprend en outre :
au moins un réactif de liaison de contrôle marqué qui se lie à un partenaire de liaison immobilisé au niveau d'une ligne de contrôle en aval de la ligne de test pour la MMP8 et confirme ainsi que le test a été correctement réalisé ; et/ou comprenant en outre :
un matériau absorbant en aval de la ligne de test (et de la ligne de contrôle, si présente) pour absorber l'échantillon en excès.

13. Système ou kit de test selon la revendication 12, dans lequel :
A) le support solide comprend un milieu de chromatographie ;
B) le support solide comprend un dispositif à flux capillaire ;
C) le dispositif de test est une bandelette de test ;
D) le dispositif de test comprend en outre un récipient pour collecter un échantillon d'urine ;
E) le dispositif de test comprend en outre une aide visuelle présentant différents motifs d'intensité de ligne de test à partir de laquelle l'utilisateur peut interpréter les résultats de la ligne de test observés ; et/ou
F) le dispositif de test comprend en outre un lecteur pour déterminer les taux protéiques des marqueurs au niveau des lignes de test respectives.

14. Procédé selon l'une quelconque des revendications 1-9 ou système ou kit de test selon l'une quelconque des revendications 10-13, dans lequel les biomarqueurs comprennent :
a. la MMP8 et la HNE ;
b. la MMP8 et la HNE et la cystatine C ;
c. la MMP8, la HNE et le fibrinogène ;
d. la MMP8, la HNE et le fibrinogène et la cystatine C ;
e. la MMP8, la HNE et la CRP ; ou
f. la MMP8, la HNE et la CRP et la cystatine C.

15. Procédé, système ou kit de test selon la revendication 14, dans lequel la ligne de test pour chaque biomarqueur, respectivement, comprend un réactif de liaison supplémentaire immobilisé qui se lie également spécifiquement à chaque biomarqueur, respectivement, en immobilisant ainsi chaque biomarqueur au niveau de la ligne de test respective afin de produire un signal via le réactif de liaison marqué qui est également spécifiquement lié à chaque biomarqueur.

16. Procédé, système ou kit de test selon la revendication 14 ou 15, dans lequel la ligne de test pour la cystatine C comprend une version immobilisée de la cystatine C ou un analogue de celle-ci capable d'entrer en compétition avec la cystatine C dans l'échantillon pour une liaison spécifique au réactif de liaison marqué.

17. Procédé selon l'une quelconque des revendications 1 à 9 ou 14 à 16, dans lequel le taux protéique d'au moins la MMP8 est détecté en utilisant un essai à flux latéral.
